# EUROPEAN PATENT SPECIFICATION

(11) **EP 3 576 656 B1**
(45) Date of publication and mention of the grant of the patent: **26.10.2022**
(21) Application number: 18747862.3
(22) Date of filing: 04.02.2018
(51) Int. Cl.: A61B 18/02, A61B 1/12, A61B 1/015, A61B 90/00

(54) **CRYOTHERAPY DEVICE FLOW CONTROL**
DURCHFLUSSREGELUNG FÜR KRYOTHERAPIEVORRICHTUNG
COMMANDE DE FLUX DE DISPOSITIF DE CRYOTHÉRAPIE

(30) Priority: 04.02.2017 US 201762454753 P
(43) Date of publication of application: 11.12.2019
(73) Proprietor: Vessi Medical Ltd., 2017400 Mobile Post Misgav (IL)
(72) Inventor: KOCHAVI, Eyal, 3452620 Haifa (IL)
(74) Representative: Tiburzi, Andrea
(86) International application number: PCT/IL2018/050124
(87) International publication number: WO 2018/142411

(56) References cited:
- WO-A1-98/52479
- WO-A1-2016/088120
- WO-A1-2016/088120
- WO-A1-2017/189781
- WO-A2-2012/060932
- ES-T3- 2 491 540
- GB-A- 2 289 413
- US-A1- 2005 081 541
- US-A1- 2007 244 353
- US-A1- 2013 218 149
- US-A1- 2014 309 655
- US-A1- 2015 080 794
- US-A1- 2015 148 791
- US-A1- 2017 014 202

## Description

### FIELD AND BACKGROUND OF THE INVENTION

The present invention, in some embodiments thereof, relates to ablation, including cryoablation, cryosurgery or cryotherapy devices and, more particularly, but not exclusively, to cryotherapy of body lumen diseases.

Cryoablation, cryosurgery or cryotherapy is a technique by which targeted areas, which may include undesired symptoms, lesions, or free nerve endings are destroyed or ablated by freezing. Tissue destruction by freezing includes direct injury to cells caused by ice crystal formation, as well as delayed injury that may be caused by apoptosis (regulated cells death) and/or vascular effects. Cryotherapy is performed by utilizing a pressurized coolant (such as CO₂, LN₂, and/or nitrous oxide), in order to directly spray the cryogenic fluid onto the treated tissue and/or to enable an indirect Joule Thompson effect to occur (using coolants, such as, Argon, Nitrogen and/or Krypton).

The use of cryosurgery to ablate tumors is expanding, primarily due to its technical ease and low morbidity rate. A potential secondary advantage to the in situ freezing of malignant diseases is the cryo-immunological response, i.e., the generation of an anti-tumor immune response triggered by the natural absorption of the malignant tissue. Clinical reports suggest that cryoablation may induce a systemic antitumor immune response, which has also been confirmed in animal models.

Body lumens may include bladder, uterus and others. Bladder cancer is one of the most common cancers. When a diagnosis is made at an early stage, the majority of patients diagnosed with bladder cancer have superficial disease and are low grade in nature; others however, are diagnosed at a later stage, which the cancer is more invasive and aggressive so surgical intervention (including cystectomy) may be utilized. Bladder cancer has a high likelihood of recurrence; therefore treatment and follow-up is critical in the prevention of recurrence and progression. It is suggested to use cryotherapy methods in order to improve treatment for bladder lesions, reduce recurrences and potentially increase bladder preservation rate.

Additional example for bladder illness is Interstitial Cystitis (IC), also known as painful bladder syndrome, a chronic condition of bladder and/or pelvic pain, ranging from mild discomfort to severe pain. Although there is no treatment that reliably eliminates IC, medications and other therapies are offered to relieve pain. For example, Botox injections inside the bladder are thought to block the sensory nerves in the bladder that transmit pain. It is suggested to use cryotherapy methods, ablating/harming free nerves ending, in order to improve treatment for IC and similar conditions, in order to increase patients' comfort for longer times.

Regarding uterine illnesses and lesions, in order to avoid major surgical intervention, such as, hysterectomy or myomectomy, hysteroscopic procedures may be utilized. Uterine lesions may include sub-mucous fibroids or large polyps or menorrhagia with normal endometria. Additional uterine-related illnesses include thin endometrium and/or Asherman's syndrome (AS), both of which hinder conception. It is suggested to use cryotherapy methods in order to improve treatment for uterine illnesses and lesions thus easing the treatment and potentially increasing uterus preservation rate. Generally, cryotherapy may aid in endometrium rejuvenation and therefore result in an increase in the conception success probability.

There are no known cryotherapy devices that are inserted into body lumen through an endoscope's working channel. On the other hand, catheter devices that are inserted via an endoscope and are used for treating the gastrointestinal (GI) tract are known in the art. The main differences between current GI devices to those required for body lumens treatments are the size of the necessary insertion cavity (esophagus or colon are significantly bigger in diameter), the need for sensing and closed-loop pressure control in order to keep lumen open while avoiding over-pressure, and the need to deal with moisture and even remaining liquids that may interfere with ablation efficacy and may add significant visualization difficulties. For example, some cryotherapy devices include the use of a cryogenic fluid jet, such that the cryogenic fluid or coolant exits through a nozzle and is directly applied onto the tissue in the form of a spray. In some of the cryotherapy devices that utilize spray, an additional aspiration channel is needed in order to evacuate the spray's expanded fluids thus preventing unwanted lumen inflation or tissue perforation. Known aspiration channels do not have the ability to control lumen volume or pressure due to the lack of sensing means and appropriate control mechanisms. In addition the distance of the nozzle from the targeted area is not well-defined so as a result, the treatment outcome is not predictable and it may be difficult for the physician to follow the cryosurgery protocol.

There is no known use for cryotherapy devices in combination with immune modulation therapy within a body lumen, either using an endoscope or not.

Therefore, there is a need for a modified endoscopic cryotherapy device that allows ablating tissue within body lumens, wherein the device has the ability to control pressure and/or volume, and further, has the ability to operate in the presence of moisture or even liquids. There is also a need for a modified cryotherapy device with ability to evacuate coolant expanded fluids through the limited size of the body lumen entrance. There is also a need to particularly define the distance between the cryo spray and the treated tissue. There is also a need to improve cryo-immunological methods and to adapt them to cryoablation within a body lumen.

The relevant prior art comprises the patent application US 2007/244353 A1 which describes "a surgical instrument has a channel dimensioned to receive a viewing instrument and enable the viewing instrument to be moved to or from a position near an optically transparent portion of a blunt, enclosed distal end of a shaft to provide unobstructed viewing through the distal end, and a position to the proximal side of an enclosed working area to provide viewing of the enclosed working area. A surgical instrument also or alternatively has a fluid routing switch within a shaft which can selectively connect a fluid infusion channel to at least one fluid export pore or a return channel. A method involves moving a viewing instrument to or from a position near an optically transparent portion of a blunt, enclosed distal shaft end and a proximal side of an enclosed working area. A method also or alternatively involves changing a position of a fluid routing switch within the shaft" (abstract).

WO2016088120 discloses a cryotherapy device with an inflow channel.

### SUMMARY OF THE INVENTION

The invention is defined in appended claim 1, preferred embodiments are described in the dependent claims.

Examples are directed to a cryotherapy device comprising:
- at least one inflow channel;
- at least one outflow channel;
- control means for controlling the evacuation of expanded cryo-fluid from a body lumen; and
- visualization means for visualizing a field of view;
wherein the control means receive data from at least one sensor that gathers data regarding at least one parameter of the body lumen and wherein the cryotherapy device is introduced into the body lumen via an endoscope or a sheath.

According to some embodiments, the device further comprising a rolling component, wherein the inflow channel is directed towards the rolling component.

According to some embodiments the device further comprising an extension, which, distances a distal end of the device from a treated area, at least partially, separates a treatment volume from a surrounding volume of the body lumen, or both. According to some embodiments, the distal ends of the visualization means, the inflow channel or both are within the treatment volume. According to some embodiments, the extension separates between the inflow channel and the outflow channel. According to some embodiments, sensing means are located within the treatment volume, outside of the treatment volume, or both inside and outside of the treatment volume.

According to some embodiments, the extension includes folded, partially folded and unfolded configuration and wherein the volume and shape of the treatment volume differs according to the configuration of the extension. According to some embodiments, the extension includes supporting components, extendable components, compliant components, non-compliant components, semi-compliant components, flexible components, non-flexible components, or any combination thereof. According to some embodiments, the extension includes overlapping components, and wherein the degree of the overlap between the overlapping components is changeable. According to some embodiments, the extension includes any number of vents.

According to some embodiments, a distal end of the device comprises a swiveling component comprising at least one inflow nozzle, which swivels together with the swiveling component.

According to some embodiments, the inflow channel and the outflow channel are the same channel. According to some embodiments, the overall diameter of the inflow channel, the outflow channel and any other channels or devices introduced is between 0.8-9.0mm. According to some embodiments, at least one tube or catheter is fed through at least one channel, and wherein said tube or catheter is at least partially flexible, bendable, kink resistant or any combination thereof. According to some embodiments, at least one inflow channel and at least one outflow channel are part of a multi-channel fabricated or extruded tube or catheter. According to some embodiments, the inflow channel, the outflow channel, or both, are at least partially braided, coiled, or both.

According to some embodiments, the inflow channel is attached to at least one inflow nozzle, and wherein the inner diameter of any one of the inflow nozzles is in the range of 0.05-0.3mm. According to some embodiments, the outflow channel is attached to at least one outflow opening, and wherein the inner diameter of any one of the outflow openings is in the range of 0.5-4.0mm.

Further disclosed is a method of treating tissue within a body lumen, said method comprising:
- introducing a cryotherapy device comprising visualization means into a body lumen via an endoscope or a sheath;
- visualizing a field of view by way of the visualization means;
- injecting cryo-fluid through at least one inflow channel of the cryotherapy device, directly or indirectly, into the body lumen, such that the cryo-fluid expands, directly or indirectly, in the body lumen thereby freezing at least part of the treated tissue; and
- evacuating expanded cryo-fluid, directly or indirectly, through at least one outflow channel of the cryotherapy device, from the body lumen;
   wherein the evacuation of the cryo-fluid is controlled by control means receiving data from at least one sensor that gathers data regarding at least one parameter of the body lumen.

According to some embodiments,
- the cryo fluid is injected into the field of view;
- a treated area or volume is within the field of view;
- the cryo fluid is evacuated from the field of view; or any combination thereof.

According to some embodiments, further comprising injecting at least one additional fluid through at least one inflow channel of the cryotherapy device, directly or indirectly, into the body lumen, wherein said additional fluid maintains the body lumen above a defined pressure, dries at least part of a treated volume or area, or any combination thereof. According to some embodiments, the defined pressure is between 10-20mBar. According to some embodiments, the additional fluid is selected from CO₂, air, argon, N2, lower pressure gas and/or warm gas.

According to some embodiments, the cryotherapy device includes a rolling component, and wherein said method comprises:
- injecting the cryo-fluid into or onto the rolling component; and
- rolling the rolling component over at least part of the treated tissue in the body lumen.

According to some embodiments, the cryotherapy device includes an extension that forms;
- a distance between a distal end of the cryotherapy device and the treated tissue
- a treatment volume that is at least partially separated a surrounding volume of the body lumen; or
- both, and wherein said method comprises:
   - injecting the cryo-fluid into the treatment volume;
   - visualizing the treatment volume by the visualization means, the distal end of which is within or outside of the treatment volume; and
   - optionally injecting an additional fluid into the treatment volume, wherein the additional fluid lowers the humidity in the treatment volume, maintains the pressure of the body lumen or treatment volume above a define pressure, or both.

According to some embodiments, said device comprises additional sensing means, wherein the distal end of each of the sensing means is within the treatment volume, outside of the treatment volume or both. According to some embodiments, the configuration of the extension is changed during the cryo treatment, such that the extension is folded, partially folded, unfolded, or any combination thereof, during the cryo-treatment, thereby changing the volume and shape of the treatment volume. According to some embodiments, a distal end of the cryotherapy device includes a swiveling component comprising at least one inflow nozzle, wherein the cryo fluid is injected through the inflow nozzle, which swivels together with the swiveling component, thereby assist with the coverage of an area or a volume with the cryo fluid.

According to some embodiments, at least one inflow channel and at least one outflow channel are the same channel, wherein the direction of flow therethrough is controlled by the control means. According to some embodiments, at least one inflow channel and at least one outflow channel are part of a multi-channel fabricated or extruded tube or catheter. According to some embodiments, sharp angular movements of the endoscope or sheath do not kink the inflow channel or the outflow channel. According to some embodiments, the inflow channel and the outflow channel are at least partially bendable, flexible or kink-resistant. According to some embodiments, the inflow channel, the outflow channel, or both, are at least partially braided, coiled, or both.

According to some embodiments, the body lumen is a bladder, a cervix, a prostate, a urethra, a ureter, a stomach or a uterus. According to some embodiments, body fluids are evacuated from the body lumen through at least one outflow channel. According to some embodiments, the control means receives parameters regarding the internal pressure in the treated body lumen, the temperature of the treated body lumen, flow rate, flow time, or any combination thereof.

According to some embodiments, the cryo-fluid is indirectly injected into the body lumen, wherein it is injected into a folded component. According to some embodiments, the folded component is a cryo-balloon. According to some embodiments, the cryo-fluid is injected into a catheter, wherein the catheter is inserted into the body lumen though does not have an opening into the body lumen, and wherein the expanded cryo-fluid is evacuated from within the catheter.

According to some embodiments, the cryo-fluid is injected together with at least one additional active component. According to some embodiments, the active component is a biological, immunological, chemical, nanoparticle or a chemotherapy entity. According to some embodiments, the active component is selected from mitomycin C, doxorubicin and dendritic cells.

According to some embodiments, the cryo-fluid is selected from liquid nitrogen, carbon dioxide (CO₂), nitrous oxide (N₂O), or any combination thereof.

Further disclosed examples are directed to a cryotherapy device, which is passed through an endoscope or a sheath, for treating at least one targeted region within a body lumen, said device comprising:
- a folded ablating cryo-balloon;
- means for introducing the folded cryo-balloon into a body lumen through an endoscope or a sheath;
- means to unfold the cryo-balloon inside the body lumen; and
- visualization means for visualizing a field of view that includes at least part of the unfolded cryo-balloon and at least a part of the targeted region.

According to some embodiments, the cryo balloon has regions that have different degrees of compliance, and wherein a region of the cryo-balloon that is active in transferring cold temperature to the targeted regions, is brought into contact with a targeted region by inflation of the balloon, movements of the balloon, or both inflation and movements of the balloon. According to some embodiments, the cryo balloon comprises at least one supporting, non-compliant, region and at least one active, compliant or semi-compliant, region, and wherein the visualization means visualizes a field of view that includes the supporting region, part of the active region, or both.

According to some embodiments, the means to unfold the cryo-balloon inside the body lumen include a jet of cryo fluid, a jet of an additional fluid, or any combination thereof, wherein any one of the jets is by any number of nozzles, and wherein optionally, any one of the nozzles swivels around an axis by any number of degrees. According to some embodiments, the cryo-balloon is compliant, semi-compliant, non-compliant, or any combination thereof. According to some embodiments, the diameter or average diameter of the cryo-balloon, when folded is below 2.5mm.

Further disclosed examples are directed to a cryotherapy device, which is passed through an endoscope or a sheath, for treating at least one treated area within a body lumen, said device comprising:
- a rolling component;
- means for introducing the rolling component into a body lumen though an endoscope or a sheath; and
- means for injecting cryo fluid into the rolling component.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present invention may be embodied as a system, method or computer program product. Accordingly, some embodiments of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, some embodiments of the present invention may take the form of a computer program product embodied in one or more computer readable medium(s) having computer readable program code embodied thereon. Implementation of the method and/or system of some embodiments of the invention can involve performing and/or completing selected tasks manually, automatically, or a combination thereof. Moreover, according to actual instrumentation and equipment of some embodiments of the method and/or system of the invention, several selected tasks could be implemented by hardware, by software or by firmware and/or by a combination thereof, e.g., using an operating system.

For example, hardware for performing selected tasks according to some embodiments of the invention could be implemented as a chip or a circuit. As software, selected tasks according to some embodiments of the invention could be implemented as a plurality of software instructions being executed by a computer using any suitable operating system. In an exemplary embodiment of the invention, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the invention. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present invention may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as monitoring pressure and/or temperature during cryotherapy, might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1A is a block diagram of a cryotherapy system, in accordance with some embodiments of the present invention;
Fig. 1B is a flow chart of a general cryotherapy process, in accordance with some embodiments of the present invention;
Figs. 1C to 1F illustrate schematic lengthwise sectional views of a cryotherapy device, during cryotherapy procedure, introduced into the treated lumen via an endoscope in accordance with some embodiments of the present invention;
Figs. 2A, 2B and 2C illustrate schematic lengthwise sectional views of a cryotherapy system, specifically presenting means by which fluids may pass in and out of the treated area in accordance with some embodiments of the present invention;
Fig. 3 illustrates a schematic lengthwise sectional view of a cryotherapy system and specifically the means by which the device's distal end is positioned and fixed in accordance with some embodiments of the present invention;
Figs. 4A to 4E illustrate schematic lengthwise sectional views of a cryotherapy device's distal end in accordance with some embodiments of the present invention;
Figs. 4F to 4H illustrate schematic transverse cross-sectional views of a cryotherapy device's distal end, in accordance with some embodiments of the present invention;
Figs. 5A to 5D illustrate schematic lengthwise sectional views of a cryotherapy device introduced through an endoscope, during a sharp angulation scenario, in accordance with some embodiments of the present invention;
Figs. 6A to 6E illustrate schematic lengthwise sectional views of the distal end of a cryotherapy device, which includes specific nozzles, providing different types of sprays, in accordance with some embodiments of the present invention;
Figs. 7A and 7B illustrate schematic lengthwise sectional views of a cryotherapy device introduced via an endoscope and especially the use of endoscope's in-flow and out-flow channels, in accordance with some embodiments of the present invention;
Figs. 8A and 8B illustrate schematic lengthwise sectional views of a cryotherapy device introduced via an endoscope within the treated lumen during ablation procedure in accordance with some embodiments of the present invention;
Figs. 8C and 8D illustrate schematic lengthwise sectional views of a cryotherapy inflated device introduced via an endoscope within the treated lumen during an ablation procedure in accordance with some embodiments of the present invention;
Figs. 8E to 8G illustrate schematic lengthwise sectional views of a cryotherapy inflated device introduced inside lumen during an ablation procedure in accordance with some embodiments of the present invention;
Figs. 9A and 9B illustrate schematic lengthwise sectional views of the distal end of a cryotherapy device, specifically presenting cryo-balloons, in accordance with some embodiments of the present invention.
Figs. 10A to 10D illustrate schematic lengthwise sectional views of a cryotherapy device, during cryotherapy procedure, introduced into the treated lumen via an endoscope or a sheath in accordance with additional embodiments of the present invention;
Fig. 11 illustrates schematic lengthwise sectional view of a cryotherapy system, presenting flow paths and means by which fluids may pass in and out of the treated lumen in accordance with some embodiments of the present invention;
Figs. 12A and 12B illustrate schematic lengthwise sectional views of a rolling cryotherapy device having a roller in accordance with some embodiments of the present invention;
Fig. 13 illustrates schematic lengthwise sectional view of a distal end of the cryotherapy device that includes an extension, in accordance with some embodiments of the present invention;
Fig. 14 illustrates schematic lengthwise sectional view of the distal end of a cryotherapy device, which includes a rotating nozzle, in accordance with some embodiments of the present invention;
Figs. 15A and 15B illustrate schematic lengthwise sectional views of a cryotherapy device introduced via an endoscope and including the use of endoscope's in-flow and out-flow channels, in accordance with some embodiments of the present invention;
Figs. 16A to 16C illustrate schematic lengthwise sectional views of a cryotherapy inflated device that includes an extension, during an ablation procedure in accordance with some embodiments of the present invention;
Figs. 16D and 16E illustrate schematic upper views of a cryotherapy device's distal end that includes an extension, in accordance with some embodiments of the present invention;
Figs. 17A to 17F illustrate schematic lengthwise sectional views of the distal end of a cryotherapy device that includes an extension, in accordance with some embodiments of the present invention;
Figs. 18A to 18C illustrate schematic transverse cross-sectional views of endoscope's visualization means and cryotherapy device's distal end that includes an extension, in accordance with some embodiments of the present invention;
Figs. 19A to 19C illustrate schematic transverse cross-sectional views of a cryotherapy device's distal end that includes an extension, in accordance with some embodiments of the present invention;
Figs. 20A and 20B illustrate schematic cross section views of a cryotherapy device's distal end that includes a wash inflow path, in accordance with some embodiments of the present invention;
Fig. 21A illustrates a flow scheme of a coolant flow and a low-pressure flow through a cryotherapy device, in accordance with some embodiments of the present invention;
Fig. 21B is a flow chart of a process for flow and/or purge control, in accordance with some embodiments of the present invention;
Figs. 22A-22F are graphs showing pressure changes inside the body lumen as a function of flow and time, in accordance with some embodiments of the present invention; and
Figs. 23A-23G illustrate schematic views of a cryotherapy device's distal end with different distance and angle measuring means (FIGS. 23E and 23G are schematic upper views of a target region following projection of a light beam or a low pressure flow), in accordance with some embodiments of the present invention;

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DESCRIPTION OF SPECIFIC EMBODIMENTS OF THE INVENTION

The present invention, in some embodiments thereof, relates to ablation, including cryoablation, cryosurgery or cryotherapy devices and, more particularly, but not exclusively, to cryotherapy of body lumen diseases.

An aspect of some embodiments relates to performing cryotherapy while controlling at least one micro-environmental parameter inside a body lumen. In some embodiments, the parameter is controlled inside a defined treatment space, for example a treatment space between a cryotherapy device and a target region. In some embodiments, the parameter is controlled to allow better visualization of the treatment region.

According to some embodiments, the parameter is controlled by controlling flow into the treatment space through at least one inflow path of a cryotherapy device. Alternatively or additionally, the at least one parameter is controlled by controlling evacuation of material from the treatment space, optionally through at least one outflow path of the cryotherapy device. In some embodiments, flow within the treatment space is controlled to increase the efficacy of the cryotherapy, for example to allow better engagement with a target tissue. Alternatively or additionally, flow within the treatment space is controlled to increase the safety of the cryotherapy, for example not to exceed a maximal pressure and/or temperature value that may lead to tissue damage.

According to some embodiments, flow into the treatment space is controlled for example to allow washing an optics assembly, for example a lens and/or a light source. Alternatively or additionally, flow is controlled, for example to allow to clear the treatment space from vapor, cryo mist and/or liquid droplets that cross a field of view between the optics assembly and the target area. In some embodiments, clearing the treatment space from vapor, cryo mist and/or liquid droplets allows for example to improve and/or optimize cryotherapy and visualization of the treated area within the body lumen. In some embodiments, flow into the treatment space is controlled to allow for example, inflation of the body lumen before and/or during the cryotherapy process. Alternatively or additionally, flow through a nozzle used for spraying cryogenic fluid is controlled to prevent clogging of the nozzle by liquid droplets or frozen particles.

According to some embodiments, the treatment space is defined by adjusting geometrical parameters of the treatment space. In some embodiments, for example, to better control micro-environmental parameters affecting cryotherapy within the lumen, the treatment area and/or the volume between the cryotherapy device and the target region is demarcated, optionally by a volume sub-divider configured to define a treatment space within the body lumen configured to define a treatment space between said distal end and said selected target region by reducing fluid mixing between the treatment space and other parts of the lumen. In some embodiments, the volume sub-divider comprises a geometrical element. In some embodiments, the geometrical element, for example an extension, a cone, or a dress optionally unfolds from a distal end of the cryotherapy device positioned within the body lumen. Alternatively or additionally the volume sub-divider comprises a surrounding fluid flow around a distal end of the cryotherapy device or around an opening of a cryogenic inflow in the distal end of the cryotherapy device. In some embodiments, the surrounding fluid flow, for example a surrounding air or gas flow generates a virtual cone around the distal end or the cryogenic flow opening defining a treatment space within the body lumen. Optionally, the surrounding fluid flow is generated for example using focused flow of gas, optionally low pressure gas around the cryogenic flow, within the body lumen.

According to some embodiments, the geometrical element allows, for example to better control vapors and/or humidity that interfere with visualization inside the lumen, for example during and/or between cryo-ablating cycles by limiting a controlled volume within the lumen. In some embodiments, the deployed geometrical element allows to generate a separate microenvironment within the body lumen, that optionally has a different, lower or higher humidity, pressure and/or temperature.

According to some embodiments, the pressure within the body lumen increases during cryotherapy. In some embodiments, pressure increases due to inflation of the body lumen with a low-pressure gas, for example to allow better visualization and/or access to a selected target region. Alternatively or additionally, the pressure within the body lumen increases due to the expansion of a cryogenic fluid within the body lumen. In some embodiments, in order to reduce the pressure levels, for example not to exceed a maximal allowed pressure value, a control unit regulates the flow into the body lumen through at least one inflow path. In some embodiments, the flow into the body lumen is stopped and/or at least partly diverted to a flow path outside the body. Alternatively, at least one flow regulator connected to an outflow path is opened to evacuate some of the material within the body lumen. In some embodiments, the flow regulator is passively opened. Alternatively the flow regulator is actively opened, for example by an electric signal. In some embodiments, the flow regulator comprises a valve for example a check-valve and/or a pump, for example a vacuum pump. In some embodiments, the flow regulator is used to control pressure inside the body lumen.

According to some embodiments, the maximal pressure value is determined based on the type of tissue, and/or based on the pressure duration. In some embodiments, the pressure values during a cryotherapy treatment lasting 30-70 minutes within a body lumen, for example the bladder include regular inflation pressure in a range of 10-30 mBar, higher pressure values in a range of 20-50 mBar during cryotherapy cycles of 10-30sec while allowing peaks in a range 50-150 mBar for a period of 1 sec or less. In some embodiments, the maximal pressure value during a cryotherapy session lasting 30-70 minutes in a body lumen, for example the bladder, is about 80 mBar, for example 50 mBar, 60 mBar, 70 mBar or any intermediate, smaller or larger value.

According to some embodiments, the temperature within the body lumen decreases during cryotherapy, for example due to the expansion of the cryogenic coolant. In some embodiments, the temperature within the body lumen is controlled, for example by controlling the flow of the coolant into the body lumen. Alternatively or additionally, the temperature is controlled by opening an outflow path to evacuate some of the material from within the body lumen. In some embodiments, a warmed washing fluid, for example a warmed gas is pushed into the body lumen, for example to increase the temperature levels inside the treatment space. Additionally or alternatively, the warmed gas is pushed towards a lens of the optics assembly for example to keep the lens in a constant temperature and/or to prevent condensation of water droplets on the lens surface.

An aspect of some embodiments relates to performing cryotherapy within a body lumen by adjusting geometrical parameters related to the treatment. In some embodiments, a geometrical relationship, for example distance and/or angle between a distal end of a cryotherapy device positioned inside a body lumen and a target area within the body lumen is determined. Optionally, at least one cryotherapy parameter is determined and/or adjusted according to the determined distance and/or angle, for example spraying duration of the cryogenic fluid, time between spraying sessions, and/or cryogenic fluid pressure.

According to some embodiments, the distance and/or angle are determined based on deployment of at least one geometrical element. In some embodiments, the geometrical element, for example an extension, a cone, or a dress optionally unfolds from a distal end of the cryotherapy device part positioned within the body lumen.

According to some embodiments, the geometrical element allows, for example to determine a distance and/or an angle between a cryo-nozzle of the cryotherapy device positioned within the body lumen and a selected target region. In some embodiments, deployment of a geometrical element, optionally with known size and/or shape, allows for example, to determine a distance to the selected target region and/or an angle between the cryo-nozzle and the target region by visualizing a contact point between the geometrical element and the internal surface of the lumen. Alternatively or additionally, the distance to the target region is determined by receiving a signal when the geometrical element contacts the internal surface.

According to some embodiments, a focused flow of gas, for example low pressure gas from the cryo-nozzle allows to determine a distance between the cryo-nozzle and a selected target region, and/or an angle between the cryo-nozzle and the target region. In some embodiments, the distance and/or angle is determined by visualizing the contact point of the gas with the internal surface of the lumen. Optionally, the focused gas flow is delivered through the cryo nozzle between cryoablation sessions. In some embodiments, the focused gas flow partly deforms the shape of the internal surface of the lumen, for example by forming an indentation or an oval shape deformation) in the contact point with the internal surface. In some embodiments, visualization of the indentation size and/or shape allows for example to determine the distance to the target region and/or the angle between the cryotherapy device and the target region.

According to some exemplary embodiments, the distance and/or angle are determined by visualizing a project light spot on the surface of the target region. In some embodiments, the distance and/or angle are determined based on the shape and/or size and/or color change of the light spot. In some embodiments, the light spot is generated by a light beam from a light source placed at the distal end of a cryotherapy device positioned within the body lumen. Optionally, the light source is generated by a laser light source or any other light source capable of generating a focused light source. In some embodiments, the distance and/or angle between the cryotherapy device and the target region are determined by visualizing the target region, optionally together with at least part of the cryotherapy device, and processing the image by one or more image processing algorithms.

An aspect of some embodiments relates to performing cryotherapy while applying washing fluid, also termed herein as clearing material, into the body lumen. In some embodiments, washing fluid, for example gas, optionally a low-pressure gas is introduced into the body lumen while coolant is sprayed at a target region. Alternatively or additionally, the washing fluid is introduced into the body lumen between spraying cycles. In some embodiments, the washing fluid is pushed into the body lumen through at least one opening near or at the distal end of the cryotherapy device.

According to some embodiments, the washing fluid is pushed into the body lumen to inflate the lumen. In some embodiments, the lumen is inflated to allow better visualization of a cryotherapy target region. Alternatively or additionally, the lumen is inflated to allow better maneuvering of the distal end of the cryotherapy device to a selected target region.

According to some embodiments, the washing fluid is introduced into the body lumen to clean an optical assembly of the cryotherapy device, for example a lens. In some embodiments, the washing gas cleans the lens for example, from moisture and/or dirt that may cover the lens. Alternatively or additionally, the washing fluid is introduced into the body lumen to clear a field of view (FOV) between the optical assembly and the target region. In some embodiments, the washing fluid is used to wash cold moisture gas, for example mist away from the optical assembly. In some embodiments, the washing gas clears the FOV from mist and/or frozen droplets formed by the interaction of the expanded cryogenic gas with liquid particles in the humidified space of the lumen.

According to some embodiments, the washing fluid, optionally a warm washing fluid generates a dynamic buffer around the lens surface ('Optics wash'), for example to prevent mist remainders (or evacuated mist) to flow towards the lens direction. In some embodiments, the washing fluid pushes away mist and/or frozen particles from the optics assembly. Alternatively or additionally, the warm washing fluid keeps the optics assembly, for example a lens of the optics assembly in a constant warm temperature to prevent the formation of mist or liquid droplets on the lens surface.

According to some embodiments, the washing fluid is used for pressure measurements. In some embodiments, as a result of pressure changes inside the lumen, pressure changes can be measured closer to the wash pressure source (upstream - e.g. inside a control unit), in a way that pressure inside the lumen can be measured/approximated (and can be used, for example, for control and/or safety means).

According to some embodiments and as described previously, the washing fluid is used, optionally simultaneously for two or more of the following: optics assembly washing, clearing mist from the FOV, inflating a body lumen optionally during the cryotherapy process and for measuring body lumen pressure levels. In some embodiments, a control unit of the cryotherapy device controls the flow distribution of the washing fluid between flow towards the optics assembly and flow for clearing mist from the FOV. Alternatively or additionally, the control unit controls coordinating the flow and timing of the washing fluid flow with the spraying of the cryogenic coolant, for example not to exceed a maximal pressure value of the body lumen. In some embodiments, the control unit controls the flow of the washing fluid into the body lumen alone or in combination with either cryogenic coolant flow or purge flow, for example low pressure flow through the nozzle, for example to make sure that the inflation level of the body lumen is higher than a minimal pressure value.

An aspect of some embodiments relates to preventing blockage of a cryo-nozzle of a cryogenic inflow path by controlling flow through the nozzle. In some embodiments, the flow through the cryo-nozzle is controlled to prevent blockage when cryogenic coolant flow is stopped. In some embodiments, when coolant flow is stopped, a purge flow through the cryo-nozzle is initiated. In some embodiments, the purge flow is a flow of a non-cryogenic gas, optionally a low-pressure gas. In some embodiments, when the cryogenic coolant fluid is stopped, a vent is opened, optionally for a predetermined time period, to release residual cryogenic fluid trapped inside the inflow path. Alternatively, the vent is opened for a time period adjusted according to the amount of residual cryogenic coolant trapped inside the cryogenic inflow path.

According to some embodiments, the cryogenic fluid as described herein in the application comprises cryo compressed fluid - liquid CO₂, in a pressure level about 50-80 Bars in the cryogenic source. Alternatively, the cryogenic fluid comprises fluids in a pressure of about 200 Bar in the cryogenic fluid source, for example Nitrogen gas. In some embodiments, the cryogenic fluid is in a pressure of several bars for example 1-10 bar but as liquid which expands to gas, for example LN₂, liquid Nitrogen which optionally expands in liquid:gas ratio of about 1:1000. In some embodiments, and without being bound by any theory, expansion of high pressure fluid inside the body lumen, decreases the pressure of the fluid and generates a cryogenic effect, for example the Joule-Thomson effect.

According to some embodiments, the washing fluid comprises a low pressure gas with a pressure of 1-4 bars at its source, and optionally in a pressure of tens of miliBars when expanded into the bladder. In some embodiments, any low-pressure gas can be used for wash and/or inflation of the body lumen. Optionally a stored high pressure gas is used after a pressure reduction, for example within a lumen of the cryotherapy device prior to release into the body lumen. A possible advantage of using the same type of gas for both washing and cryoablation is that there is no need for separate sources for both cryoablating and washing. A possible advantage of using air as a washing fluid is that it reduces the need of additional cylinder/canister connection/replacement/space/cost etc. In some embodiments, the washing fluid is warmed and/or dried, for example to reduce mist inside the treatment space and/or for washing the optical assembly. In some embodiments, when the washing gas is stored in a cylinder/canister, its water content - (measured in PPM - parts per million) - is much lower comparing to regular air.

According to some embodiments, the washing fluid is introduced into the body lumen with a rate of at least 4 liters per minute (air values), for example 5 liters per minute (air values), 7 liters per minute (air values), 10 liters per minute (air values) or any intermediate, smaller or larger value. In some embodiments, purge of low pressure fluid, for example low-pressure gas is introduced into the body lumen with a rate of up to 1 liter/minute (air values), for example 1 liter/minute (air values), 0.7 liter/minute (air values), o.5 liter/minute (air values) or any intermediate, smaller or larger value. In some embodiments, cryogenic fluid is introduced into the body lumen with a flow rate of at least 5 liters/ minute, for example 5 liter/minute, 7 liter/minute, 10 liter/minute or any intermediate, smaller or larger value.

An aspect of some embodiments relates to applying cryotherapy to a large target area or a large treatment space by a movable cryogenic inflow opening. In some embodiments, the cryotherapy device comprises a cryo inflow path with a movable nozzle, for example a nozzle that rotates to selected directions and/or at selected angles, for example to allow coverage of a large target area within the body lumen. In some embodiments, the nozzle rotates around an axis. Alternatively or additionally, the cryotherapy device comprises a swiveling element combined with a cryogenic nozzle. In some embodiments, the cryogenic fluid is sprayed through the nozzle which is capable of swiveling together with the swiveling component, thereby assisting with the coverage of an area or a volume within the body lumen.

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

It is noted that as used herein, the terms "body lumen", "treated lumen", "targeted lumen" and the like are interchangeable, unless specifically mentioned otherwise. Similarly, the terms "treated tissue", "treated area", "targeted tissue", "targeted area", "targeted region", "treated region" and the like are interchangeable, unless specifically mentioned otherwise. Further, elements regarding the distal end of the sensing means are intended to refer to the part of the sensing mean that senses the required parameter, even if the specific sensing element is not positioned particularly at the distal end of the sensing means.

Embodiments of the invention are directed to devices and systems for cryotherapy, which may be introduced into a body lumen through an endoscope or any other appropriate means (e.g., inserting a catheter directly into the lumen with no visualization or with outer imaging means, such as an ultrasound, CT and the like). The present invention is further directed to cryotherapy methods within body lumen. According to some embodiments, the cryotherapy methods include cryo-immunological processes.

It is noted that the term "endoscope" as used herein, is intended to include any type of known endoscope, as well as any type of sheath, catheter, tube or the like, that may be inserted into the body and through which any necessary working channels, visualization means and the like may be placed in the body lumen. Accordingly, the endoscope may include inherent visualization means or otherwise, the visualization means may be inserted through any type of sheath and the like, such that the visualization means may be changed during the procedure, i.e., several different kinds of visualization means may be used throughout the procedure. It is noted that the term "visualization means", or any other equivalent term used herein, is intended to include optic means, ultrasound, MRI, X-ray, and the like. Any other sensors may also be inherent in the endoscope or may be inserted therethrough into the body lumen. It is noted that the term "working channel", or any other equivalent term used herein, may be an integral path through an endoscope or a path available through any means, such as a sheath, catheter, tube or the like, through which any devices and/or sensors, such as visualization means, may be inserted into the body lumen.

According to some embodiments, the cryotherapy device includes two or more, channels, tubes, catheters, or the like, which allow both the injection of pressurized fluids and the evacuation of expanded fluids, wherein a pressurized fluid is injected into the lumen via at least one channel and the expanded fluid is evacuated from the lumen through at least one second channel. It is noted that catheters, tubes and the like are interchangeably used herein, unless specifically mentioned otherwise. According to some embodiments, at least one injection channel and at least one evacuation channel are inserted into the body lumen via an endoscope. According to some embodiments, any channel may pass or be fed through any one of the other channels. According to other embodiments, the same channel may be used for both injection and evacuation, using any appropriate type of sensor and/or dedicated algorithm to control flow direction, relative amounts and timing. It is noted that although pressurized and expanded fluids are related to above, any type of fluid may be introduced/released from the system according to any one of the above embodiments. It is noted that, as known in the art, the term "fluid" includes both gas and liquid.

Embodiments of the invention further include cryotherapy devices comprising at least one evacuation means as well as control means, by which the evacuation of the expanded fluid, and possibly the injection of the pressurized fluid, are controlled. The control means may be automatic, predefined, electronic, manual, and the like. The cryotherapy device may further include any number or type of sensors, wherein the control means receives data from those sensors and controls the evacuation and/or the injection according to predefined values, according to manual decision, according to values that may change during the process etc. Without such control means it is possible that pressure would accumulate in the targeted organ, possibly even causing rupture, since the evacuation may not be sufficient. Further, is may be necessary to control the targeted lumen's internal pressure, so that it does not drop beneath a certain, possibly pre-defined, value. Particularly, if the pressure is too low (e.g., below 10mBar), it may be difficult or even impossible to operate within the lumen and/or visually view the inside of the lumen, since at low pressures, the lumen may collapse. Accordingly, the control means may ensure that the pressure is not too high, causing damage to the lumen, or too low, causing the lumen to collapse. Further, the treated body lumen may have body liquid in it (e.g., urine in the bladder). That body liquid may freeze due to the cryotherapy process, possibly hindering the evacuation of the expended cryo fluid and therefore, it is important to include sensors in the system according to which the control means control the evacuation and possibly the injection, such that the treated lumen is not harmed. According to some embodiments, any number of sensors is inserted into the body lumen. According to some embodiments, any number of external sensors, such as ultra-sound, X-ray and the like may be used together with, or possibly instead of, internal sensors.

According to some embodiments, the inflow may be stopped when the pressure in the lumen is above between about 30mBar and 100mBar. According to some embodiments, the outflow evacuation may be continued until the pressure in the lumen is below between about 20mBar to 10mBar. According to some embodiments, the inflow may be stopped when the temperature in the lumen is below about 5-10⁰C. According to some embodiments, the inflow may be initiated when the temperature in the lumen is above about 15-20⁰C. According to some embodiments, both temperature and pressure, as well as any other appropriate parameters may be used for controlling the system.

Therefore, lumen, such as uterus, stomach, bladder, renal pelvis, urethra, and ureters can be treated according to this invention, even though gasses cannot be naturally evacuated therefrom safely. According to some embodiments, the dynamics of the system, which include both injection of cryo-fluid as well as the evacuation thereof, while monitoring and controlling both the injection and the evacuation during the entire process, prevents liquids or vapors within the lumen from freezing, thus preventing blockage and the like. According to some embodiments, in addition to the injection of the cryo-fluid, the system includes an additional flow injection source for introducing non-cryogenic fluids into the lumen when desired. For example, fluid may be injected into the lumen in order to preserve a certain volume of the lumen, which enables the user to perform necessary operations, possibly while visualizing the inside of the lumen. This may be necessary particularly between any two cryo cycles, or after the last cryo cycle. Further, fluid may be injected into the lumen in order to dry the lumen or areas therein. According to some embodiments, the lumen may be dried with dry and/or warm gas and further, the pressure in the lumen may be maintained above a predefined value, e.g., 10mBar with a gas, such as CO₂, air, argon, N₂ and the like. The combination of the injection, evacuation and control provide the necessary dynamics for the system to operate properly. Further, even lumen having small diameter entrances, such as ureters, may be treated, since the diameter of the injection and evacuation tubes may be relatively small (about 0.8-4mm overall diameter of all of the tubes together or about 4.0-9.0mm overall diameter of all of the tubes, particularly if including visualization means and the like), and further, one tube, or two adjacent tubes, may be used in order to further limit the diameter, since each additional tube used raises the overall diameter. It is noted that small diameters, e.g., about 0.8-9.0mm overall diameter, may include not only the injection and evacuation channels, but also any other channels, devices, sensors and the like, e.g., visualization means, that are introduced into the body lumen. According to some embodiments, the overall diameter of the inflow and outflow channels is between about 0.8-4.0mm. The diameter of the outer sheath may be between about 5.0-9.0mm, and the outer diameter of the visualization means is between about 3.0-5.0mm.

According to some embodiments, bladder, cervix, stomach, prostate, urethra, ureter or uterus conditions are treated. According to further embodiments, cancers of the bladder, cervix, stomach, prostate, urethra, ureter or uterus are treated. Benign growths may be treated as well. Pain conditions in the bladder, stomach, cervix, prostate or uterus may also be treated. According to further embodiments, any type of urinary tract conditions may be treated, including upper tract cancer, interstitial cystitis, bladder pain syndrome, overactive bladder (OAB) and the like.

The distal end of the cryotherapy device may include any number of holes, nozzles, fissures and the like, through which fluids may be injected/introduced/released into the body lumen. According to some embodiments, the fluids may be injected into the lumen together with any additional material, such as chemotherapy, immunotherapy and/or other chemical or biological agents. The additional material may be introduced at the same time, prior to and/or after the cryotherapy treatment for optimal results. The introduced fluid is related to herein also as a "coolant" a "cryo-fluid" and the like. It is noted that the coolant is able to ablate/freeze any desired treated region. According to some embodiments, the distal end of the cryotherapy device includes a nozzle designed for directly spraying fluid onto the targeted area and/or its surroundings. The distal end of the cryotherapy device may further include evacuation means in order to evacuate expanded fluid from a body lumen. According to some embodiments, the cryo-fluid is selected from liquid nitrogen, carbon dioxide (CO₂), nitrous oxide (N₂O), or any combination thereof. According to some embodiments, the cryo-fluid is selected from argon, nitrogen, krypton or any combination thereof, which may additionally be relevant when the cryo-fluid does not directly contact the body tissue. According to other embodiments, it may further include any additional material, such as chemotherapy, immunotherapy and/or other therapeutics such as chemical or biological agents. According to some embodiments, the additional material is introduced into the body lumen prior to, during and/or after the cryo treatment.

It is noted that the terms "targeted region", "targeted area", "treated region", "treated area", "treated lumen", "targeted lumen" and the like are interchangeable and are intended to include any type of condition that may be treated cryogenically, such as lesions (including cancerous and benign tumors, cysts, polyps and the like), nerves/nerve endings, and various symptoms, even when their specific origin is not fully understood.

According to other embodiments, the coolant remains within the injection channel (e.g., a catheter, tube and the like) and expands therein, so that the cold temperature is transferred into the tissue by a mediating distal section of the cryotherapy device. According to such embodiments, since the coolant remains in the injection channel, there is no need to evacuate the coolant from the body lumen.

According to some embodiments, the mediating distal section of the cryotherapy device is a cryo-balloon, wherein the cryo-balloon is introduced into the treated lumen via an endoscope. The cryo-balloon may be non-compliant, such that it withstands high pressures. If the balloon is non-compliant, the size of the balloon is chosen according to the size of the lumen into which it is inserted or according to shape of a treated area. According to other embodiments, the balloon may be semi-compliant or compliant. According to further embodiments, different sections of the cryo-balloon may have different degrees of compliance. The different parameters of the balloon, including the compliance of the various sections thereof, the shape of the cryo-balloon, the size of the cryo-balloon, etc., may change according to the treated lumen, the targeted area in the lumen, and the like. The balloon may be circular, oval, tubular, or have any flat, or partially flat surfaces. The balloon may also be wide in certain areas and narrow in others, depending on the intended use. When inflated, or partially inflated, the balloon may locally contact only the treated area, not the entire lumen in which the balloon is used. According to other embodiments, the inflation of the cryo-balloon, e.g., in the urethra or ureters, may bring the balloon, or parts thereof, into contact with the entire circumference of the treated area. According to some embodiments, external force, e.g., movements by the user of the device, possibly together with inflation, bring, at least part of the cryo-balloon, into contact with the treated area.

According to some embodiments, the diameter or average diameter of the cryo-balloon, when folded (when inserted through an endoscope or a working channel) is below 2.5mm. According to some embodiments, the diameter or average diameter of the cryo-balloon, when folded (when inserted through an endoscope or a working channel) is below 2.0mm. According to some embodiments, the diameter or average diameter of the balloon, when folded (when inserted through an endoscope or a working channel) is below 1.5mm.

Further, the balloon may be contacted with the targeted area by balloon expansion, partial balloon expansion, and/or by movements of the balloon that may be controlled externally by the user and/or by any appropriate mechanical and/or electronic means. The balloon expansion and/or movements may further be controlled according to data received from any internal or external sensors.

It is noted that the injection and evacuation channels are related to herein also as catheters, tubes and the like.

According to some embodiments, the cryotherapy device may include a flexible or a kink resistant catheter, such as braided or coiled tube, which may allow sharp angular movement, thus enabling targeted areas to be targeted easily regardless of their position within the body.

According to some embodiments, the cryotherapy device includes a single nozzle at the distal end of the device, allowing the operator to perform the treatment within the endoscope's field of view. According to other embodiments, the device includes at least two nozzles, wherein any two nozzles may be positioned in the same or different directions, relative to one another. For instance, any one of the nozzles may be pointed downward, in the distal direction, while other nozzles may be pointed sideways, at any desired angle and distance from the distal end of the device, thereby allowing a multi directional treatment that may treat different parts of the same targeted region, several targeted areas at once, etc. According to some embodiments, one or more of the nozzles has an opened and closed configuration. Further, any one of the nozzles may be partially opened or closed for any time period required. According to some embodiments, any number of nozzles may be electronically controlled, such that the practitioner using the device may use any number of the existing nozzles at any time point, according to the desired treatment. According to some embodiments, the user pre-defines the specific nozzles to be used. According to other embodiments, during the procedure, any one of the nozzles may be opened, partially opened, or closed at any time point, as desired, by any appropriate means, including designated sensors, computerized applications, user commands and the like.

According to some embodiments, a folded component, such as a cryo-balloon may be attached to any part of the distal end of the cryotherapy device. Accordingly, injected cryo-fluid, possibly together with any other appropriate pressure source, inflates the balloon, which in turn freezes at least part of the treated tissue. According to some embodiment, prior to the injection of cryo-fluid, the balloon is inflated by any other appropriate pressure source, allowing the preparation of the balloon for cryo-treatment, checking or testing the system or certain parameters thereof, and the like. The inflation of the balloon, including inflation rate, size etc. may be controlled by any number of sensors, as detailed above regarding the injection through nozzles. Further, any one of the nozzles may be attached on the outside of the nozzle to a cryo-balloon, so that when the cryo-fluid exits the nozzle it inflates the balloon that is attached to the outside of the nozzle.

Thus, according to some embodiments, the cryo-fluid is injected directly into the body lumen, i.e., it comes in direct contact with at least part of the tissue of the body lumen. According to such embodiments, the cryo-fluid is injected directly into the body lumen through any number of nozzles, holes, fissures and/or valves, as detailed herein. Further, if the cryo-fluid is directly injected into the body lumen, it is also directly evacuated therefrom, by any means detailed herein. According to other embodiments, cryo-fluid may be indirectly introduced into the body lumen, such that it does not come in direct contact with the tissue of the body lumen; rather, it is injected into any appropriate component that is positioned inside the body lumen, e.g., a catheter, a cryo-balloon, a tube or the like, which does not include an opening into the body lumen. The cryo-fluid expands within that component, thereby cooling at least part of the treated tissue. The cryo-fluid may then be indirectly evacuated from the body lumen, i.e., it is evacuated from the component into which it was injected. The cryo-fluid may be evacuated by active or passive means.

According to some embodiments, the folded component is a cryo-balloon which is being inflated and/or filled with coolant within the lumen in order to treat the desired area.

According to other embodiments of the current invention, any one of the endoscope's existing 'in-flow' and 'out-flow' channels may be used in order to introduce materials into or evacuate materials from the body lumen (e.g. evacuating cryotherapy expanded fluids).

According to some embodiments, the cryotherapy procedure is performed in combination with any other type of treatment, including intraluminal chemotherapeutic therapy and/or immunotherapy agents. It is noted that the cryo-fluid may be injected together with any other active components. As noted above, the additional active components may be introduced at the same time, prior to, and/or after the cryo-treatment.

The tissue cells that were frozen by the cryo-treatment are removed from the body, *inter alia,* through the lymph system, wherein an immunological response may be triggered, which may act against such cells in any part of the body, not only the part treated by cryo-therapy. According to some embodiments, the introduction of additional active components into the body lumen before or during the cryo-treatment, may cause the treated tissue to react differently to the cryo-treatment, and further, when introduced into the lymph system, the cells together with the additional active ingredient, may cause an enhanced immune response.

The additional active ingredient may be any immunological, chemical, chemotherapeutic, biological or nanoparticle entity, including, though not limited to mitomycin C, doxorubicin, dendritic cells, and the like.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary cryotherapy system

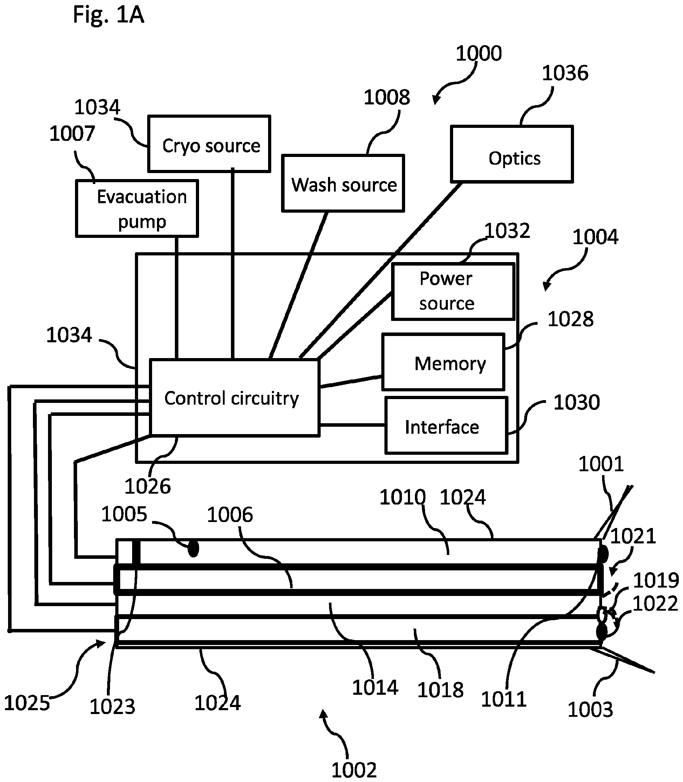

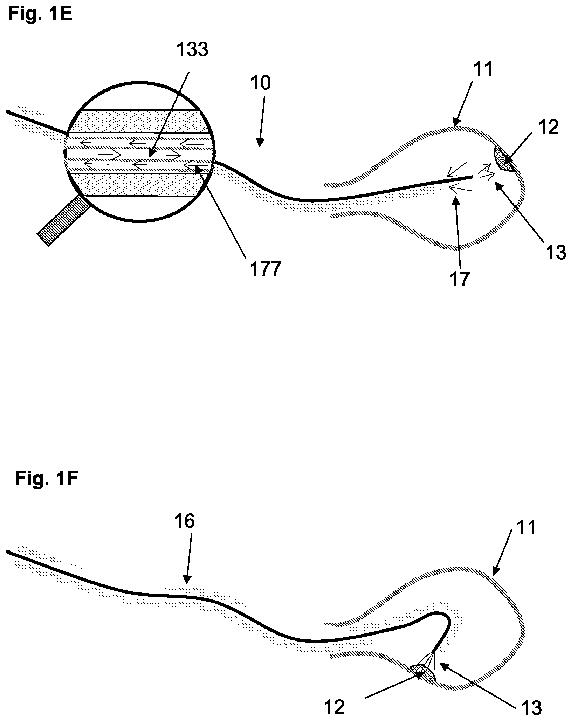

According to some exemplary embodiments, a cryotherapy system comprises a cryotherapy probe configured to be inserted at least partly into a body lumen, and a control unit connected to the cryotherapy device. In some embodiments, the control unit is positioned outside the body. Reference is now made to FIG. 1A depicting a cryotherapy system, according to some exemplary embodiments of the invention.

According to some exemplary embodiments, cryotherapy system 1000 comprises a cryotherapy probe, for example cryotherapy device 1002, and a control unit 1004 connected to the cryotherapy device 1002. In some embodiments, the cryotherapy device 1002 is an elongated probe having a distal end 1021, optionally shaped and sized to be introducible into a body lumen and to face a target region in the internal surface of the lumen, and a proximal end 1025, optionally positioned outside the body lumen. In some embodiments, the cryotherapy device 1002 is cylindrical, and has a diameter at the distal end 1021 of the cryotherapy device and the sheath around it in a range of 3 to 15 mm, for example 3 mm, 5 mm, 7 mm, 9 mm or any intermediate, smaller or larger value. In some embodiments, the diameter of a cryotherapy device not including a sheath is in a range of 0.3 to 5 mm, for example 0.5 mm, 1 mm, 2 mm or any intermediate, smaller or larger value. In some embodiments, the cryotherapy device 1002 is shaped and sized to be introduced into the body lumen through a working channel of an endoscope. Alternatively, the cryotherapy device 1002 is introduced through sheath 1024 into the body lumen.

According to some exemplary embodiments, the cryotherapy device 1002 comprises at least one flow path, for example a channel passing through a lumen of said cryotherapy device from said proximal end 1025 to the distal end 1021. In some embodiments, the at least one flow path is used as an inflow path towards the distal end 1021 and into the body lumen, and as an outflow path from the body lumen towards the proximal end 1025. In some embodiments, the cryotherapy device 1002 comprises at least one inflow path, for example inflow channel 1006 for delivering fluid or gas into the body lumen. Additionally or optionally, the cryotherapy device 1002 comprises at least one outflow path, for example outflow channel 1010 for evacuating fluids, particles and/or gas from the body lumen.

According to some exemplary embodiments, the outflow path, for example outflow channel 1010 comprises at least one outflow flow regulator, for example valve 1023 for controlling the passage of material, for example gas or fluids through the outflow path. In some embodiments, the at least one valve comprises at least one check valve, configured to be opened, optionally passively, when a pressure inside the body lumen exceeds a pre-determined value. Optionally, the outflow flow regulator is positioned near a proximal end 1025 of the cryotherapy device 1002 positioned outside the body.

According to some exemplary embodiments, the inflow channel 1006 comprises at least one forward facing cryo-nozzle configured to spray cryogenic fluid, for example cryogenic gas and/or cryogenic liquid towards a selected target region in the internal surface of the lumen. Optionally, the cryogenic fluid is stored in high-pressure. In some embodiments, and without being bound by any theory, the expansion of the high-pressure cryogenic fluid within the body lumen, lowers the pressure of the fluid rapidly which results with a cryogenic effect inside the body lumen, for example the Joule-Thomson effect.

According to some exemplary embodiments, the cryo-nozzle is an adjustable cryo-nozzle configured to spray the cryogenic fluid is an angle smaller or larger than 90 degrees to the target region. Alternatively, the cryo-nozzle is a fixed angle cryo-nozzle, fixed in an angle different than 90 degrees, for example 15 degrees, 30 degrees, 45 degrees, 55 degrees or any intermediate, smaller or larger angle different than 90 degrees. In some embodiments, the adjustable cryo-nozzle is configured to control the amount of the sprayed cryogenic fluid released through the nozzle to the lumen, for example by adjusting the opening diameter of the cryo-nozzle.

According to some exemplary embodiments, the cryotherapy device 1002 comprises at least one optical assembly, for example optics path, optionally optics channel 1018 for delivering images and/or visual signals of the tissue facing the distal end 1021 of the cryotherapy device 1002, to an optic sensor located outside the body, for example an optic sensor in the control unit 1004, or to an external optical assembly, for example an optical assembly of a different control unit. Alternatively, the optical assembly comprises at least one optic sensor, for example optic sensor 1022 at the distal end 1021 of the cryotherapy device 1002, configured to sense visual signals of the tissue facing the distal end 1021 of the cryotherapy device 1002, for example images and/or visual signals of a target region within the body lumen. Alternatively, the optical assembly comprises a sensor optionally an Ultra-Sound (US) sensor or a magnetic resonance sensor (MR). In some embodiments, the cryotherapy device 1002 comprises at least one illumination source, for example illumination source 1019. In some embodiments, the illumination source 1019 is positioned at the distal end 1021 of the cryotherapy device, optionally facing the tissue of the body lumen. Alternatively, the at least one light source is positioned inside a channel within the cryotherapy device, for example optics channel 1018. In some embodiments, the optics channel 1018 is or comprises an optical fiber, a group of optical fibers or an optical fiber cable. In some embodiments, when the optics channel 1018 is or comprises an optical fiber, a group of optical fibers or an optical cable, the light source is positioned outside the body, and optionally the optical sensor is positioned outside the body.

According to the invention, the cryotherapy device 1002 comprises at least one flow path, for example washing channel 1014 for insertion of a washing gas, optionally a low pressure gas into the body lumen. In some embodiments, the washing gas is used to inflate the body lumen, for example to allow better visualization of the internal surface of the lumen and/or to allow better access to desired regions, optionally regions selected for cryotherapy, within the body lumen. Additionally or alternatively, the washing gas is sprayed through a washing gas nozzle into the treatment region, for example to blow away condensed particles and/or clouds of condensed particles formed by the interaction of the cryogenic gas with the humidified environment inside the body lumen.

According to the invention, the washing channel 1014 comprises at least one forward-facing wash nozzle at the distal end of the cryotherapy device, facing the tissue. Optionally the forward-facing wash nozzle is configured to spray gas, for example low-pressure gas into a space between the cryotherapy device 1002 and the target region. In some embodiments, the forward-facing nozzle is an adjustable wash nozzle, for example to allow spraying wash gas sideways or in an angle to the target region. In some embodiments, the forward-facing wash nozzle sprays wash gas into a FOV of the optic sensor 1022 or into the field of view of the optics channel 1018.

According to the invention, the washing channel 1014 comprises a front washing opening and a sideways washing opening, positioned near the distal end 1021 of the cryotherapy device 1002. In some embodiments, the cryotherapy device comprises at least one washing director at the washing opening, for example a nozzle and/or a deflecting surface configured to direct washing fluid towards the optical assembly, for example a lens and/or into FOV of the optic sensor 1022 or into the FOV of the optics channel 1018. Optionally, the at least one washing director is an adjustable washing director, for example configured to allow spraying wash gas in an angle to the FOV.

According to some exemplary embodiments, at least one washing flow regulator on the washing inflow path is configured to adjust the amount of the released washing fluid through the washing opening, optionally in a response to a signal received from a control unit.

According to some exemplary embodiments, the cryotherapy device 1002 comprises at least one foldable element, for example foldable elements 1001 and 1003. In some embodiments, the at least one foldable element is configured to be folded inside an inner lumen of the cryotherapy device 1002, and to be unfolded, optionally near the distal end 1021 of the cryotherapy device 1002. In some embodiments, the at least one foldable element is unfolded to a space between the distal end 1021 of the cryotherapy device 1002 and the target region. In some embodiments, this space is termed a treatment space. In some embodiments, the foldable element comprises an extension, a cone or a dress. In some embodiments, the foldable element is unfolded within the treatment space, for example to determine a distance or an angle between the distal end of the cryotherapy device and the target region. Alternatively, the folded element is unfolded within the treatment space, for example to determine the distance and/or angle between the cryo-nozzle and the target region. In some embodiments, at least two foldable elements or a cone shaped foldable element is unfolded to narrow the treatment space, for example by creating a micro-environment within an inner space formed by the unfolded foldable element.

According to some exemplary embodiments, the control unit 1004 comprises at least one control circuitry, for example control circuitry 1026. In some embodiments, the control circuitry controls the flow of cryogenic fluid through the inflow path, for example inflow channel 1006. In some embodiments, the control circuitry 1026 controls the flow of the cryogenic fluid by controlling at least one cryo flow regulator, for example a valve positioned inside the inflow path and/or at least one valve between a cryo source 1034 and the inflow channel 1006, for example at least one valve in a tube connecting the cryo source 1006 with the control unit 1004 and/or at least one valve in a tube connecting the cryo source and the inflow path. Alternatively or additionally, the control unit 1004 controls at least one valve of the cryo-source, for example an outlet valve of the cryo-source.

According to some exemplary embodiments, the control circuitry 1026 controls the flow of a washing fluid through a wash inflow path, for example washing channel 1014. In some embodiments, the control circuitry 1026 controls the flow of the washing fluid by controlling at least one washing flow regulator, for example a valve positioned on the wash inflow path, for example washing channel 1014. Alternatively or additionally, the control circuitry 1026 controls the flow of the washing fluid by controlling at least one flow regulator between a wash source 1008 and the washing channel 1014, for example at least one valve in a tube connecting the wash source 1008 with the control unit 1004 and/or at least one valve in a tube connecting the wash source 1008 and the washing channel 1014. Alternatively or additionally, the control circuitry 1026 controls at least one valve of the wash-source, for example an outlet valve of the wash source 1008.

According to some exemplary embodiments, the control circuitry 1026 controls the flow of fluid, for example gas and/or liquid out from the body lumen through the outflow path, for example outflow channel 1010. In some embodiments, the control circuitry controls the flow through outflow channel 1010 by controlling an outflow flow regulator, for example an outflow valve inside or near a proximal end of the outflow channel, for example valve 1023. Alternatively or additionally, the control circuitry 1026 controls the activation of an evacuation pump 1007, for example a vacuum pump connected to the outflow channel. In some embodiments, the evacuation pump 1007 is used to actively evacuate fluid, for example liquid or gas out from the body lumen through the outflow path of the cryotherapy device. Optionally, the control circuitry 1026 controls at least one evacuation flow regulator, for example an evacuation valve on a tube connecting the evacuation pump 1007 and the outflow channel.

According to some exemplary embodiments, the control circuitry controls an optical assembly, for example an optic sensor positioned on the cryotherapy device 1002, for example optic sensor 1022. Alternatively or additionally, the control circuitry 1026 controls at least one optic sensor in the control unit 1002. In some embodiments, the control circuitry 1026 controls the opening or closing of an optics channel 1018, optionally by controlling an aperture within the optics channel 1018. In some embodiments, the control circuitry controls the activation of optics 1010, for example visualization means.

According to some exemplary embodiments, the cryotherapy device 1002 comprises at least one sensor 1011 located at the distal end 1021, for example to sense at least one environmental parameter of the body lumen. In some embodiments, the environmental parameter comprises temperature, pressure, and/or humidity level or any other environmental parameter. Alternatively or additionally, the cryotherapy device 1002 comprises at least one sensor positioned at least partly within the outflow path, for example sensor 1005, for sensing the environmental parameter.

According to some exemplary embodiments, sensor 1011 and/or sensor 1005 are electrically connected to the control circuitry 1026. Alternatively or additionally, the control circuitry 1026 is electrically connected to one or more sensors positioned inside or outside the body. In some embodiments, the one or more sensors are positioned near or at a distance from the cryoablation target region. Alternatively In some embodiments, at least one of the sensors transmits the measured environmental parameter values to the control circuitry 1026. In some embodiments, the control circuitry stores the measured values in memory 1028. In some embodiments, the memory 1028 comprises predetermined values of at least one environmental parameter, for example maximal pressure values, maximal temperature values or any other predetermined environmental parameter value or indications thereof. In some embodiments, memory 1028 comprises at least one cryotherapy protocol, values of at least one cryotherapy parameter or indications thereof. In some embodiments, the memory comprises log files related to the operation of the cryotherapy device 1002 or the operation of the cryotherapy system 1000.

According to some exemplary embodiments, the control unit 1002 comprises at least one interface 1030 electrically connected to the control circuitry 1026. In some embodiments, the interface 1030 comprises a sound source and/or a display. In some embodiments, the control circuitry 1026 signals the interface 1030 to generate at least one indication, for example a human detectable indication to a user of the cryotherapy device or the cryotherapy system. In some embodiments, the control circuitry 1026 signals the interface 1030 to generate an alert, for example when the measured environmental parameter values inside the body lumen exceed a pre-determined value.

According to some exemplary embodiments, the control circuitry 1026 is electrically connected to at least one cryo flow regulator on the cryo inflow path, for example a valve. In some embodiments, the control circuitry regulates the flow through the cryo inflow path, for example reducing flow within the inflow path, for example stopping flow within the inflow path and/or increasing flow within the inflow path for example by delivering a signal to the cryo flow regulator. In some embodiments, the control circuitry regulates the cryogenic fluid flow within the cryo inflow path, for example when the pressure within the body lumen is higher than a pre-determined pressure value. Alternatively, the control circuitry regulates the cryogenic fluid flow within the cryo inflow path, for example when the temperature inside the body lumen is below a pre-determined temperature value.

According to some exemplary embodiments, the control circuitry 1026 controls at least one purge flow regulator on the cryo inflow path. In some embodiments, the purge flow regulator is configured to control flow of low-pressure fluid through the cryo inflow path and optionally through the cryo-nozzle into the body lumen, for example when the cryogenic fluid flow is stopped, reduced or regulated by the control circuitry.

According to some exemplary embodiments, the control circuitry 1026 is connected to the outflow flow regulator, for example a valve. In some embodiments, the control circuitry regulates the outflow flow regulator, for example by opening the outflow path, for example when the pressure within the body lumen is higher than a predetermined value and/or when the temperature is lower than a predetermined temperature value. According to some exemplary embodiments, the control unit 1004 comprises a power source, for example power source 1032. Alternatively, the control unit 1004 is connected to an external power supply by electrical wiring. In some embodiments, the power source comprises a battery, optionally a rechargeable battery.

According to some exemplary embodiments, a control unit, for example control unit 1004 is connected to at least one inflow path of a cryotherapy device, configured to allow fluid flow from a fluid flow source into a body lumen. Optionally, the control unit is connected to at least one inflow flow regulator on the inflow path, configured to regulate fluid flow through the inflow path and into the body lumen.

In some embodiments, the control unit, for example control unit 1004 is connected to at least one outflow path of a cryotherapy device, configured to allow evacuation of fluid out from the body lumen. Optionally, the control unit is connected to at least one outflow regulator, for example a valve or a check valve on the outflow path, configured to regulate the evacuation of fluids from the body lumen through the outflow path.

In some embodiments, the control unit 1004 controls the inflow regulator and the outflow regulator to regulate pressure levels within the body lumen to reach a pressure level lower than a predetermined value. Alternatively or additionally, the control unit 1004 controls the inflow regulator and the outflow regulator to regulate temperature levels within the body lumen to a temperature level higher than a pre-determined temperature value.

According to some exemplary embodiments, the wash source 1008 comprises a fluid container, for example a gas container or a liquid container. In some embodiments, the cryo-source 1006 comprises a container of a cryogenic fluid, for example liquid nitrogen, liquid carbon dioxide, or any other cryogenic compound that can be stored as liquid or gas in high or low pressure in room temperature.

According to some exemplary embodiments, at least one of the wash source 1008, optics 1036, cryo source 1006 and/or evacuation pump 1007 is part of the cryotherapy system. Alternatively, the wash source 1008, optics 1010, cryo source 1006 and/or evacuation pump 1007 are external elements, optionally connected to the system 1000.

### Exemplary cryotherapy process

According to some exemplary embodiments, a cryotherapy process is used for ablating material, for example tissue, lesion, tumor from the internal surface of a body lumen, for example the bladder. In some embodiments, during the cryogenic ablation, also termed herein in some embodiments as cryoablation, some of the cells or tissue in the internal surface of the bladder are ablated, for example in a case where the cells or tissue are cancerous or tumorigenic cells. In some embodiments, the expanded coolant material causes some of the cells and/or part of a tissue in the internal surface of the body lumen to die in a rapid ablation effect, for example immediate necrosis or a slow ablation process, for example dismantled by body processes as apoptosis.

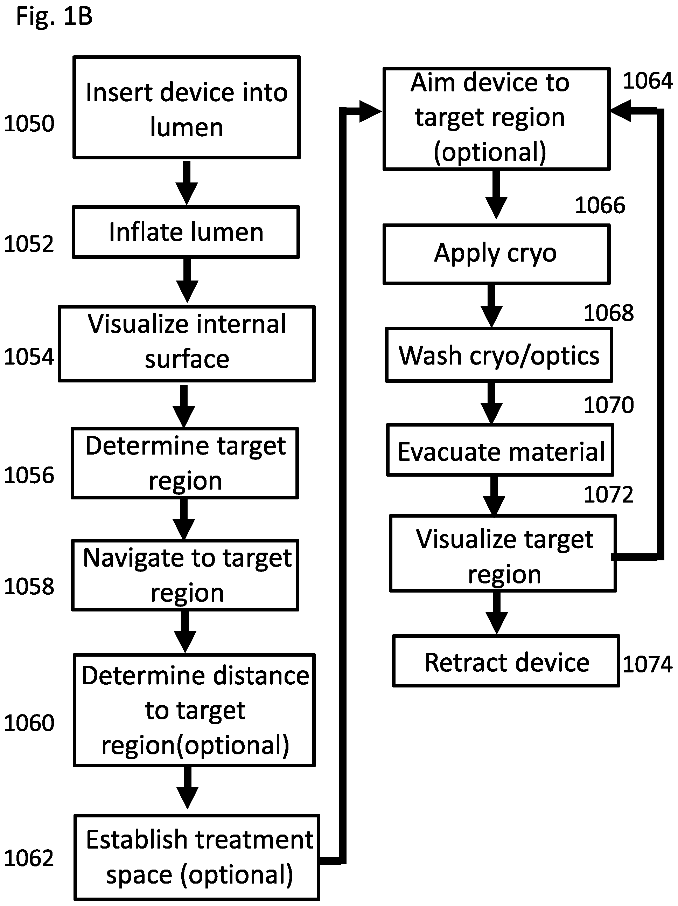

Reference is now made to FIG. 1B depicting a cryotherapy process, according to some exemplary embodiments of the invention.

According to some exemplary embodiments, at least part of a cryotherapy device is introduced into a body lumen at 1050. In some embodiments, the cryotherapy device, for example cryotherapy device 1002 of a cryotherapy system 1000 shown in FIG. 1A, is inserted to the body lumen through a working channel of an endoscope. Alternatively, the cryotherapy device is introduced into the body lumen through a sheath, for example an endoscope sheath or a dedicated sheath of the cryotherapy device.

According to some exemplary embodiments, the body lumen is inflated at 1052. In some embodiments, the device is inserted at least partially into the body lumen to allow inflation of the lumen. In some embodiments, the body lumen is inflated to allow better inspection of the internal surface of the body lumen, for example by an optical sensor optionally a camera of the cryotherapy device or an optical sensor of a different device. Alternatively or additionally, the body lumen is inflated to allow better access to different regions or selected target regions within the body lumen. In some embodiments, the body lumen is inflated by passing wash fluid, optionally a low pressure gas into the body lumen. In some embodiments, during the inflation of the body lumen, the pressure inside the body lumen is monitored by at least one sensor positioned within the body lumen or inside an outflow channel of the cryotherapy device, for example outflow channel 1010. Alternatively or additionally, the pressure is monitored by measuring the pressure or changes in pressure inside a washing flow path, optionally by a pressure sensor positioned in the washing flow path. In some embodiments, the pressure is monitored by at least one sensor, for example a pressure sensor positioned outside the body. In some embodiments, the inflation of the body lumen is controlled by a control circuitry, for example control circuitry 1026, optionally based on the signals received from the at least one sensor. In some embodiments, the body lumen is inflated to a desired or selected inflation degree.

According to some exemplary embodiments, the body lumen is inflated by washing fluid passing towards the optical assembly, for example to keep the optical assembly, for example lens clean and/or dry and/or at certain temperature. Alternatively or additionally, the body lumen is inflated by purge flow of a low-pressure fluid through the cryo-nozzle into the body lumen.

According to some exemplary embodiments, the internal surface of the body lumen is visualized at 1054. In some embodiments, the internal surface is visualized by at least optical sensor of the cryotherapy device, for example optical sensor 1022 which is optionally a camera. Alternatively, the internal surface of the body is visualized by at least one optical sensor positioned outside the body lumen which receives optical signals through an optics channel inside the cryotherapy device, for example optics channel 1018. In some embodiments, the optical signals are received by an optics unit, for example optics 1010, optionally positioned outside the body. In some embodiments, when the body lumen is visualized, at least one light source, for example a light source positioned inside the body lumen on the cryotherapy device or a light source positioned outside the body is activated.

According to some exemplary embodiments, a target region for cryotherapy is determined at 1056. In some embodiments, the target region is determined based on analysis of images showing the internal surface of the body lumen. Optionally, the target area is determined based on imaging results and/or other clinical data.

According to some exemplary embodiments, the cryotherapy device or the distal end of the cryotherapy device, for example distal end 1021 are navigated to a selected distance from the determined target region, for example to a distance of up to 50 mm from the target region, for example to a distance of 5mm, 10mm, 15mm, 20mm, 25mm or any intermediate smaller or larger distance from the target region. Additionally or alternatively, the cryotherapy device or the distal end are navigated to a selected angle with the determined target region. In some embodiments, the distal end of the cryotherapy device is navigated passively by turning a distal end of an endoscope in a selected angle. Alternatively or additionally, at least part of the cryotherapy device is actively navigated to the desired target region and/or to a desired angle between an opening of the cryo inflow path and tissue in a selected target region.

According to some exemplary embodiments, the distance to the target region is determined at 1060. In some embodiments, the distance between the distal end of the cryotherapy device and the target region is determined by unfolding a ruler or an attachment with a known length, for example a wire and touching the target region in the internal surface of the body lumen. In some embodiments, the distance is determined optically by a light beam, for example a laser light beam. Optionally, the distance is measured by a light distance meter, for example a laser light meter connected to the cryotherapy device optionally at the distal end of the cryotherapy device.

According to some exemplary embodiments, the distance between the cryotherapy device, for example the distal end of the cryotherapy device and the target region is determined by releasing a jet of air or gas from an inflow path of the cryotherapy device, for example the wash channel 1014 or from the cryo-nozzle towards the target region, and visualizing the effect of the air or gas jet on the tissue. In some embodiments, the air or gas jet form an indentation in the contact site with the inner surface of the body lumen. Optionally, the distance from the contact site and/or the angle between the cryotherapy device and the target region are determined by visualizing the depth, size, and/or shape of the indentation. In some embodiments, at least one parameter value of the cryotherapy process is determined based on the distance and/or angle, for example the duration of the cryotherapy and/or the pressure and/or amount of cryogenic fluid needed to reach the desired results of the treatment.

According to some exemplary embodiments, the treatment space is established at 1062. In some embodiments, the treatment space is established by unfolding at least one foldable element, for example a cone or a dress that enclose a treatment space between the cryotherapy device and the target region within the body lumen. In some embodiments, the treatment space is established to reduce the damage to tissue surrounding the target region due to the release of the cryogenic fluid. Alternatively and/or additionally, the treatment space is established to allow better control on micro-environmental parameters within a limited volume of the treatment space.

According to some exemplary embodiments, the cryotherapy device is aimed to the target region at 1064. In some embodiments, the distal end of the cryotherapy device is aimed to the target region. Alternatively or additionally, a nozzle, optionally an adjustable nozzle configured to spray the cryogenic fluid is aimed to the target region.

According to some exemplary embodiments, the cryogenic fluid is applied at 1066. In some embodiments, the cryogenic fluid is applied, for example sprayed through the nozzle at the target region. In some embodiments, the cryogenic fluid is applied while the body lumen is inflated, optionally by air or gas that enters the body lumen through a different inflow channel. Alternatively, when the cryogenic fluid is applied, the body lumen is inflated only by application of the cryogenic fluid and optionally by the expansion of the cryogenic fluid inside the lumen. Alternatively, the body lumen is inflated only by the washing fluid that optionally keeps the optics dry and/or in a close to constant temperature.

According to some exemplary embodiments, cryo wash and/or optics wash is applied at 1068. In some embodiments, the washing fluid, for example gas optionally a low pressure gas is applied between spraying sessions of the cryogenic fluid. Alternatively wash is applied during the spraying of the cryogenic fluid. Optionally, the washing fluid is used for inflating the body lumen. In some embodiments, the wash is applied through a wash inflow path, for example wash channel 1014 shown in FIG. 1A. In some embodiments, the wash is applied to a space in front a distal opening of the optics channel 1018 and/or to a space in front the optical sensor 1022 and/or light source 1019, for example to clear and/or push away particles formed by the cryogenic fluid inside the body lumen. Alternatively or additionally, the wash is applied to the space between the cryo nozzle and the target region, to clear the particles. In some embodiments, the particles are cleared to allow better visualization of the cryogenic fluid application at 1066 on the target region. In some embodiments, the washing fluid comprises warm gas or warm fluid with a temperature higher than 15 Celsius degrees, for example 15 Celsius degrees, 20 Celsius degrees, 25 Celsius degrees or any intermediate, smaller or larger temperature value. In some embodiments, the warm fluid is used, for example to increase the temperature within the body lumen and optionally to reduce humidity.

According to some exemplary embodiments, material from within the body lumen is evacuated at 1070. In some embodiments, some of the material for example, liquid, particles, gas is evacuated from body lumen through an outflow channel within the cryotherapy device, for example outflow channel 1010 shown in FIG. 1A. In some embodiments, the material is passively evacuated, for example by opening a valve, for example a check valve in the outflow path when the pressure inside the body lumen exceeds a predetermined value. Alternatively or additionally, the material is actively purged by actively generation of negative pressure within the outflow path, optionally by activation of a vacuum pump. In some embodiments, the material is evacuated between or during the spraying of the cryogenic fluid at 1066. In some embodiments, the material is evacuated when the pressure exceeds a predetermined pressure value. Alternatively, the material is evacuated when the temperature within the temperature inside the body lumen is lower than a predetermined value.

According to some exemplary embodiments, the target region is visualized at 1072. In some embodiments, the target region is visualized after the spraying of the cryogenic fluid, for example to determine the effect of the cryogenic fluid on the target region. Optionally, the target region is visualized to determine the success of the cryotherapy session. In some embodiments, if the treatment was not effective then the device is optionally aimed at a different target region and/or is placed in a different angle and/or distance relative to the target region.

According to some exemplary embodiments, the cryotherapy device is retracted from the body lumen at 1074. In some embodiments, the cryotherapy device is retracted when the cryotherapy session reached a desired goal, for example cryoablation of the target region. Optionally, the device is retracted to allow re-navigation to a different region within the body lumen.

### Exemplary cryotherapy device inside a body lumen

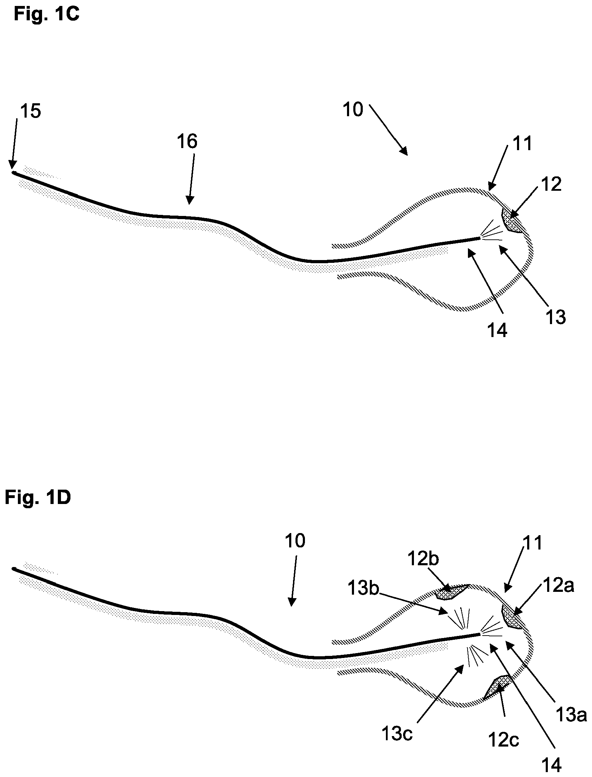

Reference is now made to FIGS. 1C to IF, which illustrate schematic lengthwise sectional views of embodiments of a cryotherapy device, for example cryotherapy device 10 during cryotherapy procedure that is fed into the treated lumen 11 through endoscope 16, according to some exemplary embodiments of the invention.

According to some exemplary embodiments, for example as shown in Figure 1C, jet 13, which may comprise an expanded high pressure (or compressed) cryogenic fluid, is sprayed from distal end 14 of device 10 and is directed at treated region 12, which may be a benign or cancerous tumor, a cyst, a polyp a free nerve ending, or a region in which certain symptoms (pain and the like) occur, even if their specific origin is not known, optionally causing the treated region 12 to be frozen, and thereby, treated or ablated.

According to some exemplary embodiments, for example as presented in Figure 1D, body lumen 11 may include more than one targeted region, e.g., regions 12a, 12b and 12c. In some embodiments, accordingly, the distal end 14 of device 10, may include more than one nozzle (not shown), wherein the jets 13a, 13b and 13c originating from each nozzle are directed at each one of the targeted regions. According to some exemplary embodiments, the distal end 14 includes numerous nozzles, each of which may have an opened and closed configuration. The nozzles may be utilized, i.e., opened, according to their relative position to the targeted region, such that the nozzles pointing at targeted region are opened and therefore, cryogenic fluid exiting those nozzles is sprayed directly at the targeted regions.

According to some exemplary embodiments, for example as presented in Figure 1E, device 10 includes both inflow 133, which provides jet 13 (or any number of jets, as presented in Figure 1B), and outflow 177, for evacuating, e.g. expanded fluid 17 from lumen 11. In some embodiments, the enlarged portion of device 10 shows that the tube for inflow 133 may be positioned within the tube for outflow 177; however, according to different embodiments, they may be positioned in any relation to one another. In addition, there may be any number of outflow and inflow tubes.

According to some exemplary embodiments, for example as illustrated in FIG. IF, endoscope 16 may be used also at sharp angles, in order for jet 13 to reach treated region 12, no matter where treated region 12 is positioned in body lumen 11.

### Exemplary cryotherapy system with inflow and outflow means

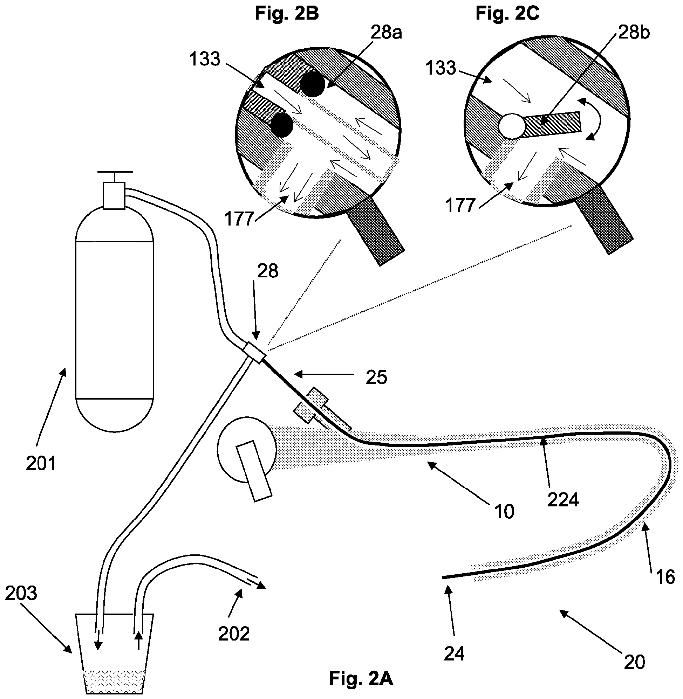

Reference is now made to FIGS. 2A, 2B and 2C, which illustrate schematic lengthwise sectional views of a cryotherapy system, specifically presenting the means by which fluids flow in and out of the treated area in accordance with some exemplary embodiments of the present invention. According to some exemplary embodiments, a cryotherapy system, for example cryotherapy system 20 includes a source of pressurized coolant 201 and evacuation means 202. In some embodiments, evacuation means 202 may be dedicated to cryotherapy system 20, general evacuation means, an outlet to the surrounding atmosphere, or any other appropriate means by which material, e.g., expanded fluids, may be evacuated from the system. According to some embodiments, evacuation means 202 includes suction provided by any appropriate pump, vacuum, or the like. According to some embodiments, evacuation means 202 may be attached to the proximal end of cryotherapy device 10. According to some embodiments, the evacuation means 202 are attached to cryotherapy device 10 via junction 28.

According to some exemplary embodiments, for example as illustrated in FIG. 2A and 2B, a cryotherapy catheter 224 is inserted into the endoscope 16 such that its distal end 24 reaches the endoscope's distal end while the proximal end of cryotherapy catheter 25 remains outside of the proximal end of endoscope 16. According to some embodiments, proximal end 25 is connected to junction 28 as detailed herein. According to some embodiments of the current invention simultaneous inflow 133 and outflow 177 are allowed (FIG. 2B). In some embodiments, seal 28a may prevent potential mixture between the two flows. In some embodiments, this may be necessary when the two flows are oriented in a coaxial manner inside the endoscope, such that there is a need to separate the flows outside the endoscope. Optional suction trap 203 may collect evacuated fluids and may protect evacuation means 202 from being contaminated.

According to some embodiments, for example as illustrated in FIG. 2C, junction 28 includes valve 28b, which controls inflow 133 and outflow 177, such that they do not flow simultaneously; rather, valve 28b determines whether inflow 133 is activated or whether outflow 177 is activated. In some embodiments, valve 28b may be operated according to any appropriate means, including predefined settings, electronic means, manual operation, and the like. In some embodiments, control of valve 28b may take pressure, time, targeted region reaction and the like into account.

According to some embodiments, the inflow may be stopped when the pressure in the lumen is above between about 30mBar and 100mBar. According to some embodiments, the outflow evacuation may be continued until the pressure in the lumen is below between about 20mBar to 10mBar. According to some embodiments, the inflow may be stopped when the temperature in the lumen is below about 5-10⁰C. According to some embodiments, the inflow may be initiated when the temperature in the lumen is above about 15-20⁰C. According to some embodiments, both temperature and pressure, as well as any other appropriate parameters, may be used for controlling the system.

According to some embodiments a cryotherapy system may include a control mechanism, for example control unit 1004 shown in FIG 1A, which controls coolant injection and/or expanded fluid evacuation. In some embodiments, the control mechanism may include predetermined parameters (e.g. cyclic) and/or may include parameters defined with feedback to data collected from the system by any appropriate sensors, such as distal pressure, distal temperature, proximal pressure, proximal temperature, comparing flow entered to flow evacuated, operation time and other optional measured parameters. In some embodiments, when such feedback related control is implemented, relevant sensing and control means are to be part of the embodiment (like CPU, firmware, flow sensor, pressure sensor, clock, etc.)

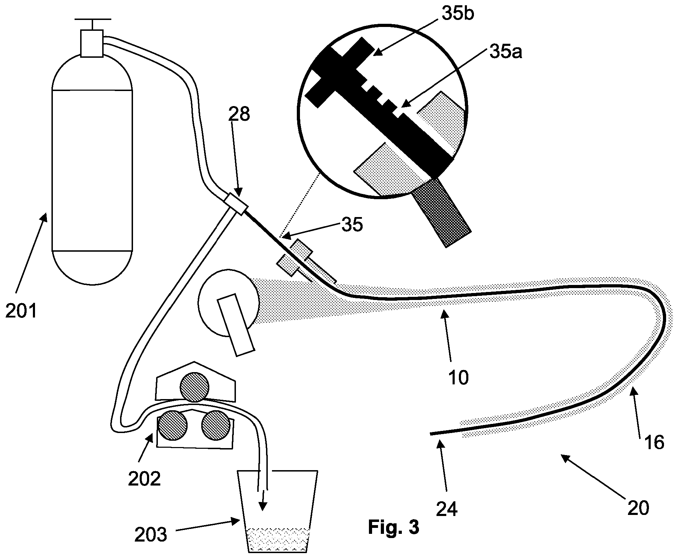

Reference is now made to FIG. 3, which illustrates schematic lengthwise sectional views of an embodiment of the cryotherapy system, specifically the location and possible fixing of the device's distal end, according to some exemplary embodiments of the invention. According to some embodiments a cryotherapy system, for example cryotherapy system 20 includes a source of pressurized coolant 201 and evacuation means 202, which are optionally mutually connected to proximal end 35 of cryotherapy device 10 at junction 28. In some embodiments, optional suction trap 203 may collect evacuated fluids as required.

### Exemplary defining a distance between a catheter's distal end and an endoscope distal end

According to some embodiments, in order to define the distance between a distal end of a cryotherapy device, for example the catheter's distal end 24 from the distal end of endoscope 16, a fixation mechanism is implemented. In some embodiments, the fixation means may be attached to proximal end 35 of cryotherapy device 10. In some embodiments, notches 35a provide a specific distance between catheter's distal end 24 from the distal end of endoscope 16 and limiter 35b may be moved and/or fixated according to each specific notch or scale in relation to operator's definition of desired distance. The desired distance may be predefined, controlled by any appropriate electronic means, controlled manually, or the like. The desired distance may be changed during the operation of the device or may be constant throughout the cryotherapy. Limiter 35b may be fixed in a certain notch 35a by any appropriate securing means, such as clips, screws, elastic bands and the like.

### Exemplary cryotherapy device distal end

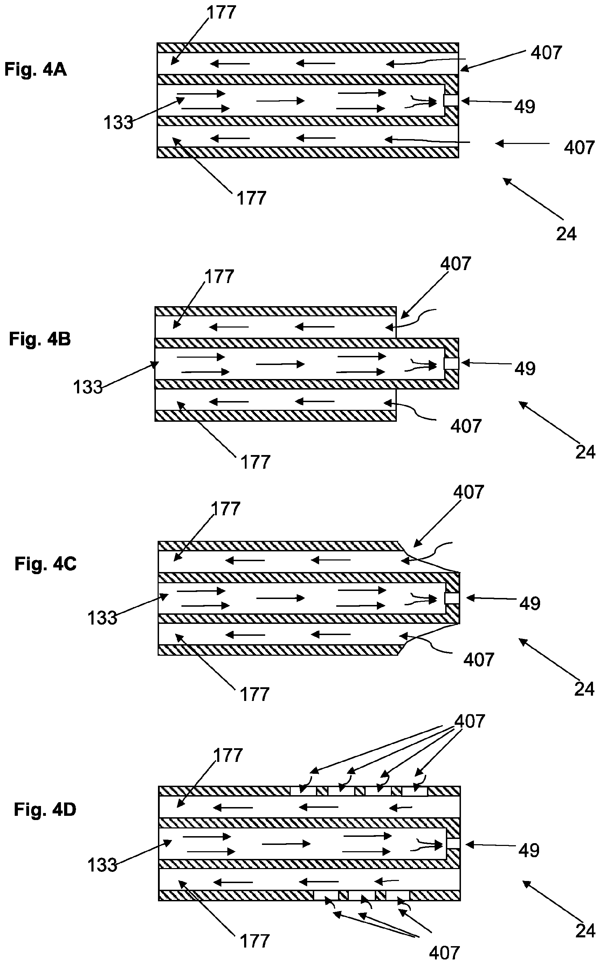

Reference is now made to FIGS. 4A to 4D, which illustrate schematic lengthwise sectional views of a cryotherapy device's distal end in accordance with some embodiments of the present invention. According to some exemplary embodiments, any number of tubes/catheters may be used both for inflow and outflow in and from the system, respectively. It is further noted that according to some exemplary embodiments, any appropriate configuration of those tubes/catheters in relation to one another is possible, including one inside the other, two adjacent tubes and the like. Some embodiments include a tube/catheter comprising several paths through which fluid may flow in any defined direction. According to some embodiments, distal end 24, comprises paths for the simultaneous flow of pressurized coolant inflow 133, to be sprayed through nozzle 49, and evacuated expanded fluid outflow 177.

According to some embodiments, as presented in Figure 4A, distal end 24 has inflow nozzle 49 and outflow openings 407 in a close or similar plane.

According to other embodiments, as illustrated in Figure 4B, inflow nozzle 49 and outflow openings 407 are positioned in different planes of distal end 24. In some embodiments, when in different planes it is possible that the inflow and outflow do not interfere with one another, thereby optimizing the freezing efficacy of inflow 133 as well as the evacuation of outflow 177.

According to other embodiments, as illustrated in Figure 4C, outflow openings 407 are positioned at a changing distance from inflow nozzle 49, e.g., having an inclined cross-section. In some embodiments, this may also provide inflows and outflows that do not interfere with one another. In some embodiments, by having a changing distance between inflow nozzle 49 and outflow openings 407, the evacuated fluid passing through outflow openings 407 should not interfere with inflow 133, though is still, at least partially, in close proximity to nozzle 49, such the efficient evacuation/suction of outflow 177 through outflow openings is possible.

According to other embodiments, as illustrated in Figure 4D, distal end 24 includes several side outflow openings 407. In some embodiments, the side outflow openings 407 may be circumferential or partially circumferential. Utilizing such side openings distances outflow 177 and inflow 133 from one another, such that they do not interfere with one another. Further, since there are several outflow openings 407 they can evacuate fluid efficiently, even though they are positioned on the sides of distal end 24.

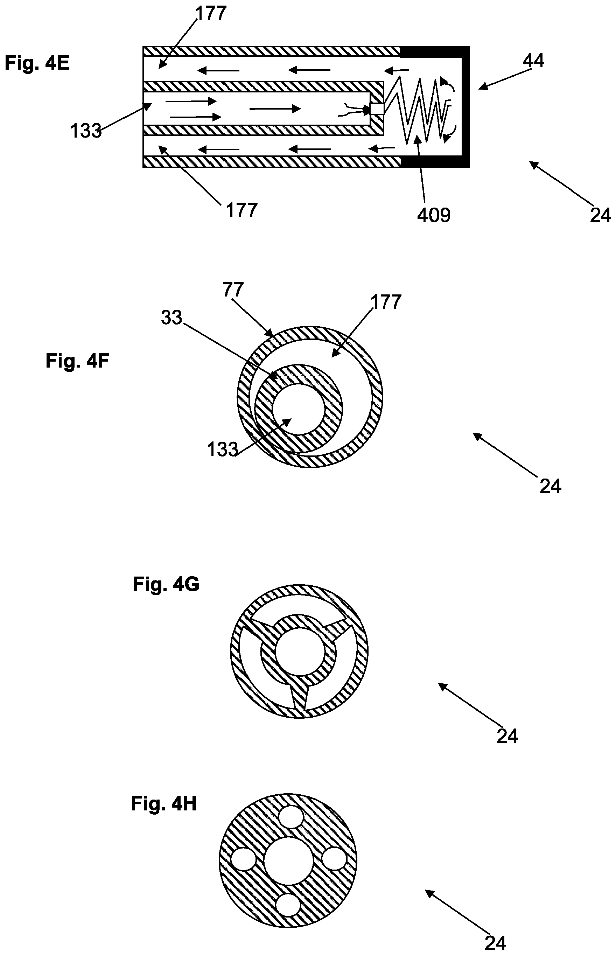

Reference is now made to FIG. 4E, which illustrates schematic lengthwise sectional view of a cryotherapy device's distal end 24 in accordance with another embodiment of the present invention. According to some embodiments, distal end 24 does not comprise any openings into the treated lumen, such that the coolant remains and expands within distal end 24. According to such an embodiment, the transfer of cold temperatures into the tissue is mediated by mediating region 44 of distal end 24. Thus, pressure does not build up in the treated body lumen. According to some embodiments, in order to further optimize temperature drop, i.e., freezing of the treated area, feedback coil 409 may be used for pre-cooling pressurized coolant inflow 133 with the already cooled expanded fluid of outflow 177.

According to some embodiments, mediating region 44 is prepared from hard material such as stainless steel, copper or brass. According to other embodiments, the mediating region 44 may be at least partially prepared from an expandable or inflatable material, such as a balloon, thereby enabling distal end 24 to assume the geometry of the treated lumen, thereby optimizing the treatment. According to further embodiments, the inflatable/expandable part of mediating region 44 may be inflated/expanded such that it assumes a shape that does not necessarily fill the treated lumen, though is able to touch or surround the targeted region. According to some embodiments, the inflatable/expandable part of mediating region 44 may be inflated/expanded according to predefined parameters, according to signals received from any appropriate sensors, automatically or manually. Further, the rate and size of inflation/expansion may differ throughout the cryotherapy treatment. As detailed above, the mediating region may be prepared from a compliant (15-200%, e.g., prepared from polyurethane, nylon elastomers and other thermoplastic elastomers), non-compliant (0-8%, e.g., prepared from PET, nylon and others) or a semi-compliant material (5-15%, e.g., prepared from polyamides and engineered nylons as polyether block amide (Pebax^{®}), and PET and polyurethane), that may be of any appropriate size and shape and that further may be brought into contact with the treated region by expansion and/or by moving the mediating region, possibly, externally by the user. In some embodiments, the expansion and/or the movement may be controlled by any appropriate means and may further be controlled according to data gathered by any internal or external sensors.

Reference is now made to FIGS. 4F to 4H, which illustrate schematic transverse cross-sectional views of a cryotherapy device. According to some embodiments, inflow 133 flows through tube/catheter 33 that is positioned within tube/catheter 77, through which outflow 177 flows. According to some embodiments, tubes/catheters 33 and 77 may be separate from one another, positioned as desired during or before the cryotherapy treatment. According to other embodiments, tubes/catheters 33 and 77 are integrated together, such that essentially only one such tube/catheter exists, including passageways for both outflow 177 and inflow 133. Thus, two tubes may be used to fabricate the desired shape or otherwise, a tube may be extruded to have the appropriate channels. It is noted that although the embodiment presented in Figure 4F shows tube 33 within tube 77, according to other embodiments, tube 77 may be placed within or beside tube 33. This is true for the embodiments shown in Figures 4G and 4H as well, wherein the position of any of the tubes may be changed according to the system/user requirements. Further, according to some embodiments, the outer tube, in the figure tube 77, may be the endoscope's working channels, through which tube 33 (or vice versa) passes.

According to further embodiments, as presented in figures 4G and 4H, the transverse cross section of distal end 24 has several openings, wherein outflow 177 passes through some of the openings, while inflow 133 passes through other openings. Any one of the openings may serve as an inflow, outflow or a sensing passage, according to the system/user requirements. A sensing passage is one through which at least one sensor passes, wherein data gathered from that sensor may be used to control the system and the use thereof. Further, any one of the passages may serve for either outflow or inflow at different times during the cryotherapy treatment, according to predefined conditions, parameters defined according to data received from any appropriate sensors, electronically or manually. It is noted that although Figures 4A-4H present specific embodiments of the distal end of the device, any other embodiments including any number or position of inflow/outflow tubes may be implemented. It is also noted that any such tubes may be prepared from a combination of several tubes, fabrication and/or extrusion.

According to other embodiments, any number of the paths/tubes/catheters passing through endoscope 16 may be used for the sensing means that may be needed for controlling the body lumen pressure or temperature, such as pressure sensing and/or temperature sensing or for any other sensors required. Additional sensing means may include sensors for calculating the lumen's volume (e.g. initial volume and changes in volume due to in and out flows), sensors for sensing the lumen's wall thickness (e.g. by ultrasound or laser light) and the like.

### Exemplary angulation of a cryotherapy device

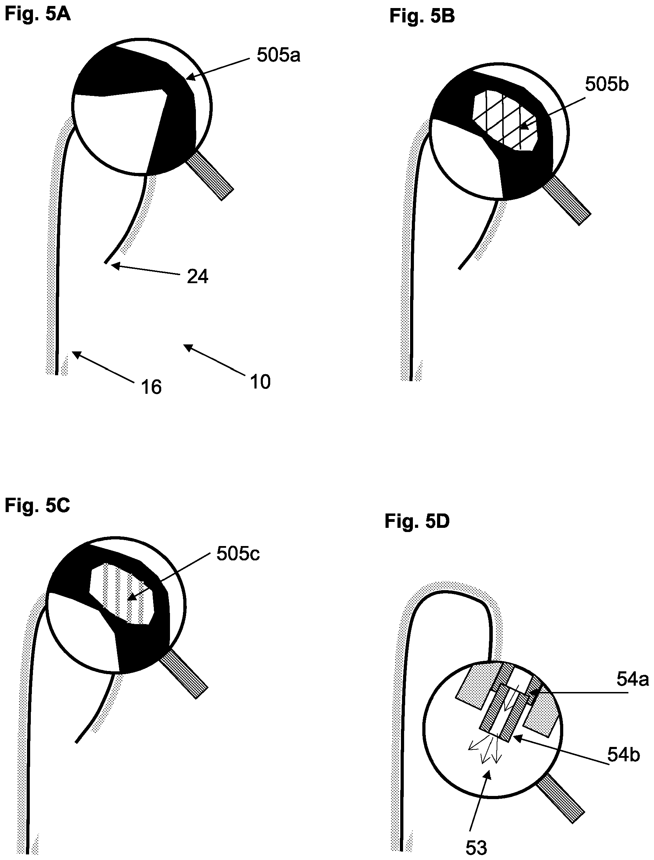

Reference is now made to FIGS. 5A to 5D, which illustrate schematic lengthwise sectional views of a cryotherapy device that is fed into the body lumen via an endoscope during sharp angulation scenario, in accordance with some embodiments of the present invention. According to some embodiments, a cryotherapy device, for example cryotherapy device 10, which is illustrated in Figure 5A, is inserted into the working channel of endoscope 16 such that distal end 24 of cryotherapy device 10 reaches the distal end of endoscope 16. In some embodiments, when the angulation of endoscope 16, including sharp angulation, is necessary for passing through certain passages and/or for targeting the treated region, it is necessary that no kinks are formed in cryotherapy device 10. In some embodiments, such kinks, for example, kinks 505a, as shown in Figure 5A, may interfere with the inflow and outflow of fluids through cryotherapy device 10. Accordingly, cryotherapy device 10 may include a tube/catheter that is flexible, bendable and/or kink resistant, such as braided tube 505b (Figure 5B) or coiled tube 505c (Figure 5C), to assist with angulation, including sharp angulation, allowing the targeting of any treated regions throughout the treated lumen, as well as passing through any necessary passageways in order to reach the body lumen. According to some embodiments, cryotherapy device 10 includes a tube/catheter, wherein only certain sections of the tube/catheter are flexible, bendable and/or kink resistant. According to other embodiments, cryotherapy device 10 includes a tube/catheter that is flexible, bendable and/or kink resistant in its entire length.

According to some embodiments, the cryotherapy device may include non-rigid flexible semiinflatable catheter 54a (Figure 5D) which may be inflated by one of device's flows (pressurized or expanded fluid) up to the working channel geometry. This is especially important when the working channel undergoes angulations. According to some embodiments, a more rigid component 54b may be found around the distal end of the catheter (Figure 5D), such that the rigid components aids in maintaining the orifice shape and direction of the catheter even when expanded, in order to keep the desired sprayed flow 53.

### Exemplary nozzles

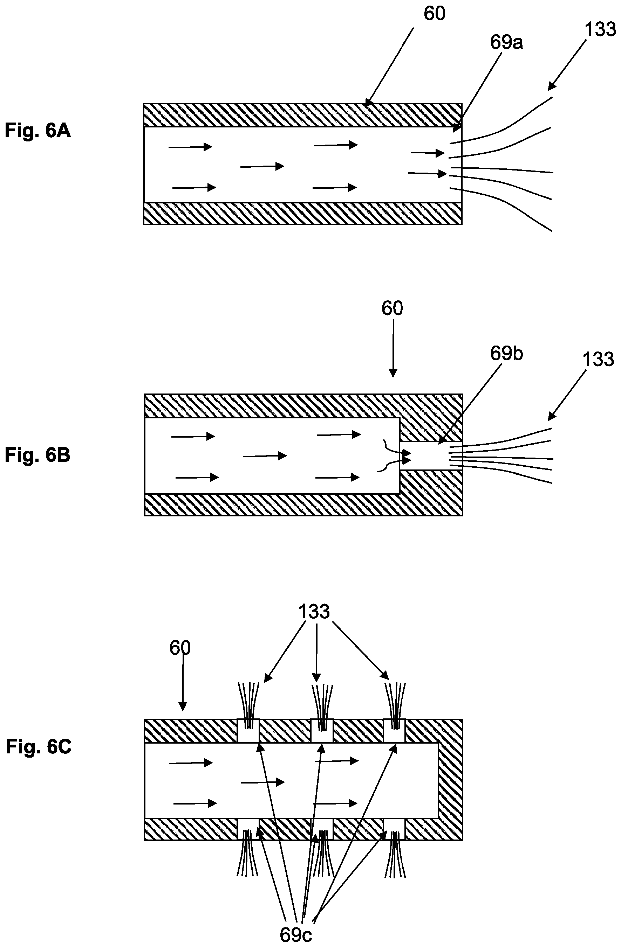

Reference is now made to FIGS. 6A to 6E, which illustrate schematic lengthwise sectional views of a cryotherapy device's distal end, specifically presenting embodiments of nozzles/holes/valves in the inflow tube/catheter and related inflow sprays of cryo-fluid in accordance with some embodiments of the present invention. A cryotherapy device, according to embodiments of the present invention, may comprise a pressurized coolant tube/catheter through which cryogenic fluid may pass until it exits the tube/catheter through a nozzle/hole/valve, thus entering the treated body lumen. Such nozzles/holes valves may be designed in any appropriate manner, such that the stream of cryogenic fluid exits into the body lumen, possibly directed at the treated region. According to one embodiment, for example as presented in Figure 6A, an inflow path, for example inflow 133 exits tube 60 via hole (nozzle/orifice) 69a, which is an opening at the distal end of tube 60. It is noted, that although not illustrated, any one of the holes/nozzles described herein may include any type of valve or the like.

According to some embodiments, as presented in Figure 6B, inflow 133 exits via a hole (nozzle/orifice) 69b, which has a reduced diameter in respect to the diameter of tube 60. Such a reduced diameter may provide cryogenic fluid sprayed at defined pressure onto the treated region and/or may enable a more efficient and accurate Joule Thompson effect.

According to some embodiments of the present invention, hole/nozzle/orifice/ 69a or 69b, as illustrated in Figures 6A and 6B, are aligned in the direction of tube 60 in order to result with frontal spray of inflow 133. According to other embodiments of the present invention, the hole/nozzle/orifice is directed in any appropriate direction, including on the side of tube 60. According to some embodiments, the direction of the spray exiting any one of the nozzles may be changed by an element in the nozzle that may be directed in any appropriate direction, wherein the direction of the element may be changed automatically, manually, by electronic means, in response to data received from any appropriate sensors, and the like.

According to some embodiments, as illustrated in Figure 6C, any number of holes in tube 60 may exist, allowing inflow 133 to exit through a number of holes 69c. Further, holes 69c, may be arranged in any desired configuration and further, may each include a valve that may be closed or opened or partially opened, as required.

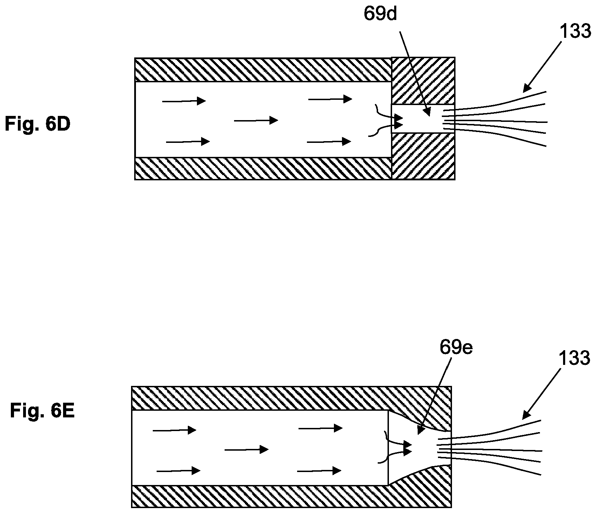

According to some embodiments, the nozzle is part of tube 60, according to other embodiments, as presented, e.g., in Figure 6D, nozzle 69d is an additional component that is attached or connected to tube 60 by any appropriate means. This connection or attachment may result from fabrication advantages or constraints. According to some embodiments, any one of the nozzles may have a dedicated shape or size, for example, as illustrated in Figure 6E, tapered nozzle 69e may be utilized. In some embodiments, the size and shape of the nozzles, as well as the number of nozzles may be optimized according to each treatment, e.g., the size or type of the treated region/lesion, the size of the body lumen in which the treated region/lesion is found, the size and shape of an inflated cryo-balloon and the like. In some embodiments, in order to achieve dedicated shape for nozzle 69e, additional process may be needed, such as thermal treatment or machining. In some embodiments, the inner diameter of any one of the inflow or injection nozzles may be in the range of about 0.05-0.3mm, while the inner diameter of any one of the evacuation or outflow nozzles may be in the range of about 0.5-4mm in average. It is noted that the cross-section of the nozzle may be of any appropriate shape, including circular, non-circular, oval, slit-shaped, or the like.

### Exemplary evacuation out-flow path

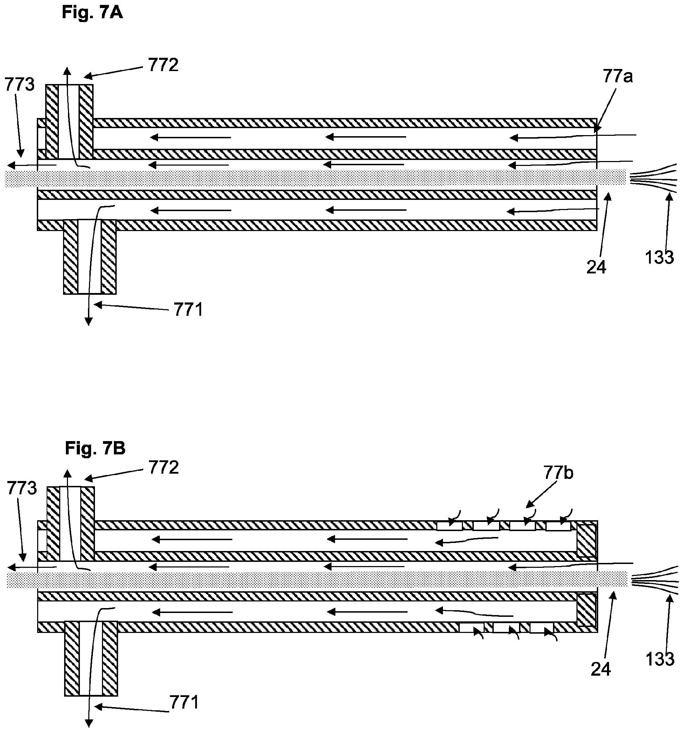

Reference is now made to FIGS. 7A and 7B, which illustrate schematic transverse cross-sectional views of a cryotherapy device's distal end within an endoscope that has in-flow and outflow paths, for example in-flow and out-flow channels, in accordance with some embodiments of the present invention. According to some embodiments, the cryotherapy device's distal end 24 directs the inflow 133 of coolant jet to the treated targeted region. The cryotherapy catheter is inserted from the proximal end of the endoscope through its in-flow channel.

According to some embodiments of the current invention, and as detailed in FIGS. 1A to 6, evacuation of the expanded coolant, as well as any other fluids present in the treated organ, is performed though a catheter/tube inserted into the organ via the endoscope, for example a cryotherapy device inserted into the organ via a working channel of an endoscope. According to some embodiments, the evacuation may be performed through one or several inherent tubes or paths that may be part of the endoscope or that may be added thereto or fed therethrough. For example, some endoscopes have an opening at their back side (e.g. cystoscopic and hysteroscopic resectoscopes) where the cryotherapy device may be inserted. In such endoscopes at least part of the evacuation can take place through this back side (773) while some evacuation can be done through the out-flow exit (771) and/or through the in-flow entrance (772). It is further noted that the inflow, outflow and lumen pressure, affected by the inflow/outflow, may be controlled using such opening.

According to some embodiments of the present invention, the opening to the evacuation tubes/catheters may be in close proximity to or at the same or similar plane to inflow 133 (see, e.g., opening 77a as illustrated in Figure 7A). According to other embodiments several circumferential side evacuation openings 77b, as illustrated in Figure 7B, are provided. As detailed herein, the position, number, size, diameter, etc. of the evacuation tubes may be altered and may be defined as required. Further, any one of the openings may be fitted with a valve allowing the opening to be opened, closed or partially closed, such that any one of the openings may be used as required. The operation of the valves may be electronic, manual, automatic, according to signal received from any appropriate sensors and the like.

### Exemplary folded ablating component

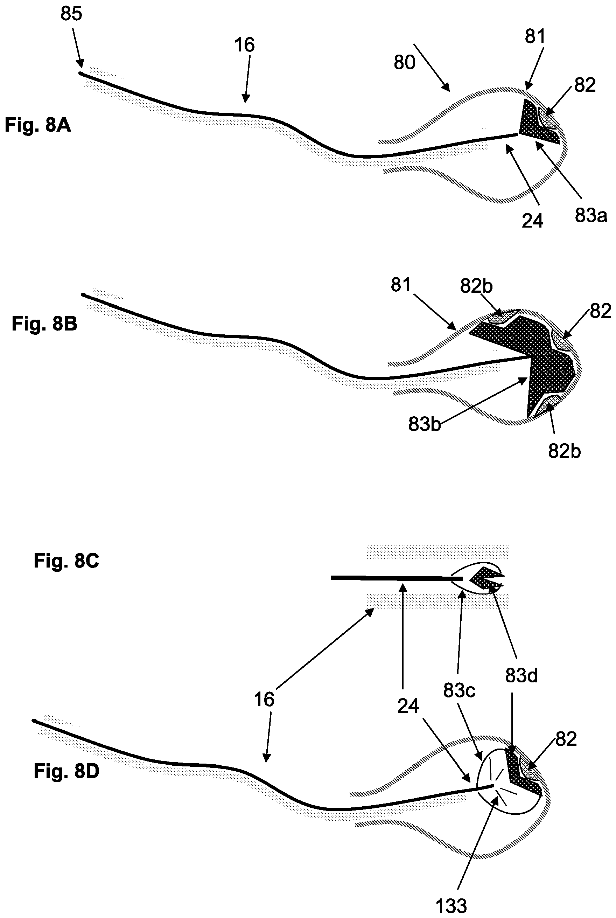

Reference is now made to FIGS. 8A and 8B, which illustrate schematic lengthwise sectional views of cryotherapy devices fed into the treated lumen through an endoscope, during ablation procedure in accordance with some embodiments of the present invention. In some embodiments, the dedicated ablating component, 83a as illustrated in FIG. 8A or 83b as illustrated in FIG. 8B, may be an unfolded cryo balloon (as partially illustrated in FIG. 4E).

According to some exemplary embodiments, in order to treat targeted region 82 a cryotherapy device, for example an ablating device 80 is inserted into endoscope 16 through its working channel in a way that device's distal end 24 exits the endoscope's distal end inside the body lumen 81, while device's proximal end 85 remains outside of the endoscope's proximal end and outside of the treated patient, as illustrated in Figure 8A. According to some embodiments of the present invention, dedicated ablating component 83a is unfolded within body lumen 81 in order to treat targeted region 82, as illustrated in Figure 8A.

According to other embodiments of the current invention, a broader ablating component 83b is unfolded within the body lumen 81 in order to treat targeted regions 82 and 82b and maybe more regions at the same time, as illustrated in Figure 8B. Possibly the entire lumen may be treated if necessary.

Reference is now made to FIGS. 8C and 8D, which illustrate schematic lengthwise sectional views of a cryotherapy inflated device introduced via an endoscope within the treated lumen during an ablation procedure in accordance with some embodiments of the present invention. In some embodiments, the inflated component may include several regions, each of which may have a different compliance. In some embodiments, the inflated component may be constructed from non-compliant or semi-compliant part (0-8%), see element 83c as illustrated in FIGS. 8C and 8D, and semi-compliant or compliant part (5-200%), see element 83d as illustrated in FIGS. 8C and 8D. According to some embodiments, element 83c may be prepared from any appropriate type of metal or plastic. According to some embodiments, element 83c may have a folded and unfolded configuration.

According to some embodiments, the cold energy is transferred to the tissue via compliant/semi-compliant element 83d. As shown in the figures, according to some embodiments, only a certain part of the cryo-balloon may come in contact with the treated area, while other regions in the treated lumen are not in direct contact with the cryo-balloon. In some embodiments, element 83d may be an inflatable component, wherein element 83c may be a structured element designed to hold and/or partially define the shape of element 83d. In some embodiments, the inflated component 83d may be kept unfolded while within endoscope 16 (FIG. 8C) and its shape is such that when inflated (by inflow 133 or other means), element 83d is directed toward the targeted area, transferring cold energy thereto.

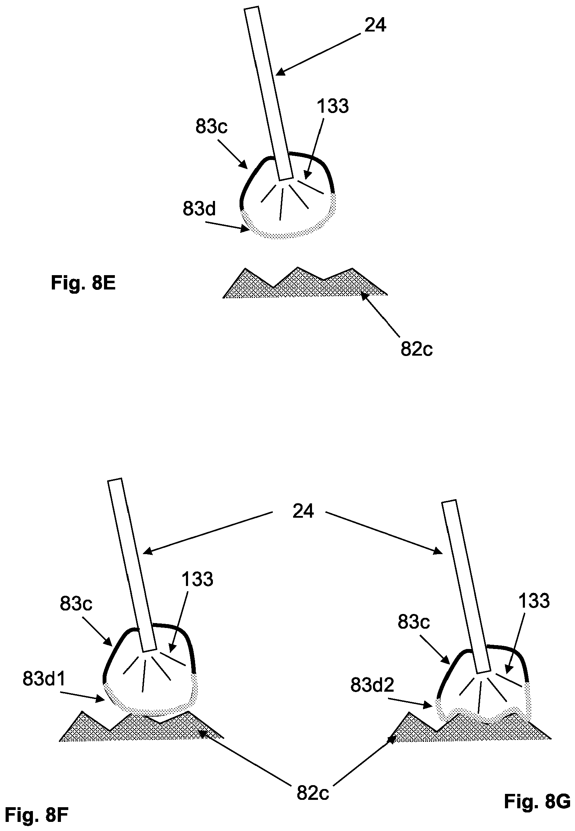

Reference is now made to FIGS. 8E to 8G, which illustrate schematic lengthwise sectional views of a cryotherapy inflated device introduced inside lumen during an ablation procedure in accordance with some embodiments of the present invention. FIG. 8E illustrates the inflated component inside the lumen close to the treated area 82c, having an active part 83d. FIG. 8F illustrates inflated component with low compliance (0-8%) active region 83d1, while FIG. 8G illustrates inflated component with high or semi compliance (5-200%) active region 83d2. As illustrated in the figures, use of a high or semi compliance inflated element may result with optimal fitting of the device to the treated area, since the contact of the device with the treated area is optimal.

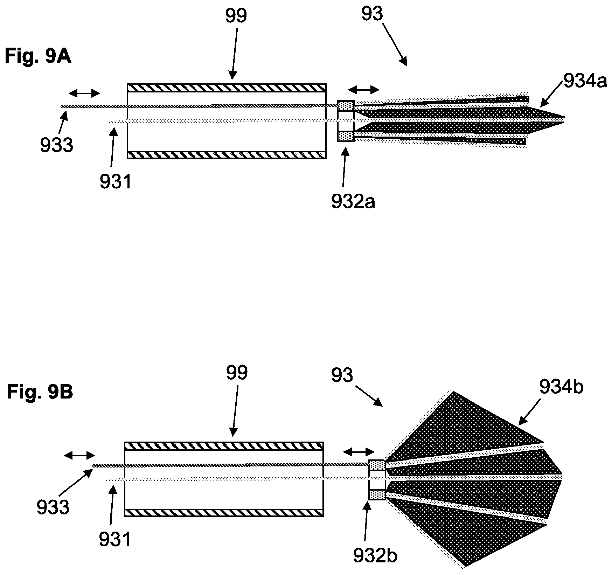

Reference is now made to FIGS. 9A and 9B, which illustrate schematic lengthwise sectional views of an ablation device's distal end 93 in accordance with some embodiments of the present invention. According to some embodiments, folded distal end 934a, which is illustrated in Figure 9A, may comprise supporting ribs as well as a cryo-balloon components. According to some embodiments of the current invention, folded distal end 934a is inserted into the body lumen through sheath 99 (which may be the endoscope's working channel itself) by pushing catheter or wire 931. While the ablating distal end 934a is positioned within the body lumen in the proximity of the targeted region (see FIGS. 8A and 8B), user may manipulate bar (or lever, valve, faucet or controller) 933, in order to unfold the ablating distal end 934b as illustrated in Figure 9B, and then activate the ablating cryo mechanism.

### Exemplary cryotherapy device with visualizing means within endoscope

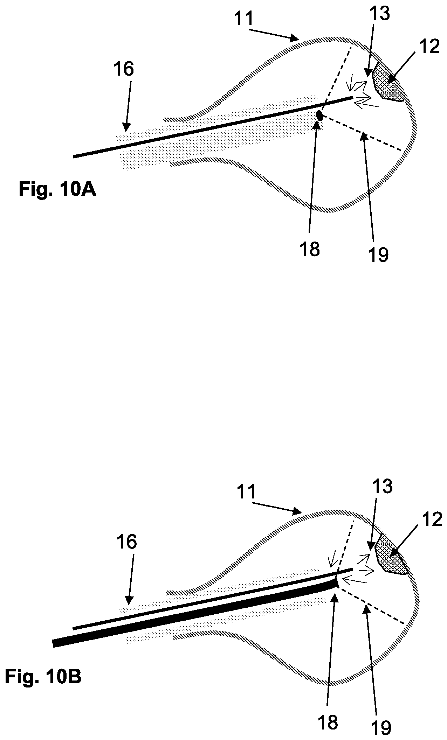

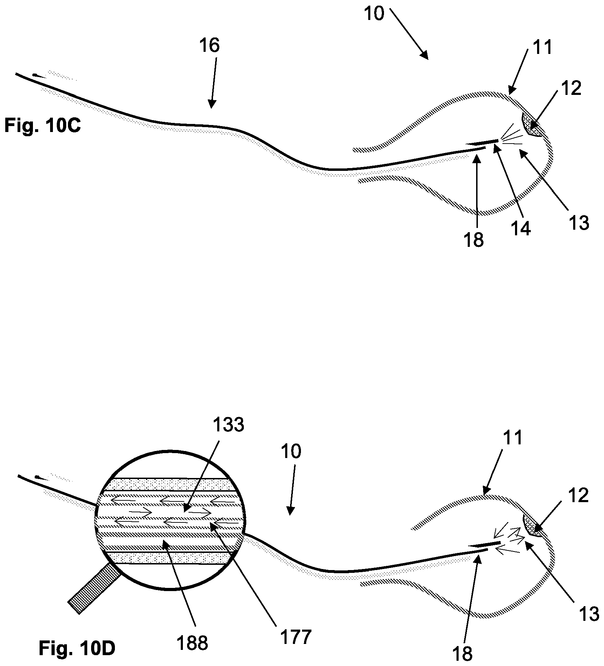

Reference is now made to FIGS. 10A to 10D, which, in relations to the embodiments of Figure 1A, illustrate schematic lengthwise sectional views of additional embodiments of a cryotherapy device, for example cryotherapy device 10 during cryotherapy procedure, wherein cryotherapy device 10 is introduced into the treated lumen 11 through endoscope 16.

According to some embodiments, and as illustrated in FIG. 10A, endoscope 16 may include visualization means 18 for visualizing field of view 19 (FOV), wherein jet 13 and/or treated area 12 are within FOV 19.

According to other embodiments, and for example as illustrated in FIGS. 10B to 10D, endoscope 16 is a sheath like device in which devices may be inserted into the lumen, through the sheath, by various means, e.g., using space 188, for example as illustrated at FIG. 10D. Accordingly, sheath 16 may allow the insertion of different types of visualization means 18 for viewing FOV 19, cryotherapy device 14 and the like. In some embodiments, outflow 177 for example may have a dedicated outflow channel, for example as illustrated in FIG. 10D or it may flow through endoscope's free spaces 188 (FIG. 10D).

### Exemplary cryotherapy system with means for injecting fluids

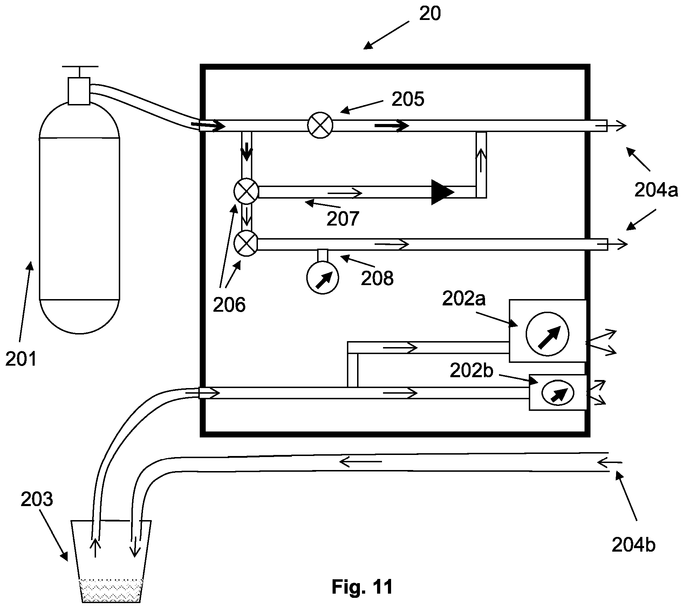

Reference is now made to FIG. 11, which illustrate schematic lengthwise sectional view of a cryotherapy system, similar to the system illustrated in Figure 1A or in Figure 2, though specifically presenting flow paths and means by which fluids flow in and out of the treated lumen in accordance with some embodiments of the present invention, including for example means for injecting fluids other than the cryo fluid. According to some embodiments, cryotherapy system 20 includes a source of pressurized coolant 201 and evacuation means 202a and 202b. It is noted that any one or both of evacuation means 202a and 202b may be utilized and further, any other appropriate evacuation means may also be used. In some embodiments, system 20 further includes inflow paths 204a for directing fluids into the lumen and outflow path 204b for evacuating fluids from the lumen. It is noted that any appropriate number of inflow and outflow paths may be utilized. In some embodiments, evacuation means 202a and/or 202b may be dedicated to cryotherapy system 20, and may further be any known evacuation means, including an outlet to the surrounding atmosphere, or any other appropriate means by which material, e.g., expanded fluids, may be evacuated from the system. According to some embodiments, evacuation means 202a and/or 202b includes suction provided by any appropriate pump, vacuum, or the like. According to some embodiments, the evacuation means for example evacuation means 202a and/or 202b may be attached to the proximal end of cryotherapy device, for example cryotherapy device 1002 shown in Fig. 1A, optionally to the proximal end of the outflow path 1010 or to outflow path 204b shown in Fig. 11. According to some embodiments, optional suction trap, for example suction trap 203 may collect evacuated fluids and may protect evacuation means, for example evacuation means 202a and/or 202b from being contaminated.

According to some embodiments, for example as illustrated in FIG. 11, the inflow and outflow fluids may originate from a combination of sources, such as source 201. It is noted that any number of sources may be used together or in any required sequence. According to some embodiments, source 201 may be reduced (e.g. reduce pressure and/or flow rate) by any one or both of reducers 206, thus providing an inflow of pressurized coolant 205 and/or reduced fluent 207 at different times or simultaneously (e.g. by using path 208 as illustrated at FIG. 11). It is noted that different sources may be used (not shown) and further, as detailed, the same source may be used for providing different pressures and/or flow rates. According to some embodiments path 205 may be used for delivering a coolant to the targeted tissue while path 207 may be used for keeping the lumen inflated and/or for dehydrating between the cryo cycles. Thus, in some embodiments, the pressure of the fluid in each one of paths 205, 207 and the like may be either the same or different and further, may change over time, as required. According to other embodiments, path 208 may be used in parallel to path 205 and may be used for pressure measurement, inflation control and/or as dehydrating means.

According to other embodiments, instead of using reducers 206, flows 207 and 208 may be originate from additional pressure sources (not shown), optionally transferred by dedicated pumps.

According to some embodiments, for example as illustrated in FIG. 11, evacuation means 202a and/or 202b may be utilized simultaneously or at different times. In some embodiments, while source 202a may have abilities to handle high pressures and/or high flow rate and the like, source 202b may have fine tuning abilities to allow accuracy and/or for allowing the system to provide lower pressures, lower flow rates and the like.

According to some exemplary embodiments, the various pressure and evacuation means may be operated according to any appropriate means, including predefined settings, electronic means, manual operation, and the like. In some embodiments, control of the different means (205, 206, 207, 208, 202a, 202b) may take pressure, time, targeted region reaction and the like into account.

According to some embodiments, the inflow may be stopped when the pressure in the lumen is above between about 30mBar-100mBar. According to some embodiments, the outflow evacuation may be continued until the pressure in the lumen is below between about 20mBar-10mBar. According to some embodiments, the inflow may be stopped when the temperature in the lumen is below about 5-10⁰C. According to some embodiments, the inflow may be initiated when the temperature in the lumen is above about 15-20°C. According to some embodiments, both temperature and pressure, as well as any other appropriate parameters, may be used for controlling the system.

According to some embodiments the cryotherapy system may include a control unit, for example a control mechanism, which controls coolant injection and/or expanded fluid evacuation. The control mechanism may include predetermined parameters (e.g. cyclic) and/or may include parameters defined by feedback according to data collected from the system by any appropriate sensors, measuring parameters such as distal pressure, distal temperature, proximal pressure, proximal temperature, comparing flow entered to flow evacuated, operation time and other optional measured parameters. In some embodiments, when such feedback related control is implemented, relevant sensing and control means, such as a control circuitry for example CPU, firmware, flow sensor, pressure sensor, clock and the like, may be included in the device, or operated adjacent thereto.

### Exemplary rolling cryotherapy device

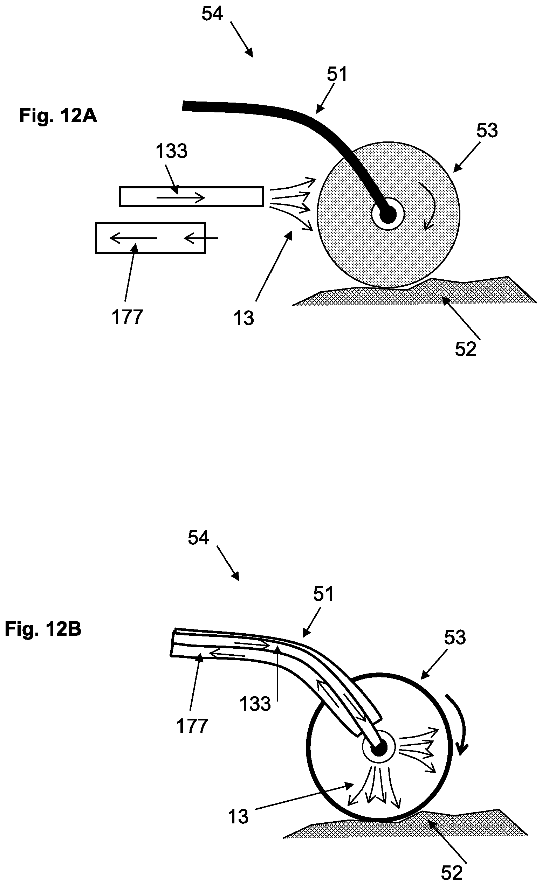

Reference is now made to FIGS. 12A and 12B, which illustrate schematic lengthwise sectional views of a rolling cryotherapy device 54 that includes rolling component 53 which may be cooled externally (FIG. 12A) or internally (FIG. 12B), in accordance with some embodiments of the present invention. According to some embodiments, cryotherapy device 54, which is illustrated for example in Figure 12A, has a rolling component 53 which has the ability to absorb cryo energy (e.g. made of metal) from external source as jet 13 and to cool the treated tissue 52. According to other embodiments, and as illustrated for example in Figure 12B, the rolling component 53 may have an internal cooling jet 13 which expands inside rolling component 53. According to some embodiments, rolling component 53 is cooled both externally and internally. According to some embodiments, the cryogenic treatment may be performed by rolling component 53 over treated area 52, rather than directly spraying coolant onto treated area 52. According to some embodiments, rolling component 53 may be replaced with any component that may transfer the cryo energy to treated area 52, such as a sliding component or an ironing component.

### Exemplary geometrical arrangement of cryo tip

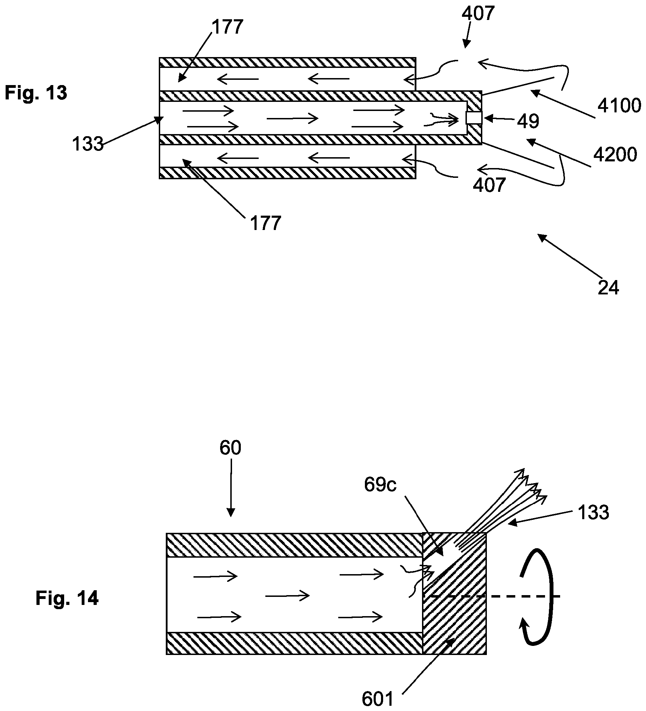

Reference is now made to FIG. 13, which illustrates schematic lengthwise sectional view of a cryotherapy device's distal end in accordance with additional embodiments to those presented in Figure 4.

According to some embodiments, for example as presented in FIGS. 4A to 4C, distal end 24 has inflow nozzle 49 and outflow openings 407 in a close or similar plane.

According to other embodiments, as illustrated in FIG. 13, inflow nozzle 49 and outflow openings 407 are separated by extension 4100 (e.g. cone, dress, flaps). In some embodiments, due to the separation between inflow nozzle 49 and outflow openings 407, when using extension 4100 it is possible that the inflow and outflow do not interfere with one another, thereby optimizing the freezing efficacy of inflow 133 as well as the evacuation of outflow 177. In addition, and according to some embodiments, extension 4100 controls the size of the treated area, at least to an extent, since it essentially separates, at least partially, between the treated and non-treated areas and isolates the treated area, such that only the treated area receives the cryo energy and the like. According to some embodiments, extension 4100 distances the distal end of the cryotherapy device from the treated area. In some embodiments, when extension 4100 distances the distal end of the cryotherapy device from the treated area it may or may not separate between inflow 133 and outflow 177, between the treated area and the non-treated area, or between the treatment volume 4200 and the surrounding body lumen volume, as detailed herein. In this respect, in some embodiments, the distal end of the cryotherapy device is intended to include the distal end of any element in the cryotherapy device, including the endoscope/sheath, the inflow or outflow channel, the optics or visualization means and the like.

According to some embodiments, extension 4100 defines a treatment space, for example treatment volume 4200, which essentially is the volume formed between the distal end of the cryotherapy device, the treated area, and extension 4100. Thus, treated volume 4200 is, at least partially, separated from the surrounding volume of the treated lumen. It is noted that although extension 4100 does not necessarily touch the treated area such that a closed volume is not formed, treatment volume 4200 is defined as the volume formed if extension 4100 would be lengthened to touch the treated area.

According to some embodiments, extension 4100, at least partially, separates the treatment volume from the surrounding lumen volume, such that the hydration levels in the treatment volume may be maintained, as well as the conditions necessary for visualizing the treated area and/or the treatment volume 4200. Particularly, as detailed above, in some embodiments, it is possible to introduce fluids other than the cryo fluid that may dry, or at least lower the humidity in, the treated area and/or the treatment volume 4200. In some embodiments, it is also possible to evacuate hydrated gases or even liquids from the treatment volume, in order to enhance the visibility. In some embodiments, when the treated area and/or the treated volume 4200 are, at least partially, separated from the surrounding lumen environment by any element, such as extension 4100, it is optionally easier to control the conditions in the treatment volume as well as on the treated area, including the hydration thereof and the visibility conditions found therein.

Reference is now made to FIG. 14 (related to FIGS. 6A-E), which illustrates schematic lengthwise sectional view of a cryotherapy device's distal end, specifically presenting rotating/swiveling, or at least partially rotating nozzles/holes/valves in the inflow tube/catheter and related inflow sprays of cryo-fluid in accordance with some embodiments of the present invention. It is noted that, similarly, the outflow tube/catheter may also be fitted with any such rotating elements.

According to some exemplary embodiments, for example as illustrated in FIGS. 6A to 6E, jets of cryo fluid (or any other fluid) may be directed in specific directions (front, side, etc.). According to other embodiments, a combination of directions (e.g. both front and side) may assist while covering an area/volume with spray, such that the area/volume is evenly cooled. In some embodiments, the combination of directions of spray may also be used when inflating a geometrically-shaped inflatable device, such that not only is the inflatable device inflated, it is also optionally cooled evenly (e.g. a flatter shape balloon).

According to some embodiments, the nozzle is part of tube 60 (FIGS. 6A to 6C). According to other embodiments, for example as presented, e.g., in Figure 14, nozzle 69c is part of an additional component 601 that is attached or connected to tube 60 by any appropriate means which allows it to swivel, at least partially (e.g. around an axis). It is noted that although only one nozzle 69c is illustrated, any number of such nozzles may be included and may be positioned at any point on component 601. In some embodiments, the inner diameter of any one of the inflow or injection nozzles may be in the range of about 0.05-0.3mm, while it is noted that the cross-section of the nozzle may be of any appropriate shape, including circular, non-circular, oval, slit-shaped, or the like. According to some embodiments, a swivel like nozzle (e.g. 69c as part of 601) may provide the coverage of an area/volume with spray, such that the area/volume is evenly cooled. In some embodiments, the combination of directions of spray may also be used when inflating a geometrically-shaped inflatable device, such that not only is the inflatable device inflated, it is also cooled evenly (e.g. a flatter shape balloon). It is noted that component 601 may swivel around any appropriate axis and further, may swivel any number of degrees. In some embodiments, the swiveling may be controlled manually, mechanically, automatically, by feedback, by predefined parameters or by any other appropriate means According to some embodiments, since tube 60 and nozzle 69c are not in a straight line, the force of inflow 133 may cause component 601 to swivel around any appropriate axis.

### Exemplary distal end of a cryotherapy device with visualization means

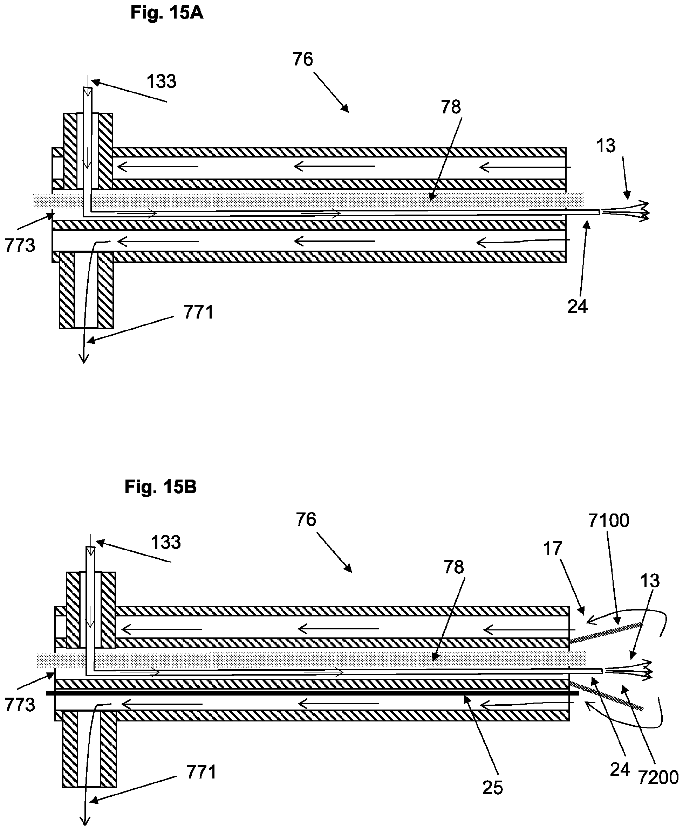

Reference is now made to FIGS. 15A and 15B, which illustrate schematic transverse cross-sectional views of a cryotherapy device's distal end within an endoscope that has in-flow and outflow channels, in accordance with additional embodiments to those presented in Figures 7A and 7B. According to some embodiments, the cryotherapy device's distal end 24 directs inflow 133 of coolant jet 13 to the treated targeted region. In some embodiments, the cryotherapy catheter is inserted from the proximal end of the endoscope through its in-flow channel. In some embodiments, visualization means 78 is inserted through the endoscope in order to allow visualization of the treated area, prior, during and/or after the activation of the cryotherapy operation.

According to some embodiments of the present invention, the opening to the evacuation tubes/catheters may be in close proximity to, or at the same or similar plane to, jet 13 (as illustrated in Figure 15A). According to other embodiments, and as illustrated in Figure 15B, cryotherapy device 76 may include extension 7100. In some embodiments, extension 7100 may control the conditions in treated volume 7200 as well as the treated area and the conditions thereon, by, at least partially, separating treatment volume 7200 and/or the treated area from the surrounding lumen environment. As detailed above, in some embodiments, the separation of treatment volume 7200 and/or the treated area from the general surroundings of the lumen may control the dehydration of treatment volume 7200 and/or of the treated area and provide better visualization (e.g., by visualization means 78) of treatment volume 7200, the treated area and the like. In some embodiments, in comparison to extension 4100, as illustrated in FIG. 13, both the cryotherapy and visualization means may be included within treatment volume 7200, whereas in the embodiment presented in Fig. 13, only the cryotherapy means are present within the inner volume of the extension, i.e., treatment volume 4200. In some embodiments, sensing means 25 may be located outside treatment volume 7200, as illustrated in FIG. 15B, in order to control the surrounding lumen environment. In some other embodiments sensing means may be inside treatment volume 7200, such as to control the conditions within treatment volume 7200. According to some embodiments, sensors may be placed both within treatment volume 7200 and outside treatment volume 7200, thereby optionally controlling the conditions in both the treatment volume and in the surrounding lumen environment.

According to some embodiments, extension 7100 distances the distal end of the cryotherapy device from the treated area. In some embodiments, when extension 7100 distances the distal end of the cryotherapy device from the treated area it may or may not separate between inflow 13 and outflow 17, between the treated area and the non-treated area, or between the treatment volume 7200 and the surrounding body lumen volume, as detailed herein. In this respect, the distal end of the cryotherapy device is intended to include the distal end of any element in the cryotherapy device, including the endoscope/sheath, the inflow or outflow channel, the optics or visualization means and the like. In some embodiments, such a defined distance, especially between the cryo jet 13 and the targeted area, may optimize cryoablation's outcome predictability and potential efficacy.

In some embodiments, the defined distance between the cryotherapy device and the target region or tissue in the target region is 5 mm, 10 mm, 15 mm, 20 mm or any intermediate, smaller or larger distance. In some embodiments, the minimal distance for spraying cryogenic fluid is at least 4 mm, for example 4 mm, 5 mm, 6 mm or any intermediate, smaller or larger value. In some embodiments, spraying cryogenic fluid at a distance lower than 4 mm may cause the tip/nozzle to stick to the tissue, for example due to cryo adhesive properties. Additionally, when using a tapered cryo nozzle or a cryo tip, getting closer than 4 mm increase the tissue puncturing risk.

### Exemplary cryotherapy inflatable device

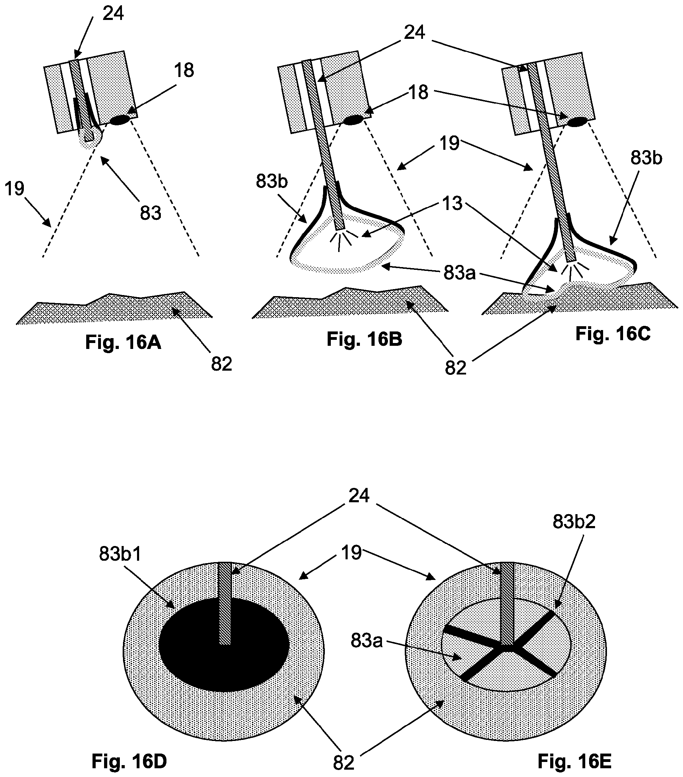

Reference is now made to FIGS. 16A to 16C, which illustrate schematic lengthwise sectional views of a cryotherapy inflatable device during an ablation procedure in accordance with some embodiments of the present invention. FIG. 16A illustrates inflatable component 83 in its folded configuration. In some embodiments, while inflatable component 83 is in its folded configuration, cryotherapy device distal end 24 may be in close proximity to the treated area 82, such that visualization means 18 can clearly observe treated area 82, providing a clear field of view, FOV, 19.

FIG. 16B illustrates unfolded inflatable component 83, having an active region/section/part/element 83a and supporting region/section/part/element 83b, according to some exemplary embodiments. In some embodiments, the supporting element 83b may be an integral part of inflatable component 83, e.g., supporting element 83b may be a painted region of inflatable component 83, a surface treated region of folded component 83 or the like. According to other embodiments, supporting element 83b may be an added or external element, added onto, fused to or adjacent to folded component 83. In some embodiments, other than providing support to inflatable element 83, supporting element 83b may have several functions. In some embodiments, for example, supporting element 83b may absorb the illumination of visualization means 18, such that any type of glare is overcome, such that the user may visualize the surrounding of treated area 82. Alternatively or additionally, supporting element 83b may be painted with anti-reflection dye and/or its surface may undergo any appropriate type of surface treatment in order to make it more light-absorbing (diffuse vs. specular reflection). For example, supporting element 83b may not transfer cold (e.g. may be double walled at this area or can be made of isolating material). In addition, supporting element 83b may provide support such that active element 83a assumes a relatively flat shape, rather than a completely rounded shape, e.g., active element 83a may be shaped like a cone or a bell. FIG.
16C illustrates the device's distal end 24 during active cryotherapy, according to some exemplary embodiments. In some embodiments, for example as illustrated in FIG. 16C, while active element 83a is in contact with the treated area 82, the visualization means 18 visualize supporting element 83b which covers the active element 83a, which may be useful, as detailed below. It is further noted that active element 83a may be equivalent to any one of elements 83d, 83d1 and/or 83d2, detailed in Figures 8C to 8G and therefore, any of the embodiments detailed herein regarding 83d, 83d1 and/or 83d2 are considered to be possible embodiments for element 83a in Figures 16B to 16E.

Reference is now made to FIGS. 16D and 16E, which illustrate schematic upper views 19 (as seen from visualization means 18, FIG. 16C) of a cryotherapy device's distal end 24, in accordance with some embodiments of the present invention. According to some embodiments, for example as illustrated in FIG. 16D, supporting element 83b1 may totally block visualization of the active inflated element 83a, while still allowing good visualization of treated tissue's surroundings 82. In some embodiments, such blockage may be required when active element 83a causes reflection (glazing, glare) which interferes with the ability to perform the ablation procedure.

According to other embodiments, for example as illustrated in FIG. 16E, supporting element 83b2 may only partially block the visualization of active inflated element 83a, while allowing some visualization of active element 83a in addition to the treated tissue's surroundings 82. Supporting element 83b2 may be designed so as to provide only such partial blockage when active part 83a does not cause interfering reflection, and supporting element 83b2 functions mainly as a support for shaping inflatable active component 83a, e.g., active component 83a may assume a relatively flat shape due to supporting element 83b2.

According to some embodiments, inflatable components may use simple frontal flow nozzles in order to cool the inflatable volume (from the inside). According to some embodiments, the inflatable components are inflated by the cryo fluid. According to some embodiments, the inflated components may be inflated by any combination and/or sequence of fluids. According to other embodiments, special inner nozzles, as swivel jet as illustrated in FIG. 14 (or a combination of front and side nozzles, as described in FIGS. 6b and 6C), may assist with distributing the cryo fluid in a shaped inflatable components (e.g. flatter balloon, as illustrated in FIGS. 16B to 16E).

### Exemplary cryotherapy device with geometrical extensions

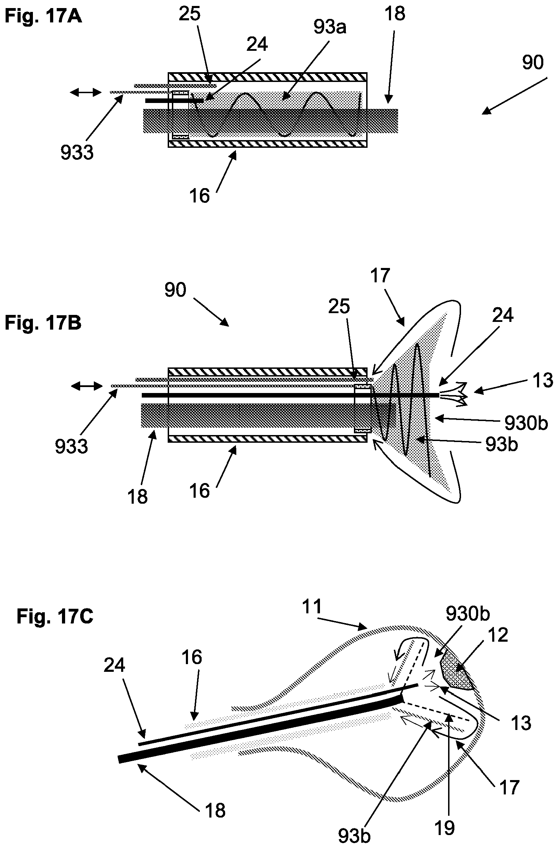

Reference is now made to FIGS. 17A to 17F, which illustrate schematic lengthwise sectional views of distal end 90 of the cryotherapy device in accordance with some embodiments of the present invention. According to some embodiments, for example as illustrated in Figure 17A, during lumen screening or during insertion into the lumen, the components of the cryotherapy device may be held inside endoscope or sheath 16, while using the input from the visualization means 18 to direct the sheath/endoscope to the proximity of the treated area. According to some embodiments, cryotherapy device's components include an extension, sensing means 25 and cryotherapy catheter 24, wherein the extension may be in different configurations including a folded configuration 93a (Figure 17A) and an unfolded configuration 93b (Figure 17B) and optionally any other partially folded configurations. In some embodiments, distal end 90 may further include dedicated evacuation means. According to other embodiments, the remaining volume within the working channel or sheath may be used as evacuation means. According to some embodiments, the user may manipulate bar (or lever, valve, faucet or controller) 933, in order to unfold the distal end components and then activate the ablating mechanism, for example as illustrated in Figure 17B. In some embodiments, unfolded extension 93b may comprise supporting ribs or wires as well as supporting components (e.g. flexible polymer), and similarly to extensions 4100 and 7100 (illustrated in Figures 13 and 15B, respectively), may, at least partially separate the treated area and/or treatment volume 930b from the general lumen environment and may further control the conditions in the inner volume defined by the extension, i.e., treatment volume 930b. In some embodiments, both cryotherapy and visualization means may be included within treatment volume 930b. In some embodiments sensing means 25 may be located outside of treatment volume 930b, as illustrated in FIG. 17B, in order to control the surrounding lumen environment. In some other embodiments sensing means may be inside treatment volume 930b or both inside and outside treatment volume 930b, in order to improve mutual control. Fig 17C illustrates cryotherapy activation inside lumen 11, according to some exemplary embodiments of the invention. In some embodiments, cryo jet 13 treats treated area 12 while evacuation flow 17 is directed or pushed outside of treatment volume 930b, for example as defined by unfolded extension 93b. According to some embodiments, for example as illustrated in FIGS. 17B and 17C, unfolded extension 93b may also define the distance between cryo jet 13 and the treated area 12, assuming that unfolded extension 93b touches, or is in close proximity to the tissue surrounding treated area 12. In some embodiments, such a defined distance may help with optimizing ablation parameters as well as the results obtained by the treatment.

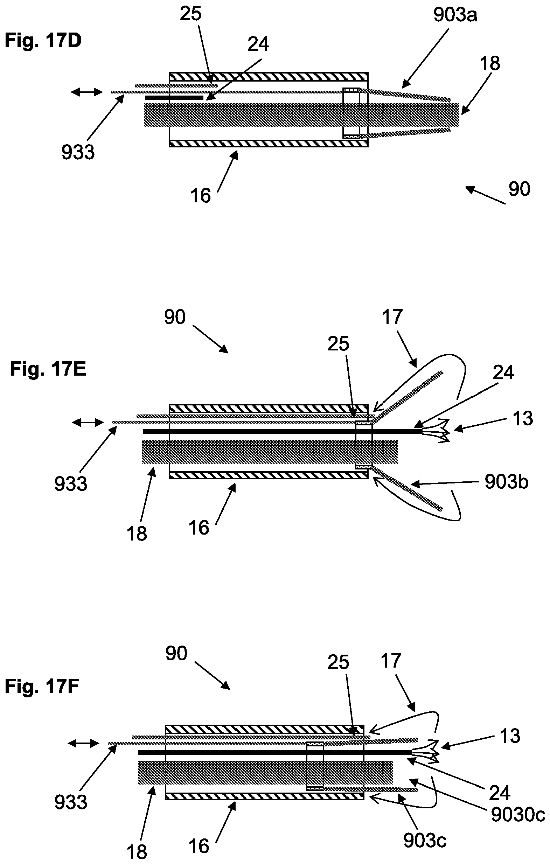

According to other embodiments, for example as illustrated in FIGS. 17D to 17F, user may manipulate controller (or lever, valve, faucet or bar) 933 in order to change the geometry and/or functionality of the extension (e.g. 903a, 903b, 903c). According to some embodiments, for example as illustrated in FIG. 17D, the extension 903a may be positioned around visualization means 18, such as optionally to function as a smoothing element enabling the insertion of endoscope 16 into the lumen, while visualizing the lumen as well as the path thereto with visualization means 18, with no interference of the other components (e.g. cryotherapy catheter 24, sensing means 25, etc.), till the activation of the ablation procedure when those components are needed.

According to some embodiments, for example as illustrated in FIG. 17E, extension 903b may be used similarly to extension 93b, as illustrated in FIGS. 17B and 17C, according to the manipulation of controller 933. In some embodiments, similar to extension 93b (FIGS. 17B and 17C), the predefined geometry of extension 903b may define the distance between cryo jet 13 and the treated area, thus optimizing the performance of the device. According to other embodiments, for example as illustrated in FIG. 17F, additional geometries of extension 903c may be used, using controller 933, in order to apply different set-point (e.g. distance from tissue) or in order to allow ablation during different constraints (e.g. limited space, unsmooth target area, sharp angulation). Thus, controller 933 may be used to control both the inner volume defined by the extension, i.e., treatment volume 9030c, and the distance of the distal end of the device from the treated area.

### Exemplary geometrical extensions

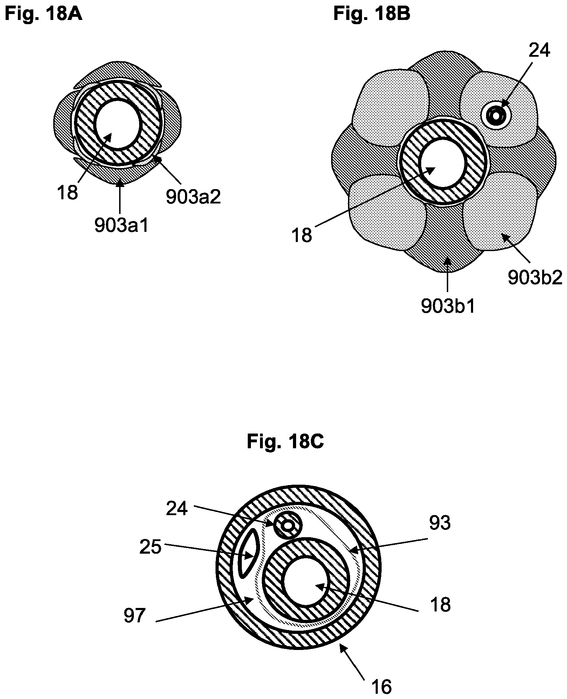

Reference is now made to FIGS. 18A to 18C, which illustrate schematic transverse cross-sectional views of endoscope's visualization means and cryotherapy device's distal end, in accordance with some embodiments of the present invention. According to some embodiments, for example as illustrated in FIG. 18A, extension 903a (which is illustrated in FIG. 17D in lengthwise sectional view) can be prepared from overlapping leaves or several materials (e.g. rigid leaves 903a1 and flexible leaves 903a2). In some embodiments, when unfolding the extension 903b, as illustrated in FIG. 18B (and in FIG. 17E in lengthwise sectional view), the overlapping leaves, 903b1 and 903b2 or different materials may optionally form a cone shape or dress-like isolating extension 903. As illustrated in FIG. 18B, both visualization means 18 and cryotherapy catheter 24 are within the treatment volume (e.g. cone, dress) defined by the unfolded extension, such that the user can visualize the ablation process.

According to other embodiments, for example as illustrated in FIG. 18C, extension 93 may be folded within the endoscope or sheath 16 (93a, illustrated in FIG. 17A in lengthwise sectional view) prior to unfolding and use. In some embodiments, the extension may then be partially unfolded, e.g., by partially extending it out of the endoscope/sheath, for example as shown in Figures 17D, 17F and 18A. Further, the extension may be optionally fully unfolded, for example as presented in Figures 17B, 17C, 17E and 18B. It is noted that any part of the cryo treatment may be performed when the extension is fully folded, partially folded and/or fully unfolded.

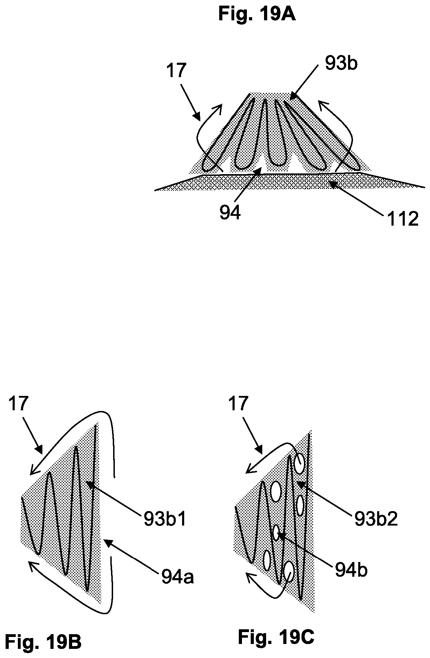

Reference is now made to FIGS. 19A to 19C, which illustrate schematic transverse cross-sectional views of a cryotherapy device's distal end, specifically presenting means to isolate the treated area / volume, in accordance with some embodiments of the present invention. According to some embodiments, for example as illustrated in FIG. 19A, unfolded extension 93b may include vents 94 to allow expended fluid 17 to exit or be removed from the inner volume defined by extension 93b and flow toward the evacuation means openings (not shown). In some embodiments, extension 93b (or cone, dress) may be prepared form a supporting structure (like Nitinol, super elastic material wires/ribs) and a fabric / polymer / folded surface or by rigid and flexible overlapping leaves (for example as illustrated in FIGS. 18A and 18B).

According to some embodiments, unfolded extension 93b may be positioned at a certain, possibly pre-defined, distance from treated area 112 so expended fluid 17 can naturally exit the inner volume defined by extension 93b. According to other embodiments, for example as illustrated in FIG. 19A, unfolded extension 93b is in close proximity to or touches treated area 112, such that vents 94 allow expended fluid 17 to flow toward the evacuation means openings (now shown).

FIG. 19B illustrates extension (or cone, dress) 93b1 comprising flat ending 94a, according to some exemplary embodiments of the invention. In some embodiments, extension 93b1, for example as illustrated in FIG. 19B, can be used when extension 93b1 is held at a certain, possibly predefined, distance from the treated area, or when tissue is not smooth or whenever expanded fluid 17 can exit the inner volume defined by extension 93b1, by any appropriate means. According to other embodiments, for example as illustrated in FIG. 19C, unfolded extension 93b2 includes vents within the walls of the extension, 94b, in order to ease the flow of expended fluid 17 out of the inner volume defined by the extension. In some embodiments, for example as demonstrated in FIG. 19C, extension 93b2 may include vents in different sizes and locations.

### Exemplary cryotherapy device distal end with washing inflow path

According to some exemplary embodiments, the cryotherapy device comprises at least one inflow wash path, for example to allow introducing of a washing fluid, for example washing liquid or washing gas into the body lumen. In some embodiments, the washing fluid is introduced into the body lumen through at least one wash opening, for example at least one side opening and/or at least one front opening located at the distal end of the cryotherapy device, optionally near an opening of an inflow path used for release of a coolant material. In some embodiments, the at least one side opening is positioned in a selected angle for directing the washing fluid towards an optical assembly and/or a distal opening of an optics channel. In some embodiments, the cryotherapy device comprises at least one wash flow director, for example a nozzle or a deflecting surface in the at least one wash opening. In some embodiments, the at least one flow director is configured to direct the washing fluid towards the optical assembly, a FOV between the cryotherapy device and the target region and/or to a selected treatment space within said body lumen.

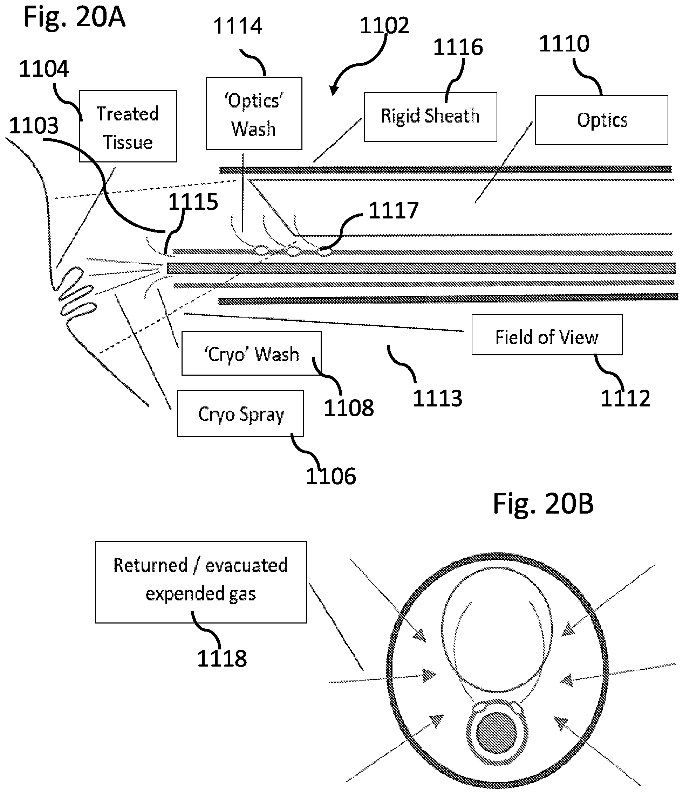

Reference is now made to FIGS. 20A and 20B depicting a distal end of a cryotherapy device having at least one inflow wash path into the body lumen, according to some exemplary embodiments of the invention.

According to some exemplary embodiments, a distal end of a cryotherapy device, for example distal end 1113 is shaped and sized to be inserted into a body lumen, for example through a working channel of an endoscope or within sheath, optionally a rigid sheath 1116. In some embodiments, the distal end 1113 is navigated towards a target region, for example treated tissue 1104 at the internal surface of the body lumen.

According to some exemplary embodiments, the cryotherapy device comprises at least one inflow path for coolant delivery into the body lumen, for cryoablating the treated tissue. In some embodiments, the coolant is delivered through a cryo inflow path, for example a cryo inflow channel. In some embodiments, the cryo spray 1106 is sprayed at the treated tissue in the target region through a nozzle.

According to some exemplary embodiments, the cryotherapy device comprises at least one optics element, for example optics 1110. In some embodiments, optics 1110 allows visualization within a FOV 1112, which optionally allows visualizing the effect of the cryo spray 1106 on the treated tissue 1104. In some embodiments, during the release of coolant into the body lumen, condensed particles are formed, optionally due to the interaction of the coolant material with liquid droplets in the humidified environment within the body lumen. In some embodiments, the formation of the condensed particles generates a cloud of particles that prevent or limits the visualization of the cryo spray effect.

According to some exemplary embodiments, the cryotherapy device comprises at least one wash inflow path for delivery of washing fluid into the body lumen, for example gas to push away the condensed particles from the FOV 1112. In some embodiments, the washing fluid is introduced through the inflow wash path and through an opening, for example opening 1115 facing the treated tissue, for example to allow cryo wash 1108. Alternatively or additionally, the inflow wash path comprises at least one sideways opening, for example sideways opening 1117 for introducing washing fluid near a distal opening of optics 1110, for example to allow optics wash 1114.

According to some exemplary embodiments, for example as shown in FIG. 20B, the cryotherapy device comprises at least one outflow path, for example to allow evacuation, also termed herein purge, of some of the gas, particles and/or liquids out from the body lumen. In some embodiments, for example as shown in FIG. 20B, the outflow path is a path surrounding the inflow paths and the optics, optionally enclosed within the sheath, for example rigid sheath 1116. In some embodiments, the outflow path is used for returned or evacuated expended gas 1118. In some embodiments, the outflow path is defined by the space within the rigid sheath and between the inflow paths and/or the optics 1110.

### Exemplary flow control

According to some exemplary embodiments, flow inside the body lumen is regulated, for example to avoid damage to tissue due to high pressure levels and/or low temperature levels. In some embodiments, flow is regulated to allow an effective cryotherapy process and/or better visualization of the target region.

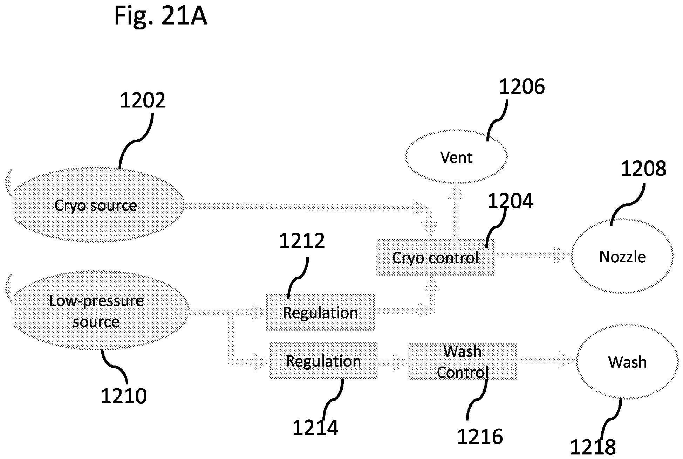

Reference is now made to FIG. 21A depicting a scheme of flow control elements, according to some exemplary embodiments of the invention.

According to some exemplary embodiments, cryogenic coolant is stored in a cryo source 1202 and is delivered through at least one inflow path, for example cryo flow path of a cryotherapy device to a nozzle 1208. In some embodiments, a cryo flow regulator, for example cryo control 1204 is positioned in the cryotherapy device, for example cryotherapy device 1002 and/or in a control unit, for example control unit 1004, shown in FIG. 1A. In some embodiments, cryo control 1204 controls the amount, for example the percentage of cryogenic fluid, for example cryogenic coolant that flows towards nozzle 1208 or through vent 1206, configured to allow release of cryogenic coolant outside the body. In some embodiments, the cryo control is under the control of control circuitry 1026. In some embodiments, the cryo control comprises a valve, for example a solenoid valve which has an inner orifice which is usually being closed by a spring - normally closed - and being opened by an electrical induced force of the solenoid, which opens the flow path.

According to some exemplary embodiments, the control circuitry signals the cryo control to allow passage of 100% towards nozzle 1208, and optionally 0% towards vent 1206. Alternatively, the control circuitry signals the cryo control to allow passage of 0% towards nozzle 1208 and 100% towards vent 1206. In some embodiments, the control circuitry determines the cryogenic coolant flow distribution between the vent 1206 and the nozzle 1208 based on the pressure level and/or temperature inside the body lumen or within the flow paths. Alternatively or additionally, the control circuitry determines the cryogenic coolant flow distribution between the vent 1206 and the nozzle 1208 based on a distance from the target region or an angle between the cryotherapy device distal end or the cryo nozzle and the target region.

According to some exemplary embodiments, a low-pressure source 1210 is connected through a different inflow path of a cryotherapy device to a wash opening 1218, for example a wash opening. In some embodiments, the wash opening comprises a slot or a notch. In some embodiments, a regulation mechanism, for example regulation 1212 regulates the flow of the low-pressure gas through the cryo flow path towards nozzle 1208. In some embodiments, low-pressure gas is directed by regulation 1212 towards the cryo nozzle 1208 when the cryogenic coolant flow is stopped and optionally, the vent is closed, for example to 'clean' the nozzle and/or to prevent moisture from getting in while cryo flow is off. Additionally or alternatively, low-pressure flow, through cryo nozzle 1208 when the cryotherapy device distal end is close to a targeted tissue, marks the tissue, for example by shallow indentation, indicating that the cryotherapy device distal end or a cryo catheter tip is close enough to the tissue, for example for optimal cryo activation and/or for preventing tip puncturing the lumen wall and/or for preventing tissue adhesion by the cryo flow.

According to some exemplary embodiments, the low-pressure flow is directed by regulation mechanism 1214 towards a wash control unit 1216 which determines the amount and pressure flow through the wash opening 1218. In some embodiments, for example between or during coolant spraying sessions the low pressure flow washes the treatment space and/or is directed towards the optics of the cryotherapy device, for example to wash the optics field of view.

### Exemplary control during cryotherapy

According to some exemplary embodiments, at least one micro-environmental parameter of the cryotherapy treatment space is controlled. In some embodiments, a cryotherapy system determines a treatment space within a body lumen. In some embodiments, the treatment space is determined to control at least one micro-environmental parameter in a limited and optionally in a more controllable space.

In some embodiments, the cryotherapy treatment space is determined based on a distance of the cryotherapy device from a selected target region. Alternatively or additionally, the treatment space is determined based on an angle between the cryotherapy device and the treatment region. Optionally the treatment space is determined based on the field of view of an imager of the cryotherapy device.

According to some exemplary embodiments, the efficacy of a cryotherapy process within the body lumen is controlled. In some embodiments, the release of a cryogenic coolant material into the body lumen is controlled. Additionally or optionally, the evacuation of the cryogenic coolant fluid from the body lumen is controlled. In some embodiments, the effect of the cryogenic coolant material on the internal tissue of the body lumen is controlled, for example to increase the efficacy of the treatment.

According to some exemplary embodiments, at least one safety parameter of a cryotherapy process is controlled. In some embodiments, the pressure and/or temperature within the body lumen during a cryotherapy process are controlled. In some embodiments, if the pressure within the body lumen is higher than a predetermined pressure value then the introduction of cryogenic fluid into the body lumen is stopped. Alternatively or additionally, the introduction of a non-cariogenic gas for clearing and/or inflating the body lumen is stopped. In some embodiments, some of the fluid, for example cariogenic gas or caryogenic liquid is evacuated through an outflow channel, either passively by opening a check valve or actively by activating a pump, for example a vacuum pump.

### Exemplary control within a defined treatment space

According to some exemplary embodiments, a treatment space is defined by limiting the space between the cryotherapy device and the target region, for example to allow better visualization of the target region. In some embodiments, the treatment space is defined by at least one extension, for example a cone or a dress that is optionally an unfolded and/or an extended element. In some embodiments, the at least one extension is put in contact with the internal surface of the body lumen, optionally around a selected target area. Alternatively or additionally, the treatment space is defined by a conic-shaped flow formed by the washing flow.

According to some exemplary embodiments, at least one environmental parameter is controlled within the defined treatment space, for example an environmental parameter that affects visualization of the target region. In some embodiments, the environmental parameter comprises temperature, and/or humidity in the treatment space.

According to some exemplary embodiments, temperature levels within the defined treatment space are controlled by evacuating some of the cryogenic fluid from the defined space. Alternatively, warm air is introduced into the defined treatment space through an inflow path of the cryotherapy device. Alternatively, an IR light source illuminates the treatment space to increase the temperature levels or to allow visualization through the mist. In some embodiments, humidity levels within the defined treatment space are controlled by introducing gas for dehydrating the space.

According to some exemplary embodiments, mist or formed particles are cleared from the defined treatment area by using wash fluid, for example to remove humidity from the FOV to allow better visualization of the target region. Alternatively, some of the mist particles are evacuated from the defined space through an outflow path in the cryotherapy device.

### Exemplary flow control process

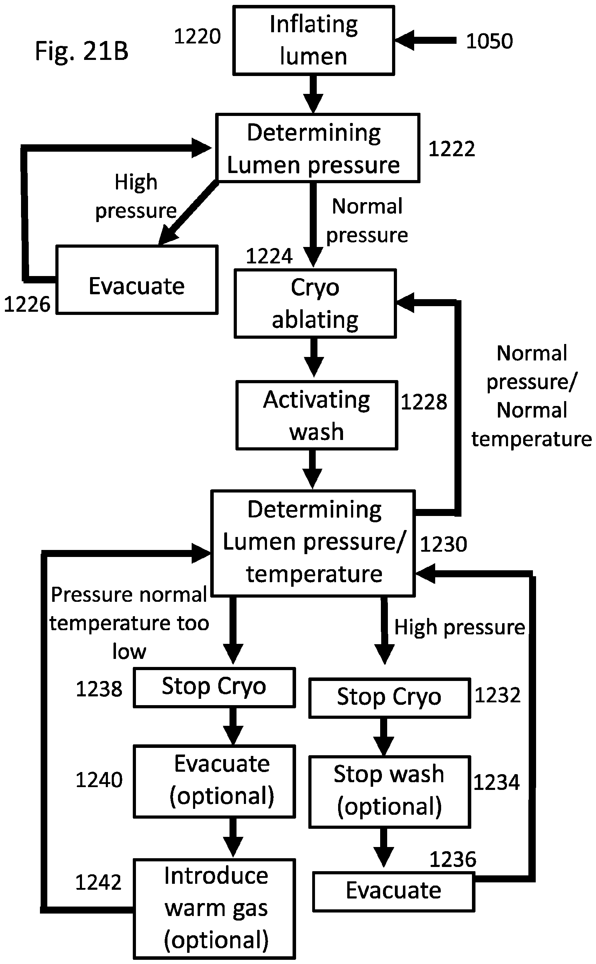

According to some exemplary embodiments, at least one micro-environmental parameter, for example pressure, temperature and/or humidity within the body lumen is monitored before during and/or after a cryotherapy process. In some embodiments, low pressure flow and/or coolant flow into the body lumen is controlled based on measurements of the at least one micro-environmental parameter, for example not to exceed a maximal pressure value or to lower the temperature within the body lumen below a pre-determined value. Reference is now made to FIG. 21B depicting a flow control process, according to some exemplary embodiments of the invention.

According to some exemplary embodiments, at least part of a cryotherapy device is introduced into a body lumen, as previously described at 1050.

According to some exemplary embodiments, the body lumen is inflated at 1220. In some embodiments, the body lumen is inflated by a low-pressure flow, or by a combination of a low-pressure flow and expanded coolant flow.

According to some exemplary embodiments, pressure levels inside the lumen are determined at 1222. In some embodiments, the pressure levels are determined based on signals received from at least one sensor, for example a pressure sensor positioned inside the body lumen, and/or based on at least one sensor positioned outside the body lumen or inside a flow path of the cryotherapy device, for example an outflow path. In some embodiments, the pressure levels inside the lumen are determined by monitoring pressure levels and/or pressure changes within a wash inflow path, optionally by a sensor positioned outside the body. Alternatively, the sensor is positioned within part of the washing inflow path positioned inside the body.

According to some exemplary embodiments, if the determined pressure post inflating is normal, and/or in a desired range of values then the coolant is introduced into the body lumen for cryoablating a selected target region at 1224. In some embodiments, the coolant is introduced during a predetermined time period. Alternatively, if the determined pressure levels are too high, then at least some of the gas within the body lumen is evacuated, optionally through an outflow channel, at 1226.

According to some exemplary embodiments, wash is activated and introduced into the body lumen at 1228. Alternatively the washing fluid is used for inflating the body lumen at 1220. In some embodiments, wash is introduced through the cryo nozzle, during purge or through a wash inflow channel, for example to wash an optical assembly and/or the treatment space between the distal end of the cryotherapy device and the target region. Alternatively or additionally, the wash is used around the cryo spray, for example to push away the cryo spray from the optical assembly and/or to result with a virtual cone/dress that optionally defines a micro-environment.

According to some exemplary embodiments, pressure and/or temperature within the body lumen are determined at 1230. In some embodiments, the pressure is determined post cryo ablating and/or post wash to determine if the pressure within the body lumen does not exceed a maximal allowed pressure value. Additionally or alternatively, the temperature is determined before cryotherapy, during cryotherapy and post cryotherapy to determine if the introduced coolant did not lower the temperature levels inside the body lumen below a pre-determined value. In some embodiments, if the pressure and temperature are in a range of allowed values, then optionally a consecutive cryo ablating coolant flow is introduced into the body lumen at 1224.

According to some exemplary embodiments, if the pressure is within a normal range of values but the temperature is too low, then the cryo ablating process is stopped at 1238. In some embodiments, at least some of the fluid within the body lumen, for example gas or liquid is evacuated at 1240. Alternatively or additionally, warm gas is introduced into the body lumen at 1242, for example to increase the temperature within the body lumen. Alternatively, the warm gas is introduced into the body lumen through the inflow wash channel during the cryotherapy, and optionally during the inflation of the body lumen.. In some embodiments, the pressure and /or the temperature are determined at 1230, after some of the gas is evacuated and/or warm gas is introduced into the body lumen.

According to some exemplary embodiments, if the pressure inside the body lumen is too high, then the cryo ablating is stopped at 1232, and optionally the wash is stopped at 1234. In some embodiments, some of the gas inside the body lumen is evacuated at 1236.

### Exemplary pressure monitoring inside lumen

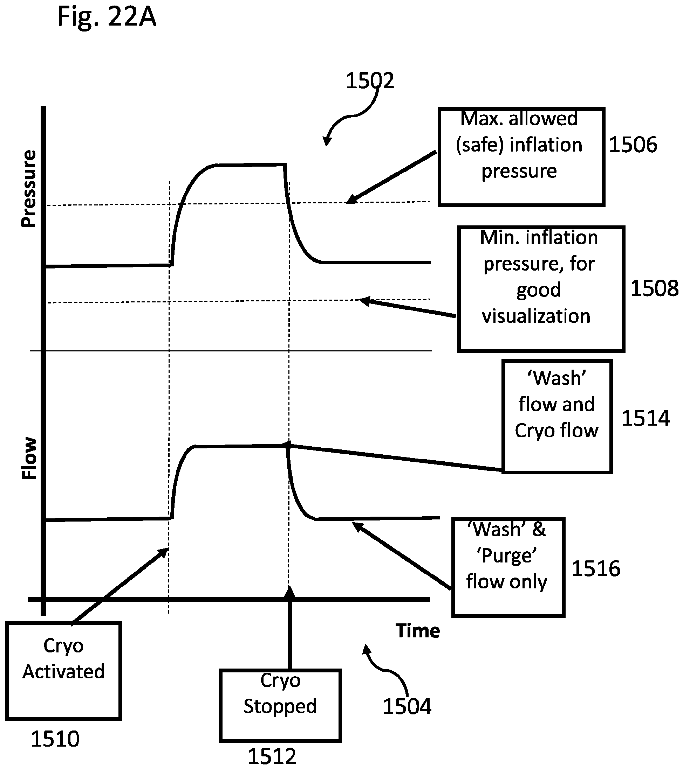

Reference is now made to FIGs. 22A-22F depicting pressure monitoring within a range of pre-determined pressure levels within the body lumen. According to some exemplary embodiments, in the pressure graphs 1502 pressure is desirably maintained between a minimal inflation pressure value 1508, and a maximal inflation pressure value 1506. In some embodiments, when wash flow, cryo flow and purge flow are activated, as shown in graph 1504 the pressure levels inside the body lumen are being changed accordingly.

In some embodiments, pressure within the body lumen is maintained above the minimal inflation pressure value, for example to allow better visualization of a target region and/or to allow better accessibility to the target region. In some embodiments, the minimal pressure inside the bladder is at least 10 mBar, for example 10 mBar, 12 mBar, 15 mBar or any intermediate, smaller or larger value. In some embodiments, pressure inside the body lumen is maintained below a maximal pressure value, for example to prevent tissue damage. In some embodiments, the maximal pressure inside the bladder is about 30 mBar.

According to some exemplary embodiments, for example as shown in FIG. 22A, in a cryo activation cycle, when wash and purge flow are applied at 1516, pressure is within the desired range, between the minimal pressure value 1508 and the maximal pressure value 1506. In some embodiments, when cryo flow is activated at 1510, pressure increases above the maximal allowed pressure value, until cryo flow is stopped at 1512.

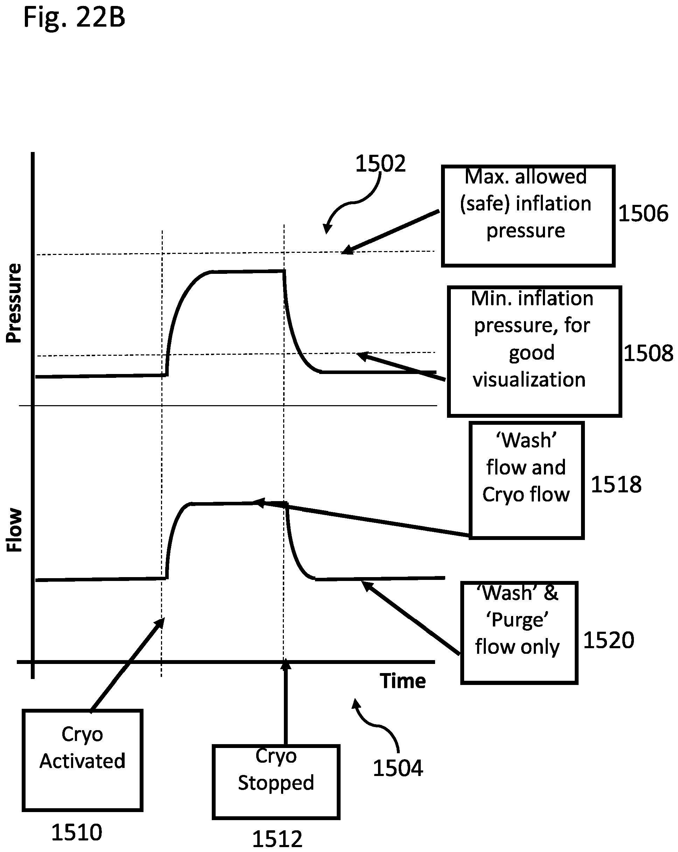

According to some exemplary embodiments, for example as shown in FIG. 22B, when the constant wash flow and purge flow are activated in an overall flow 1520, and the evacuation opening is wider, the pressure inside the body lumen is below the minimal pressure value 1508. In some embodiments, a partial or a complete collapse of the body lumen due to a low inflation pressure prevents good visualization of the target region and/or limits or prevents the navigation of the cryotherapy device within the body lumen, which may reduce the efficacy of the cryo ablating treatment. In some embodiments, when cryo flow is activated at 1510, the overall flow 1518 leads to an overall pressure is within the desired range during cryoablation.

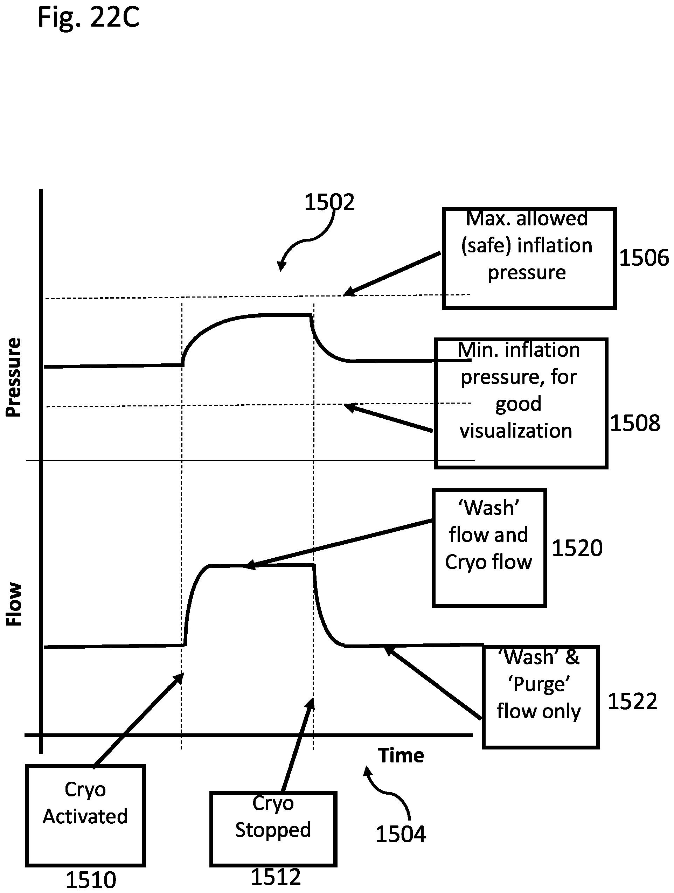

According to some exemplary embodiments, for example as shown in FIG. 22C, when using a valve, for example a check valve, fluid evacuation out from the body lumen is optionally controlled and the constant flow 1522 is causing the pressure, for example when cryo flow is not activated, to be higher than a minimal pressure needed for inflation. In some embodiments, when cryo is activated, the pressure is elevated but is still below the maximal pressure value. In some embodiments, this situation is both safe and should allow work inside the lumen, for example by allowing good visualization and ability to maneuver within the body lumen.

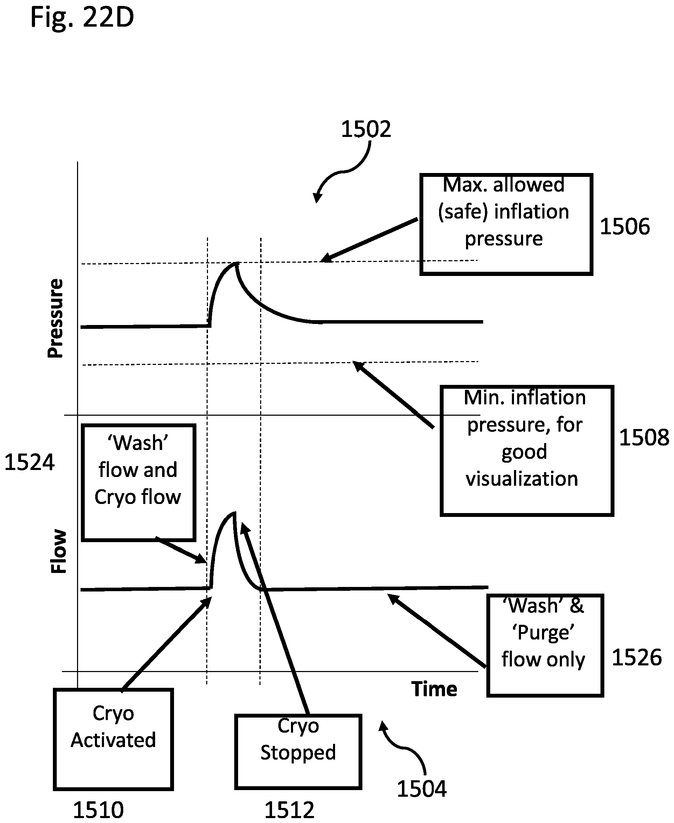

According to some exemplary embodiments, for example as shown in FIG. 22D an active feedback on flow control is used to maintain the pressure inside the body lumen below the maximal pressure value. In some embodiments, cryo flow is activated at 1510 to introduce coolant into the body lumen with a wash flow. In some embodiments, the cryo flow and the wash flow at 1524 increase the pressure level to the maximal allowed pressure value, as optionally measured by a sensor optionally through an inflow path, for example the inflow wash path. In some embodiments, when the pressure level reaches the maximal allowed pressure level, an active flow control or an active flow regulator stops cryo flow. Additionally, if the pressure still increases, the active flow control stops also the wash flow.

According to some exemplary embodiments, the 'Wash' flow has several purposes, for example, to constantly inflate the lumen, for example to prevent the partial or fully collapse to optionally allow good visualization and ability to maneuver inside the lumen. Additionally or alternatively, wash flow is used to clean the optical assembly, for example an optics lens from moisture and other dirt and optionally to keep the optical assembly optics in a relatively constant temperature, for example in a temperature level with variations in a range of up to 30%, for example 10%, 15%, 20% or any intermediate smaller or larger variation percentage. In some embodiments, wash flow is used to wash cold moisture gases (mist) away from the optics ('Cryo wash' - create insulation around the cryo spray) and optionally to build a dynamic buffer around the lens surface ('Optics wash'), in a way that it is hard for mist remainders (or evacuated mist) to flow towards the lens direction. Alternatively and/or additionally, the cryo wash is used for pressure measurement. In some embodiments, as a result of pressure changes inside the lumen, pressure changes can be measured closer to the wash pressure source (upstream - e.g. inside the controlling console), in a way that pressure inside the lumen can be measured/approximated and optionally can be used, for example, for control and/or safety means.

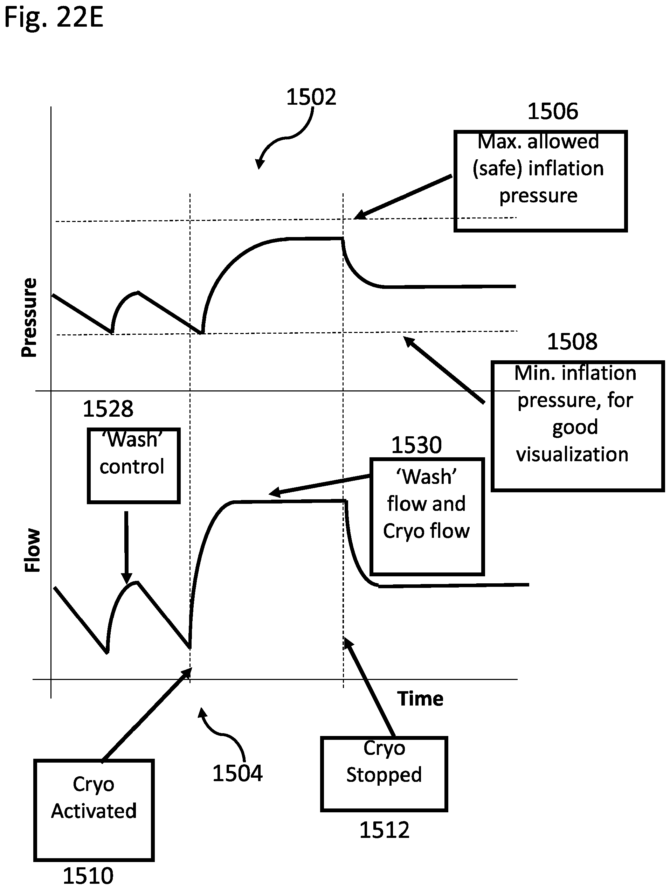

According to some exemplary embodiments, for example as shown in FIG. 22E an active control of the wash flow is applied at 1528, for example to make sure that the pressure level is between a minimal pressure value necessary for inflation of the body lumen and lower than a maximal pressure value. Optionally controlling wash flow allows to save washing fluid for example during long observation or visualization time periods between cryo spraying sessions. In some embodiments, when cryo is applied at 1504, the active wash flow control is deactivated.

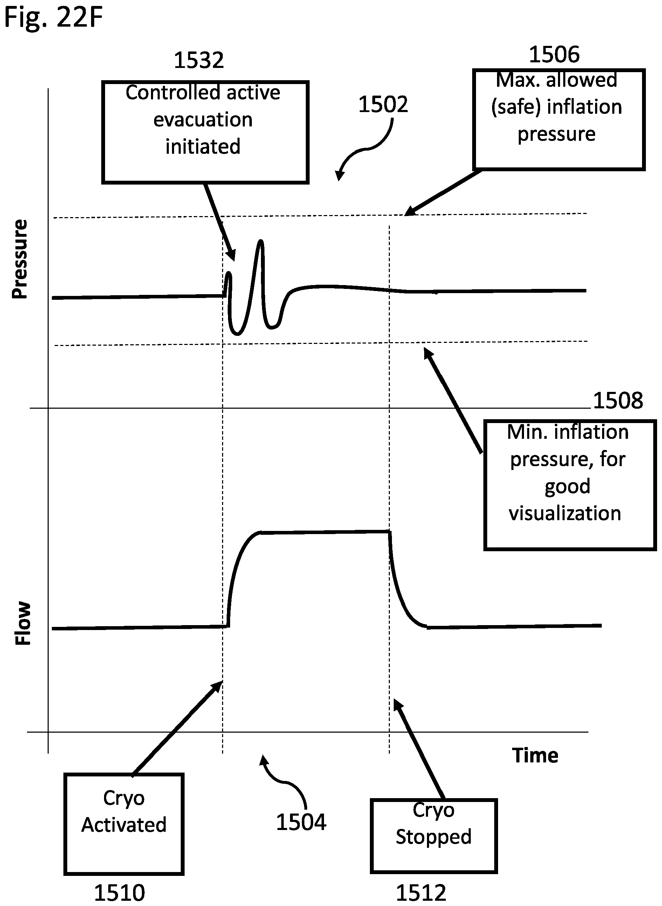

According to some exemplary embodiments, for example as shown in FIG. 22F, in addition to active flow control, an active control on evacuation of material from the body lumen is also activated. In some embodiments, when cryo flow is activated at 1510, an active evacuation control is activated at 1532, for example by activating a vacuum pump to evacuate some fluids and/or other materials from the body lumen which will optionally reduce the pressure level.

### Exemplary determining a distance and an angle to a target region

According to some exemplary embodiments, the distance between the distal end of the cryotherapy device to the target region is determined, for example to allow better control on the effect of the coolant on the tissue. In some embodiments, spraying coolant too close to the tissue may lead to damage in more deeper layers of the tissue that were not selected as a target for the cryotherapy. Alternatively, spraying coolant from a large distance on the target region may result with an ineffective treatment, for example due to the generation of mist and/or due to visualization difficulties. Additionally, determining the distance to the target region allows, for example to adjust at least one parameter of the cryotherapy process for example coolant pressure and/or duration of coolant spraying.

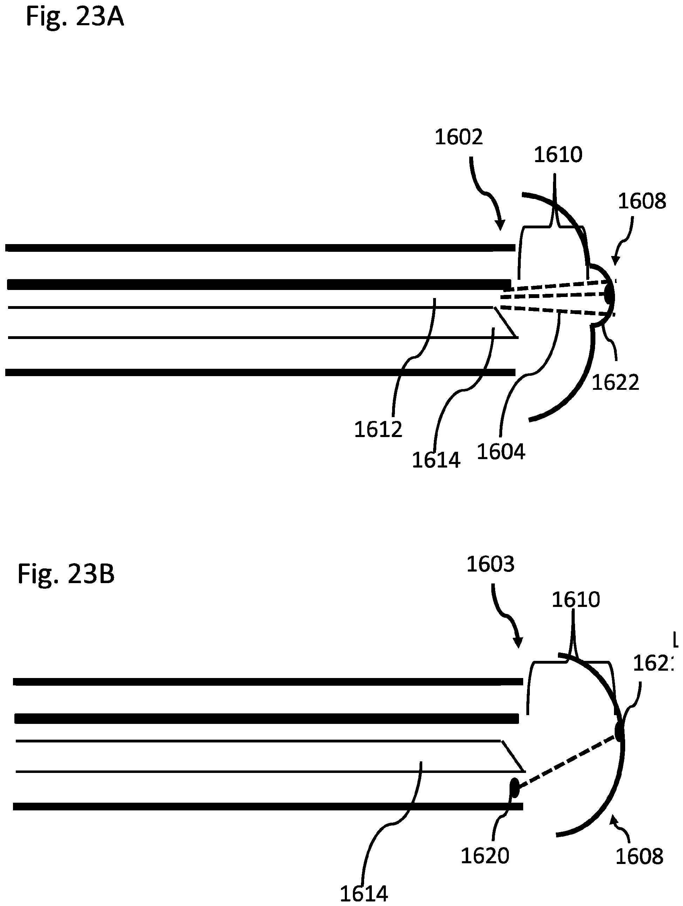

According to some exemplary embodiments, for example as shown in FIG. 23A, a distal end 1602 of a cryotherapy device is advanced towards a target region 1608. In some embodiments, a low pressure gas flow 1604 is released optionally from a wash flow path 1612 towards the target region 1608 and/or through the cryo nozzle by a purge flow, when the cryogenic fluid flow is stopped. In some embodiments, the pressure of the low-pressure gas generates an indentation 1622 in the body lumen surface. In some embodiments, the distance 1610 between the distal end 1602 to the target region 1608 is determined by visualizing the depth and/or the shape of the indentation 1622 using optics 1614.

According to some exemplary embodiments, for example as shown in FIG. 23B, the distal end 1603 of the cryotherapy device comprises at least one illumination source 1620 configured to project a light beam towards the target region 1608. In some embodiments, the distance 1610 between the distal end 1603 and the target region 1608 is determined by visualizing the size and/or shape of the projected point 1621 at the target region 1608, for example manually or automatically by image processing. Alternatively or additionally, the cryotherapy device comprises a light meter, optionally a laser light meter for measuring the distance to the target region.

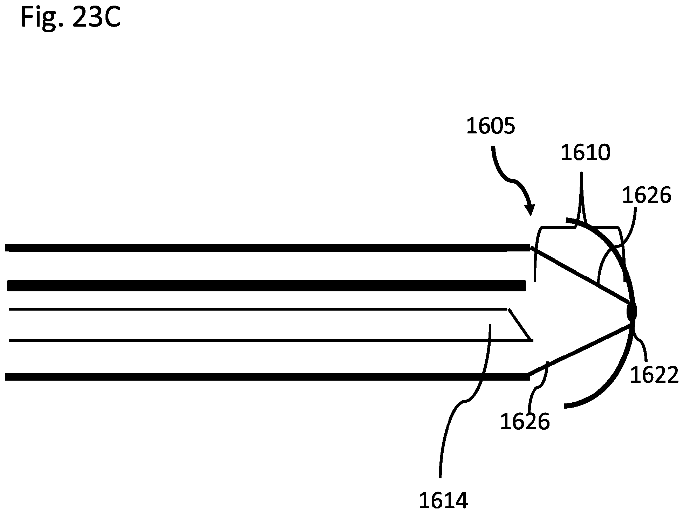

According to some exemplary embodiments, for example as shown in FIG. 23C, the cryotherapy device comprises at least one foldable element, for example folded elements 1626 which are optionally an extension, a dress or a cone. Optionally, the folded elements have a known length in an unfolded state. In some embodiments, the folded elements 1626 are configured to be folded inside a lumen of the cryotherapy device during navigation towards the target region, and to unfold when reaching the target region. In some embodiments, when the elements are unfolded they contact a tissue near or at the target region. In some embodiments, visualizing the contact point between the folded elements and the tissue allows, for example to determine the distance 1610 between the distal end 1605 and the target region.

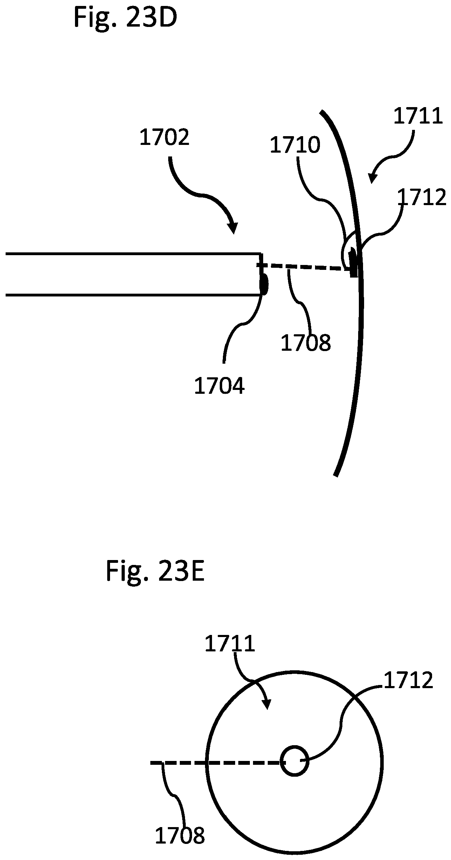

According to some exemplary embodiments, for example as shown in FIGs. 23D and 23E, an angle between the distal end 1702 and a target region 1711 is determined based on a shape and size of a light spot 1712 projected on the target region. In some embodiments, for example as shown in FIG. 23D, a light beam 1708 is projected at a 90 degrees angle 1710 by an illumination source on the target region 1711. In some embodiments, visualization of the shape and/or size of the light spot allows to determine the relative angle between the distal end 1702 and the target region 1711. In some embodiments, when projected at a 90 degrees angle, the light spot 1712 has the smallest size, length and/or diameter relative to other projection angles. Optionally, when projected at a 90 degrees angle, the light spot has a round shape, for example as shown in an upper view of the target region in Fig. 23E.

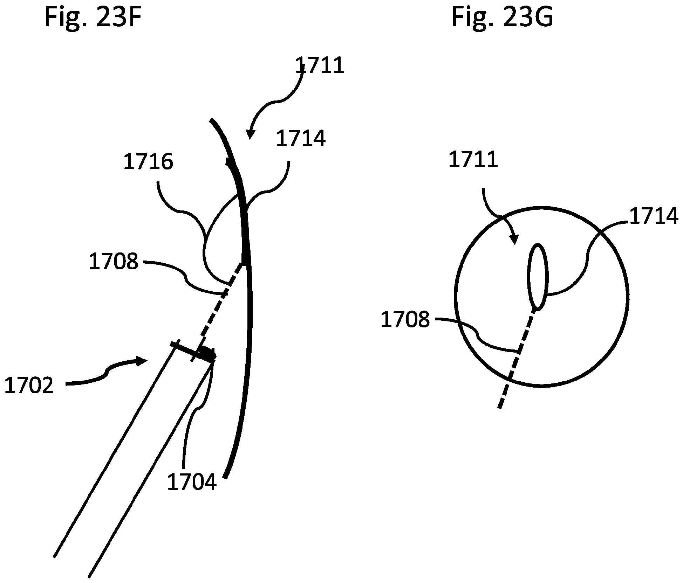

According to some exemplary embodiments, for example as shown in FIG. 23F the light beam 1708 is projected at an angle 1716 which is larger than 90 degrees. In some embodiments, when projecting a light beam at angle larger than 90 degrees, the light spot 1714 is larger, and optionally more oval compared to the light spot 1712, for example as shown in FIG. 23G depicting an upper view of the target region 1711. Alternatively or additionally, the same as the illumination effect, purge flow may result in the same oval shape related to angle, or bigger circle related to bigger distance between cryo tip and the treated target tissue.

According to some exemplary embodiments, at least one parameter of the cryotherapy, for example pressure of the cryogenic fluid to be released into the body lumen, position of the cryotherapy device distal end, and/or cryo spraying duration is modified or adjusted, optionally automatically based on the determined distance and/or angle. In some embodiments, a new treatment space and/or a new FOV is defined based on the determined angle and/or distance. In some embodiments, the washing fluid flow direction is adjusted or modified, for example to direct the washing fluid towards the new defined treatment space and/or towards the new defined FOV.

The preceding specific embodiments are illustrative of the practice of the techniques of this disclosure. It is to be understood, therefore, that other expedients known to those skilled in the art or disclosed herein may be employed without departing from the scope of the following claims.

It is expected that during the life of a patent maturing from this application many relevant cryotherapy devices will be developed; the scope of the term cryotherapy device is intended to include all such new technologies *a priori.* As used herein with reference to quantity or value, the term "about" means "within ± 10 % of'.

The terms "comprises", "comprising", "includes", "including", "has", "having" and their conjugates mean "including but not limited to".

The term "consisting of' means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular forms "a", "an" and "the" include plural references unless the context clearly dictates otherwise. For example, the term "a compound" or "at least one compound" may include a plurality of compounds, including mixtures thereof.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

## Claims

1. A cryotherapy system (1000) for ablating tissue in a target region (1104) at an internal surface of a body lumen by cryogenic fluid, comprising,
an elongated tubular cryotherapy device (10, 1002) having a proximal end (1025) and a distal end (1021, 1113) shaped and sized to be positioned inside a body lumen, comprising:
a cryo inflow path (133, 1006) within said elongated tubular cryotherapy device (10, 1002), fluidically connecting a cryogenic fluid source with at least one forward facing cryo opening (69a) at said distal end (1021, 1113) of said cryotherapy device (10, 1002), wherein said cryo inflow path (133, 1006) is configured to allow cryogenic fluid flow from said cryogenic fluid source, and to direct said cryogenic fluid at said target region within said body lumen through said cryo opening (69a);
an optics assembly configured to visualize a field of view (1112) between said optics assembly and said target region inside said body lumen;
a washing inflow path (1014) within said elongated tubular cryotherapy device (10, 1002), fluidically connecting a washing fluid source with at least one sideways washing opening (1117) at said elongated therapy device distal end configured to aim washing fluid towards one or both of said optics assembly and said field of view (1112), and at least one front washing opening (1115) at said elongated therapy device distal end.

2. The system (1000) of claim 1, wherein said least one sideways washing opening is configured to direct said washing fluid towards said field of view (1112) and/or away of said optics assembly.

3. The system (1000) of any one of claims 1 or 2, wherein said cryotherapy device comprises at least one washing flow director at said at least one front washing opening, and said washing flow director comprises a nozzle and/or a deflecting surface that results with a flow shaped as a virtual cone/dress that optionally defines a micro-environment.

4. The system (1000) of any one of the previous claims, wherein said at least one sideways washing opening is configured to direct said washing fluid towards said optics assembly that comprises at least one lens and/or at least one illumination source.

5. The system (1000) of any one of the previous claims, comprising at least one washing flow regulator that controls flow through said washing inflow path.

6. The system (1000) of claim 5, comprising at least one cryo flow regulator on said cryo inflow path, configured to control flow of said cryogenic fluid through said cryo inflow path into said body lumen.

7. The system (1000) of claim 6, comprising:
a control circuitry connected to said at least one cryo flow regulator and/or said at least one washing flow regulator; and
at least one pressure sensor connected to said control circuitry, configured to measure body lumen pressure or changes in said body lumen pressure, and wherein said control circuitry controls flow within said cryo inflow path and/or said washing inflow path, based on said at least one pressure sensor measurements.

8. The system (1000) of claim 7, wherein said control circuitry stops washing fluid flow into said body lumen through said washing inflow path if a pressure level measured by the at least one pressure sensor is higher than a predetermined maximal pressure level.

9. The system (1000) of claim 6, comprising:
a control circuitry connected to said at least one cryo flow regulator and/or at least one washing flow regulator; and
at least one temperature sensor connected to said control circuitry, configured to measure temperature levels and/or temperature changes inside said body lumen, and wherein said control circuitry controls the flow of said cryogenic fluid through said cryo inflow path and/or controls said washing inflow path into said body lumen, based on said measured temperature values.

10. The system (1000) of any one of the previous claims, wherein said device comprises at least one outflow path extending from said body lumen and outside the body configured to evacuate fluid from a distal opening at said distal end (1021, 1113) towards a proximal opening of said cryotherapy device; and
at least one outflow regulator on said outflow path configured to control fluid evacuation from said body lumen through said at least one outflow path.

11. The system (1000) of claim 10, wherein said outflow regulator comprises a check valve configured to open when a pressure measured inside said body lumen is higher than a predetermined value.

12. The system (1000) of any one of the previous claims, wherein said elongated tubular cryotherapy device comprises:
at least one purge flow regulator on a purge inflow path partly overlapping with said cryo inflow path (133, 1006), configured to allow a non-cryogenic purge fluid flow through said cryo inflow path (133, 1006) into said body lumen when said cryogenic fluid flow is reduced thereby reducing backflow into said cryo inflow path (133, 1006) from said lumen.

13. The system (1000) of any one of the previous claims wherein said body lumen comprises a bladder.

14. The system (1000) of any one of the previous claims wherein said body lumen comprises a cervix, a prostate, a urethra, a ureter, a stomach or a uterus.

15. The system (1000) of any one of the previous claims, wherein said elongated tubular cryotherapy device (10, 1002) which is shaped and sized to be introduced into said body lumen through a working channel of an endoscope, wherein said device has an overall diameter in a range of 0.8-9 mm.

## Patentansprüche

1. Kryotherapiesystem (1000) zur Ablation von Gewebe in einem Zielbereich (1104) an einer inneren Oberfläche
eines Körperlumens durch kryogene Flüssigkeit, das Folgendes umfasst,
ein längliches, röhrenförmiges Kryotherapiegerät (10, 1002) mit einem proximalen Ende (1025) und einem distalen Ende (1021, 1113), das so geformt und bemessen ist, dass es innerhalb eines Körperlumens positioniert werden kann, Folgendes umfassend:
einen Kryozuflusspfad (133, 1006) innerhalb des länglichen röhrenförmigen Kryotherapiegeräts (10, 1002), der eine Kryoflüssigkeitsquelle mit mindestens einer nach vorne weisenden Kryo-Öffnung (69a) am besagten distalen Ende (1021, 1113) des Geräts (10, 1002) strömungsmäßig verbindet, wobei der besagte Kryo-Zuflusspfad (133, 1006) so konfiguriert ist, dass er ein Kryoflüssigkeitsdurchfluss von der besagten Kryoflüssigkeitsquelle ermöglicht und die Kryoflüssigkeit durch die Kryo-Öffnung (69a) auf den besagten Zielbereich innerhalb des besagten Körperlumens richtet;
eine Optikbaugruppe, die so konfiguriert ist, dass sie ein Sichtfeld (1112) zwischen der besagten Optikbaugruppe und dem besagten Zielbereich innerhalb des besagten Körperlumens sichtbar macht;
einen Wascheinströmungspfad (1014) innerhalb des besagten länglichen, röhrenförmigen Kryotherapiegeräts (10, 1002), der eine Waschmittelquelle mit mindestens einer seitlichen Waschöffnung (1117) am distalen Ende des besagten länglichen Therapiegeräts strömungsmäßig verbindet,
die so konfiguriert ist, dass sie die Waschflüssigkeit auf die besagte Optikbaugruppe und/oder das besagte Sichtfeld (1112) richtet, und mindestens eine vordere Waschöffnung (1115) am distalen Ende des besagten länglichen Therapiegeräts.

2. System (1000) nach Anspruch 1, wobei die besgte mindestens eine seitliche Waschöffnung so konfiguriert ist, dass sie das besagte Waschmittel in Richtung des besagten Sichtfelds (1112) und/oder von der besagten Optikanordnung wegleitet.

3. Das System (1000) nach einem der Ansprüche 1 oder 2, wobei das besagte kryotherapeutische Gerät mindestens einen Waschdurchflussleiter an der besagten mindestens einen vorderen Waschöffnung umfasst, und der besagte Waschdurchflussleiter eine Düse und/oder eine Ablenkfläche umfasst, die zu einer Strömung führt, die als virtueller Kegel/Mantel geformt ist und optional eine Mikroumgebung definiert.

4. Das System (1000) nach einem der vorhergehenden Ansprüche, wobei die besagte mindestens eine seitliche Waschöffnung so konfiguriert ist, dass sie das besagte Waschmittel in Richtung der besagten optischen Anordnung leitet, die mindestens eine Linse und/oder mindestens eine Beleuchtungsquelle umfasst.

5. Das System (1000) nach einem der vorhergehenden Ansprüche umfasst mindestens einen Waschdurchflussregler, der den Durchfluss durch den besagten Waschzuflussweg steuert.

6. System (1000) nach Anspruch 5, das mindestens einen Kryostromregler an dem besagten Kryozuflusspfad umfasst, der so konfiguriert ist, dass er den Durchfluss der besagten kryogenen Flüssigkeit durch den Kryozuflusspfad in das besagte
Körperlumen regelt.

7. Das System (1000) nach Anspruch 6, das Folgendes umfasst:
eine Steuerschaltung, die mit dem besagten mindestens einen Kryodurchflussregler und/oder dem besagten mindestens einen Waschmitteldurchflussregler verbunden ist; und
mindestens einen Drucksensor, der mit dem besagten Steuerschaltkreis verbunden ist und so konfiguriert ist, dass er den besagten Körperlumendruck oder
Änderungen des Körperlumendrucks misst, wobei der besagte Steuerschaltkreis den Durchfluss innerhalb des besagten Kryozuflusspfads und/oder des Waschmittelzuflusspfads basierend auf den besagten Messungen des mindestens einen Drucksensors steuert.

8. System (1000) nach Anspruch 7, wobei die besagte Steuerschaltung den Waschmitteldurchfluss in das besagte Körperlumen durch den besagten Waschmittelzuflusspfad stoppt, wenn ein von dem mindestens einen Drucksensor gemessener Druckpegel höher als ein vorbestimmter maximaler Druckpegel ist.

9. Das System (1000) nach Anspruch 6, das Folgendes umfasst:
eine Steuerschaltung, die mit dem besagten mindestens einen Kryodurchflussregler und/oder dem mindestens einen Waschdurchflussregler verbunden ist; und
mindestens einen Temperatursensor, der mit dem besagten Steuerschaltkreis verbunden ist und so konfiguriert ist, dass er Temperaturniveaus und/oder Temperaturänderungen innerhalb des besagten Körperlumens misst, wobei der besagte Steuerschaltkreis den Durchfluss der besagten kryogenen Flüssigkeit durch den besagten Kryozuflusspfad steuert und/oder den besagten Waschmittelzuflusspfad in das besagte Körperlumen steuert, basierend auf den besagten gemessenen Temperaturwerten.

10. System (1000) nach einem der vorhergehenden Ansprüche, wobei das besagte Gerät mindestens einen Abflusspfad umfasst, der sich von dem besagten Körperlumen und außerhalb des Körpers erstreckt und so konfiguriert ist, dass er Flüssigkeit von einer distalen Öffnung an dem besagten distalen Ende (1021, 1113) zu einer proximalen Öffnung des besagten Kryotherapiegeräts ableitet; und
mindestens einen Ausflussregler auf dem besagten Ausflusspfad, der so konfiguriert ist, dass er die Flüssigkeitsabfuhr aus dem besagten
Körperlumen durch den besagten mindestens einen Ausflusspfad steuert.

11. System (1000) nach Anspruch 10, wobei der besagte Ausflussregler ein Rückschlagventil umfasst, das so konfiguriert ist, dass es sich öffnet, wenn ein im Inneren des besagten Körperlumens gemessener Druck höher als ein vorbestimmter Wert ist.

12. Das System (1000) nach einem der vorhergehenden Ansprüche, wobei das besagte längliche, röhrenförmige Gerät zur Kryotherapie Folgendes umfasst:
mindestens einen Spüldurchflussregler auf einem Spüleinflusspfad, der sich teilweise mit dem besagten Kryoeinflusspfad (133, 1006) überlappt, der so konfiguriert ist, dass er einen nicht-kryogenen Spüldurchfluss durch den besagten Kryoeinflusspfad (133, 1006) in das besagte Körperlumen ermöglicht, wenn der besagte kryogene Flüssigkeitsdurchfluss reduziert wird, wodurch der Rückfluss in den besagten Kryoeinflusspfad (133, 1006) aus dem besagten Lumen reduziert wird.

13. Das System (1000) nach einem der vorhergehenden Ansprüche, wobei das besagte Körperlumen eine Harnblase.

14. Das System (1000) nach einem der vorhergehenden Ansprüche, wobei das besagte Körperlumen einen Gebärmutterhals, eine Prostata, eine Harnröhre, einen Harnleiter, einen Magen oder einen Uterus umfasst.

15. System (1000) nach einem der vorhergehenden Ansprüche, wobei das besagte längliche, röhrenförmige Kryotherapiegerät (10, 1002) so geformt und bemessen ist, dass es durch einen Arbeitskanal eines Endoskops in das besagte Körperlumen eingeführt werden kann, wobei das Gerät einen Gesamtdurchmesser in einem Bereich von 0,8-9 mm aufweist.

## Revendications

1. Système de cryothérapie (1000) pour l'ablation de tissus dans une région cible (1104) au niveau d'une surface interne d'une lumière corporelle par un fluide cryogénique, comprenant:
un dispositif de cryothérapie tubulaire allongé (10, 1002) ayant une extrémité proximale (1025) et une extrémité distale (1021, 1113) formée et dimensionnée pour être positionnée à l'intérieur d'une lumière corporelle, comprenant:
un trajet d'entrée de fluide cryogénique (133, 1006) à l'intérieur dudit dispositif de cryothérapie tubulaire allongé (10, 1002), reliant de manière fluidique une source de fluide cryogénique à au moins une ouverture de fluide cryogénique orientée vers l'avant (69a) au niveau de ladite extrémité distale (1021, 1113) dudit dispositif de cryothérapie (10, 1002), dans lequel ledit trajet d'entrée de fluide cryogénique (133, 1006) est configuré pour permettre un écoulement de fluide cryogénique depuis ladite source de fluide cryogénique, et pour diriger ledit fluide cryogénique vers ladite région cible à l'intérieur de ladite lumière corporelle à travers ladite ouverture cryogénique (69a);
un ensemble optique configuré pour visualiser un champ de vision (1112) entre ledit ensemble optique et ladite région cible à l'intérieur de ladite lumière corporelle;
un chemin d'entrée de lavage (1014) à l'intérieur dudit dispositif de cryothérapie tubulaire allongé (10, 1002), reliant de manière fluide une source de fluide de lavage avec au moins une ouverture de lavage latérale (1117) au niveau de ladite extrémité distale du dispositif de thérapie allongé configurée pour diriger le fluide de lavage vers l'un ou les deux dudit ensemble optique et dudit champ de vision (1112), et au moins une ouverture de lavage avant (1115) au niveau de ladite extrémité distale du dispositif de thérapie allongé.

2. Système (1000) selon la revendication 1, dans lequel ladite au moins une ouverture de lavage latérale est configurée pour diriger ledit fluide de lavage vers ledit champ de vision (1112) et/ou loin dudit ensemble optique.

3. Système (1000) selon l'une quelconque des revendications 1 ou 2, dans lequel ledit dispositif de cryothérapie comprend au moins un directeur d'écoulement de lavage au niveau de ladite au moins une ouverture de lavage avant, et ledit directeur d'écoulement de lavage comprend une buse et/ou une surface de déviation qui donne lieu à un écoulement en forme de cône/dressage virtuel qui définit éventuellement un micro-environnement.

4. Système (1000) selon l'une quelconque des revendications précédentes, dans lequel ladite au moins une ouverture de lavage latérale est configurée pour diriger ledit fluide de lavage vers ledit ensemble optique qui comprend au moins une lentille et/ou au moins une source d'éclairage.

5. Système (1000) selon l'une quelconque des revendications précédentes, comprenant au moins un régulateur de débit de lavage qui contrôle le débit à travers ledit chemin d'entrée de lavage.

6. Système (1000) selon la revendication 5, comprenant au moins un régulateur de débit cryogénique sur ledit chemin d'entrée cryogénique, configuré pour contrôler le débit dudit fluide cryogénique à travers ledit chemin d'entrée cryogénique dans ladite lumière corporelle.

7. Système (1000) selon la revendication 6, comprenant:
un circuit de commande connecté audit au moins un régulateur de débit cryogénique et/ou audit au moins un régulateur de débit de lavage; et
au moins un capteur de pression connecté audit circuit de commande, configuré pour mesurer la pression de la lumière corporelle ou des changements dans ladite pression de la lumière corporelle, et
dans lequel ledit circuit de commande contrôle l'écoulement dans ledit chemin d'écoulement d'entrée cryogénique et/ou ledit chemin d'écoulement d'entrée de lavage, sur la base desdites au moins une mesures du capteur de pression.

8. Système (1000) selon la revendication 7, dans lequel ledit circuit de commande arrête l'écoulement de fluide de lavage dans ladite lumière corporelle à travers ledit chemin d'entrée de lavage si un niveau de pression mesuré par l'au moins un capteur de pression est supérieur à un niveau de pression maximal prédéterminé.

9. Système (1000) selon la revendication 6, comprenant:
un circuit de commande connecté audit au moins un régulateur de débit cryogénique et/ou au moins un régulateur de débit de lavage; et
au moins un capteur de température connecté audit circuit de commande, configuré pour mesurer les niveaux de température et/ou les changements de température à l'intérieur de ladite lumière corporelle, et dans lequel ledit circuit de commande contrôle l'écoulement dudit fluide cryogénique à travers ladite voie d'écoulement cryogénique et/ou contrôle ladite voie d'écoulement de lavage dans ladite lumière corporelle, sur la base desdites valeurs de température mesurées.

10. Système (1000) selon l'une quelconque des revendications précédentes, dans lequel ledit dispositif comprend au moins un chemin d'écoulement s'étendant depuis ladite lumière corporelle et à l'extérieur du corps configuré pour évacuer le fluide depuis une ouverture distale à ladite extrémité distale (1021, 1113) vers une ouverture proximale dudit dispositif de cryothérapie; et
au moins un régulateur d'écoulement sur ledit chemin d'écoulement configuré pour contrôler l'évacuation du fluide de ladite lumière corporelle à travers ledit au moins un chemin d'écoulement.

11. Système (1000) selon la revendication 10, dans lequel ledit régulateur d'écoulement comprend une soupape de retenue configurée pour s'ouvrir lorsqu'une pression mesurée à l'intérieur de ladite lumière corporelle est supérieure à une valeur prédéterminée.

12. Système (1000) selon l'une quelconque des revendications précédentes, dans lequel ledit dispositif de cryothérapie tubulaire allongé comprend:
au moins un régulateur d'écoulement de purge sur un chemin d'écoulement d'entrée de purge chevauchant partiellement ledit chemin d'écoulement d'entrée cryogénique (133, 1006), configuré pour permettre un écoulement de fluide de purge non cryogénique à travers ledit chemin d'écoulement d'entrée cryogénique (133, 1006) dans ladite lumière corporelle lorsque ledit écoulement de fluide cryogénique est réduit, réduisant ainsi le reflux dans ledit chemin d'écoulement d'entrée cryogénique (133, 1006) depuis ladite lumière.

13. Système (1000) selon l'une quelconque des revendications précédentes, dans lequel ladite lumière corporelle comprend une vessie.

14. Système (1000) selon l'une quelconque des revendications précédentes, dans lequel ladite lumière corporelle comprend un col de l'utérus, une prostate, un urètre, un uretère, un estomac ou un utérus.

15. Système (1000) selon l'une quelconque des revendications précédentes, dans lequel ledit dispositif de cryothérapie tubulaire allongé (10, 1002) qui est formé et dimensionné pour être introduit dans ladite lumière corporelle à travers un canal de travail d'un endoscope, dans lequel ledit dispositif a un diamètre global dans une gamme de 0,8-9 mm.
